(19) Europäisches Patentamt
European Patent Office
Office européen des brevets

(11) EP 0 921 116 B1

(12) **EUROPÄISCHE PATENTSCHRIFT**

(45) Veröffentlichungstag und Bekanntmachung des Hinweises auf die Patenterteilung:
**18.06.2003 Patentblatt 2003/25**

(51) Int Cl.[7]: **C07C 257/18**, C07C 259/18, C07C 255/59, C07C 271/64, C07D 295/08, A61K 31/16, A61K 31/395

(21) Anmeldenummer: **98122169.0**

(22) Anmeldetag: **26.11.1998**

(54) **N-(4-carbamimido-phenyl)-glycinamidderivate**

N-(4-carbamimido-phenyl)-glycine amide derivatives

Dérivés de N-(4-carbamimido-phényl)glycine amide

(84) Benannte Vertragsstaaten:
**AT BE CH CY DE DK ES FI FR GB GR IE IT LI LU MC NL PT SE**
Benannte Erstreckungsstaaten:
**LT LV RO SI**

(30) Priorität: **04.12.1997 EP 97121285**
**10.11.1998 EP 98121374**

(43) Veröffentlichungstag der Anmeldung:
**09.06.1999 Patentblatt 1999/23**

(73) Patentinhaber: **F. HOFFMANN-LA ROCHE AG**
**4070 Basel (CH)**

(72) Erfinder:
- **Gröbke, Katrin**
  **4056 Basel (CH)**
- **Ji, Yu-Hua**
  **San Mateo, California 94402 (US)**
- **Wallbaum, Sabine**
  **79639 Grenzach-Wyhlen (DE)**
- **Weber, Lutz**
  **79639 Grenzach-Wyhlen (DE)**
- **Ackermann, Jean**
  **4125 Riehen (CH)**

(74) Vertreter: **Witte, Hubert, Dr. et al**
**F.Hoffmann-La Roche AG**
**Patent Department (PLP),**
**124 Grenzacherstrasse**
**4070 Basel (CH)**

(56) Entgegenhaltungen:
**EP-A- 0 739 886**
**WO-A-96/37464**
**WO-A-97/30971**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 921 116 B1

Printed by Jouve, 75001 PARIS (FR)

## Beschreibung

Die Erfindung betrifft neue N-(4-Carbamimido-phenyl)-glycinamidderivate, insbesondere solche der Formel

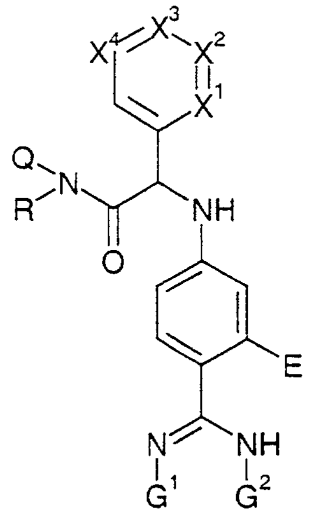

I

worin

| | |
|---|---|
| E | Wasserstoff oder OH, |
| Q | Wasserstoff oder Alkyl, |
| R | Aryl, Cycloalkyl oder durch $R^1$, $R^2$ und $R^3$ substituiertes Alkyl, |
| $R^1$ | Wasserstoff, COOH, COO-Alkyl oder Aryl, |
| $R^2$ | Wasserstoff, Aryl, Cycloalkyl oder Heteroaryl, |
| $R^3$ | Wasserstoff, Aryl oder (in einer anderen als die $\alpha$-Stellung zum N-Atom an dem die Alkylgruppe R gebunden ist) OH, Alkoxy oder gegebenenfalls geschütztes Amino, |
| N(Q,R) | durch COOH oder COO-Alkyl substituiertes Pyrrolidino,Piperidino oder 1,2,3,4-Tetrahydroisochinolin-3-yl, |
| drei von $X^1$ bis $X^4$ | unabhängig voneinander eine Gruppe $C(R^a)$, $C(R^b)$ oder $C(R^c)$ und das vierte eine Gruppe $C(R^d)$ oder N, |
| $R^a$ bis $R^d$ | unabhängig voneinander H, OH, $NO_2$, Dialkyl-amino, Halogen, Alkyl, Alkoxy, Aryloxy, Aralkyloxy, Heteroaryl-alkyloxy, Heterocyclyl-alkyloxy, COOH, COO-Alkyl, NH-$SO_2$-Alkyl, $NHSO_2$-Aryl, NHCO-Alkyl, NHCO-Aryl, NHCOO-Alkyl, NHCO-Alkyl-$NH_2$, NHCO-Alkyl-NH -G, Aralkyl-CONH, Alkyl-O-alkyl-CONH, Aryl-O-alkyl-CONH, Alkyl-COOH, Alkyl-COO-alkyl, O-Alkyl-COOH oder O-Alkyl-COO-alkyl, oder |

zwei benachbarte Gruppen $R^a$ bis $R^d$ zusammen Alkylendioxy sind,
wobei nicht mehr als drei von $R^a$ bis $R^d$ die gleiche Bedeutung haben sollen und $X^1$ nicht CCOOH oder CCOO-Alkyl sein soll,

| | |
|---|---|
| G | eine Aminoschutzgruppe und |
| eins von $G^1$ und $G^2$ | Wasserstoff und das andere Wasserstoff, Alkyl, OH, Alkoxy, Aroyl, Alkanoyl-$OCH_2$, Aroyl-$OCH_2$ |

oder eine Gruppe COO-$R^g$ oder OCO-$R^g$, $R^g$ gegebenenfalls durch Halogen, OH, Alkoxy, COOH oder COO-Alkyl substituiertes Alkyl,

sowie Hydrate oder Solvate und physiologische verwendbare Salze davon.

Ferner betrifft die Erfindung Verfahren zur Herstellung der obigen Verbindungen, pharmazeutische Präparate, die solche Verbindungen enthalten, sowie die Verwendung dieser Verbindungen bei der Herstellung von pharmazeutischen Präparaten.

Beispiele von physiologisch verwendbaren Salzen dieser Verbindungen der Formel I sind Salze mit physiologisch verträglichen Mineralsäuren, wie Salzsäure, Schwefelsäure, schweflige Säure oder Phosphorsäure; oder mit organischen Säuren, wie Methansulfonsäure, p-Toluolsulfonsäure, Essigsäure, Trifluoressigsäure, Zitronensäure, Fumarsäure, Maleinsäure, Weinsäure, Bernsteinsäure oder Salicylsäure.

Die Verbindungen der Formel I können solvatisiert, insbesondere hydratisiert sein. Die Hydratisierung kann im Zuge des Herstellungsverfahrens erfolgen oder allmählich als Folge hygroskopischer Eigenschaften einer zunächst wasserfreien Verbindung der Formel I auftreten.

Die Verbindungen der Formel I enthalten zumindest ein asymmetrisches C-Atom und können daher als Enantiomerengemisch oder als optisch reine Verbindungen vorliegen.

Im Rahmen der vorliegenden Erfindung bezeichnet "Alkyl" allein oder in Kombination, wie in COO-Alkyl, Alkoxy, Dialkyl-amino, Aralkyloxy, Heteroaryl-alkyloxy, Heterocyclyl-alkyloxy, $NHSO_2$-Alkyl, NHCO-Alkyl, Aralkyl-CONH, Alkyl-O-alkyl-CONH, Alkyl-COOH, CCOO-Alkyl, Alkanoyl u. dgl., eine bis zu 6, vorzugsweise bis zu 4 C-Atome enthaltende geradkettige oder verzweigte Gruppe. Beispiele davon sind Methyl, Ethyl und Butyl. "Aryl" allein oder in Kombination, wie in Aryloxy, Aralkyl, $NHSO_2$-Aryl, NHCO-Aryl, Aralkyl-CONH, Aryl-O-alkyl-CONH, Aroyl u.dgl., bezeichnet Gruppen, wie Phenyl oder Naphthyl, die substituiert sein können, z.B. durch Halogen, wie Brom, Fluor oder Chlor; Alkoxy, wie Methoxy, Ethoxy oder Propoxy; Alkylendioxy, wie Methylendioxy; Hydroxy, Nitro, Amino, Amidino, Sulfamoyl, Phenyl, Phenoxy, COOH oder COO-Alkyl, wie $COOCH_3$ oder $COOC_2H_5$. Beispiele von Arylgruppen sind Phenyl und Amidinophenyl, von Aralkylgruppen: Benzyl, Phenethyl, Naphthylmethyl, Mono- oder Dimethoxy-benzyl, Aminobenzyl oder Nitrobenzyl, von Aryloxygruppen: Phenoxy oder Methoxycarbonyl-phenoxy, von Aralkyloxygruppen: Benzyloxy, Nitrobenzyloxy, Bromobenzyloxy, Dichloro-benzyloxy, Methoxy-benzyloxy, Phenoxy-benzyloxy, Phenethoxy oder Biphenylyl-methoxy. "Cycloalkyl-alkyl" bezeichnet über eine Alkylgruppe gesättigte Gruppen mit 3 bis 7 C-Atomen. Ein Beispiel davon ist Cyclopropylmethyl. "Heteroaryl-alkyl" allein oder in "Heteroaryl-alkyloxy" bezeichnet eine über eine Alkyl- bzw. Alkoxygruppe gebundene 5- bis 10-gliedrige aromatische Gruppe, die aus ein oder zwei Ringen besteht und ein oder mehrere N-Atome enthält. Beispiele davon sind Imidazolylethyl und Indolylethyl bzw. Pyridinylmethoxy, Indolylmethoxy, Quinolinylmethoxy oder Isochinolinylmethoxy. "Heterocyclyl-alkyloxy" bezeichnet eine über eine Alkoxygruppe gebundene 5- bis 10-gliedrige gesättigte oder ungesättigte nicht-aromatische Gruppe, die aus ein oder zwei Ringen besteht und ein oder mehrere Heteroatome N, O und/oder S enthält. Beispiele davon sind Morpholinylethoxy, Thiomorpholinylethoxy und Tetrahydroquinolinylmethoxy. Beispiele von Gruppen COO-Alkyl, $NHSO_2$-Alkyl und $NHSO_2$-Aryl sind $COOCH_3$, $NHSO_2CH_3$ bzw. $NHSO_2C_6H_5$. Ein Beispiel einer Alkylendioxygruppe ist Methylendioxy.

Bevorzugte Verbindungen der Formel I sind diejenigen, worin $R^1$ Aryl, insbesondere Phenyl oder Amidinophenyl; Aralkyl, insbesondere Benzyl, Phenethyl, Naphthylmethyl, Mono- oder Dimethoxy-benzyl, Aminobenzyl oder Nitrobenzyl; Benzhydryl; Cycloalkyl-alkyl, insbesondere Cyclopropylmethyl; oder Heteroaryl-alkyl, insbesondere Imidazolylethyl oder Indolylethyl ist.

Bevorzugte Verbindungen der Formel I sind ferner diejenigen,

worin Q Wasserstoff oder Methyl, und/oder

worin R Aryl, wie Phenyl, Amidinophenyl oder Carboxyphenyl, oder

worin R durch $R^1$, $R^2$ und $R^3$ substituiertes Alkyl ist und

worin $R^1$ eine Gruppe COOH oder COO-Alkyl, $R^2$ Aryl oder H, und $R^3$ Aryl, H, Amino oder geschütztes Amino, oder

worin $R^1$ H, $R^2$ Aryl oder Heteroaryl und $R^3$ H oder OH ist.

Bevorzugte durch $R^1$, $R^2$ und $R^3$ substituierte Alkylgruppen R sind Methyl, Ethyl, Propyl und Isopropyl.

Bevorzugte Arylgruppen R, $R^1$, $R^2$ oder $R^3$ sind durch COOH oder COO-Alkyl, wie $COOCH_3$ oder $COOC_2H_5$; $NO_2$, $NH_2$, $SO_2NH_2$ oder ein oder zwei Gruppen OH oder Alkoxy, wie $OCH_3$ oder $OC_2H_5$, substituiertes Phenyl.

Bevorzugte Cycloalkylgruppen R oder $R^2$ sind gegebenenfalls durch COOH oder COO-Alkyl substituiertes Cyclopentyl.

Bevorzugte Heteroarylgruppen $R^2$ sind Imidazolyl, Indolyl und Pyridinyl.

Bevorzugte COO-Alkylgruppen $R^1$ sind $COOCH_3$ und $COOC_2H_5$.

Eine bevorzugte geschützte Aminogruppe $R^3$ ist NH-Boc (t-Butoxycarbonylamino).

Bevorzugte Verbindungen der Formel I sind ferner diejenigen, worin eins von $G^1$ oder $G^2$ eine Gruppe OH oder COO-Alkyl, wie $COOCH_3$ oder $COOC_2H_5$ ist.

Bevorzugte Verbindungen der Formel I sind ferner diejenigen,

worin $X^1$ eine Gruppe $C(R^a)$, in der $R^a$ H, OH, $NO_2$, Halogen, insbesondere Fluor; Alkoxy, insbesondere Methoxy; $NHSO_2$-Alkyl oder $NHSO_2$-Aryl, insbesondere Methansulfonylamino bzw. Phenylsulfonylamino und/oder

worin $X^2$ eine Gruppe $C(R^b)$, in der $R^b$ H, OH, $NO_2$, Alkyl, insbesondere Methyl; Alkoxy, insbesondere Methoxy, Ethoxy oder Butoxy; Aryloxy, insbesondere Phenoxy; Aralkyloxy, insbesondere Benzyloxy, und/oder

worin $X^3$ eine Gruppe $C(R^c)$, in der $R^c$ H, OH, $NO_2$, Dialkylamino, insbesondere Dimethylamino; Halogen, insbesondere Brom; Alkyl, insbesondere Methyl; Alkoxy, insbesondere Methoxy, Ethoxy oder Propoxy; Aryloxy, insbesondere Methoxycarbonyl-phenoxy; Aralkyloxy, insbesondere Benzyloxy, Nitrobenzyloxy, Bromobenzyloxy, Dichloro-benzyloxy, Methoxy-benzyloxy, Phenoxy-benzyloxy, Phenethoxy oder Biphenylyl-methoxy; Heteroaryl-alkyloxy, insbesondere Pyridinyl-methoxy, Indolylmethoxy, Quinolinylmethoxy oder Isochinolinylmethoxy; oder Heterocyclyl-alkyloxy, insbesondere Morpholinylethoxy, Thiomorpholinylethoxy oder Tetrahydroquinolinylmethoxy ist, oder

worin $R^b$ und $R^c$ zusammen eine Alkylendioxygruppe, insbesondere die Methylendioxygruppe, bilden und/oder

worin $X^4$ eine Gruppe $C(R^d)$, in der $R^d$ H, $NO_2$, Alkoxy, insbesondere Methoxy; oder Aralkyloxy, insbesondere Benzyloxy ist.

Bevorzugte Verbindungen der Formel I sinf ferner diejenigen, worin $X^1$ bis $X^4$ unabhängig voneinander eine Gruppe $C(R^a)$ bis $C(R^d)$ in der $R^a$ bis $R^d$ NHCO-Alkyl, wie NH-Acetyl, NHCO-Aryl, wie NH-Benzoyl, NHCOO-Alkyl, wie $NHCOOCH_3$, NHCO-Alkyl- (gegebenenfalls geschütztes Amino), wie $NHCOCH_2NH_2$, $NHCOCH_2CH_2NH_2$ und $NHCOCH_2$NH-Boc; Aralkyl-CONH, wie Benzyl-CONH, Alkyl-O-alkyl-CONH, wie $CH_3OCH_2CONH$, Alkyl-COOH, wie $CH_2CH_2COOH$, O-Alkyl-COOH, wie $O(CH_2)_{1\ oder\ 3}COOH$ und $OCH(CH_3)COOH$, Aryloxy, wie $OC_6H_4COOH$ und Aralkyloxy, wie $OCH_2C_6H_4COOH$, wobei an Stelle einer Gruppe COOH eine Gruppe COO-Alkyl, wie COO($CH_3$ oder $C_2H_5$) vorliegen kann.

Bevorzugte Verbindungen der Formel I sind ferner diejenigen, worin eins von $X^1$ bis $X^4$ ein N-Atom und die drei anderen unabhängig voneinander eine Gruppe $C(R^a)$, $C(R^b)$ oder $C(R^c)$ sind, insbesondere worin eins von $R^a$, $R^b$ und $R^c$ für H und die zwei anderen für nieder-Alkoxy, wie Methoxy oder Ethoxy, stehen.

Bevorzugte Verbindungen der Formel I sind ferner diejenigen, worin eins von $G^1$ und $G^2$ Wasserstoff und das andere für Aroyl, insbesondere gegebenenfalls durch Halobenzoyl, wie Fluorobenzoyl, Alkyl-$COOCH_2$-benzoyl oder Phenyl-$COOCH_2$-benzoyl, oder für Haloalkyl-OCO, wie Trichlorethyl-OCO steht.

Besonders bevorzugt sind die Verbindungen, worin E, $G^1$, $G^2$ und Q Wasserstoff sind, und / oder

worin R durch $R^1$, $R^2$ und $R^3$ substituiertes Alkyl, insbesondere Methyl oder Ethyl, insbesondere worin $R^1$ Aryl, speziell Phenyl, oder COOH, $R^2$ Wasserstoff oder Aryl, speziell Phenyl, und $R^3$ Wasserstoff ist, und / oder

worin $X^1$ bis $X^4$ eine Gruppe $C(R^a)$ bis $C(R^d)$, insbesondere worin $R^a$ H, Aralkyloxy, speziell Carboxy-benzyloxy; $NHSO_2$-Aryl, speziell Phenylsulfonylamino; Aralkyl-CONH, speziell Phenylacetylamino, oder O-Alkyl-COOH, speziell Carboxymethoxy, $R^b$ H oder Alkoxy, speziell Methoxy oder Ethoxy, $R^c$ H, Alkoxy, speziell Methoxy, oder Aralkyloxy, speziell Benzyloxy, und $R^d$ H oder Alkoxy, speziell Methoxy ist.

Beispiele von solchen Verbindungen sind:

(R,S)-N-Benzyl-2-(4-benzyloxy-3-ethoxy-phenyl)-2-(4-carbamimidoylphenylamino)-acetamid,
2-(2-Phenylsulfonylamino-4-benzyloxy-5-methoxy-phenyl)-N-benzyl-2-(4-carbamimidoyl-phenylamino)-acetamid, und / ferner
(RS)-[2-(4-Benzyloxy-3-methoxy-phenyl)-2-(4-carbamimidoyl-phenylamino)-acetylamino]-diphenyl-essigsäure,
(RS)- und (SR)-3-[(RS)-2-(4-Benzyloxy-3-methoxy-phenyl)-2-(4-carbamimidoylphenylamino)-acetylamino] -3-phenyl-propionsäure,
(S)-[(R)-2-(4-Benzyloxy-3-methoxy-phenyl)-2-(4-carbamimidoyl-phenylamino)-acetylamino]-phenyl-essigsäure,
(S)-[(S)-2-(4-Carbamimidoyl-phenylamino)-2-(3,5-dimethoxy-phenyl)-acetylamino]-phenyl-essigsäure,

(RS)-3-[2-[Benzylcarbamoyl-(4-carbamimidoyl-phenylamino)-methyl]-4,5-dimethoxy-phenoxymethyl]-benzoesäure,
(RS)-[2-[Benzylcarbamoyl-(4-carbamimidoyl-phenylamino)-methyl]-4,5-dimethoxy-phenoxy]-essigsäure,
(S)-[(R)-2-(2-Phenylsulfonylamino-4-benzyloxy-5-methoxy-phenyl)-2-(4-carbamimidoyl-phenylamino)-acetylamino]-phenylessigsäure,
(S)-[(S)-2-(2-Phenylsulfonylamino-4-benzyloxy-5-methoxy-phenyl)-2-(4-carbamimidoyl-phenylamino)-acetylamino]-phenyl-essigsäure,
(S)-[(R)-2-(4-Benzyloxy-5-methoxy-2-phenylacetylamino-phenyl)-2-(4-carbamimidoyl-phenylamino)-acetylamino]-penyl-essigsäure
(S)-[(S)-2-(4-Benzyloxy-5-methoxy-2-phenylacetylamino-phenyl)-2-(4-carbamimidoyl-phenylamino)-acetylamino]-penyl-essigsäure.

Weitere Beispiele von Verbindungen der Formel I sind die folgenden:

(S)-2-[(R)-2-(4-Benzyloxy-3-methoxy-phenyl)-2-(4-carbamimidoyl-phenylamino)-acetylamino]-propionsäure,

(S)-2-[(S)-2-(4-Benzyloxy-3-methoxy-phenyl)-2-(4-carbamimidoyl-phenylamino)-acetylamino]-propionsäure,

(S)-2-[(R)-2-(4-Benzyloxy-3-methoxy-phenyl)-2-(4-carbamimidoyl-phenylamino)-acetylamino] -3-phenyl-propionsäure,

(S)-2-[(S)-2-(4-Benzyloxy-3-methoxy-phenyl)-2-(4-carbamimidoyl-phenylamino)-acetylamino]-3-phenyl-propionsäure,

(S)-2-[(R)-2-(4-Benzyloxy-3-methoxy-phenyl)-2-(4-carbamimidoyl-phenylamino)-acetylamino]-3-(4-hydroxy-phenyl)-propionsäure,

(S)-2-[(S)-2-(4-Benzyloxy-3-methoxy-phenyl)-2-(4-carbamimidoyl-phenylamino)-acetylamino]-3-(4-hydroxy-phenyl)-propionsäure,

(S)-1-[(R)- und -[(S)-(4-Benzyloxy-3-methoxy-phenyl)-(4-carbamimidoylphenylamino)-acetyl]-pyrrolidin-2-carbonsäure,

(RS)-2-[2-(4-Benzyloxy-3-methoxy-phenyl)-2-(4-carbamimidoyl-phenylamino)-acetylamino]-2-methyl-propionsäure,

(S)- [[(R)-(4-Benzyloxy-3-methoxy-phenyl)-(4-carbamimidoyl-phenylamino)-acetyl]-methyl-amino]-phenyl-essigsäure,

(S)-[[(S)-(4-Benzyloxy-3-methoxy-phenyl)-(4-carbamimidoyl-phenylamino)-acetyl]-methyl-amino] -phenyl-essigsäure,

(RS)- und (SR)-1-[(RS)-(4-Benzyloxy-3-methoxy-phenyl)-(4-carbamimidoylphenylamino)-acetyl]-pyrrolidin-3-carbonsäure,

(RS)- oder (SR)-2-[(RS)-(4-Benzyloxy-3-methoxy-phenyl}-(4-carbamimidoylphenylamino)-acetyl]-1,2,3,4-tetrahydro-isochinolin-3-carbonsäure,

(RS)-1-[2-(4-Benzyloxy-3-methoxy-phenyl)-2-(4-carbamimidoyl-phenylamino)-acetylamino]-cyclopentancarbonsäure,

(S)-2-[(R)-2-(4-Benzyloxy-3-methoxy-phenyl)-2-(4-carbamimidoyl-phenylamino)-acetylamino]-4-tert-butoxycarbonylamino-buttersäure,

(S)-2-[(S)-2-(4-Benzyloxy-3-methoxy-phenyl)-2-(4-carbamimidoyl-phenylamino)-acetylamino]-4-tert-butoxycarbonylamino-buttersäure,

(S)-2-[(R)-2-(4-Benzyloxy-3-methoxy-phenyl)-2-(4-carbamimidoyl-phenylamino)-acetylamino]-3-tert-butoxycarbonylamino-propionsäure,

(S)-2-[(S)-2-(4-Benzyloxy-3-methoxy-phenyl)-2-(4-carbamimidoyl-phenylamino)-acetylamino]-3-tert-butoxycarbonylamino-propionsäure,

(RS)-2-[2-(4-Benzyloxy-3-methoxy-phenyl)-2-(4-carbamimidoyl-phenylamino)-acetylamino]-benzoesäure,

(RS)-3-[2-(4-Benzyloxy-3-methoxy-phenyl)-2-(4-carbamimidoyl-phenylamino)-acetylamino]-benzoesäure,

(RS)-4-[2-(4-Benzyloxy-3-methoxy-phenyl)-2-(4-carbamimidoyl-phenylamino)-acetylamino]-benzoesäure,

(E)- und/oder (Z)-(S)-2-[(R)- and -[(S)-2-(4-Benzyloxy-3-methoxy-phenyl)-2-[4-(Nhydroxycarbamimidoyl)-phenylamino]-acetylamino]-propionsäure,

(E)- und/oder (Z)-(S)-2-[(R)-2-(4-Benzyloxy-3-methoxy-phenyl)-2-[4-(Nhydroxycarbamimidoyl)-phenylamino]-acetylamino]-3-phenyl-propionsäure,

(E)- und/oder (Z)-(S)-2- [(S)-2-(4-Benzyloxy-3-methoxy-phenyl)-2-[4-(Nhydroxycarbamimidoyl)-phenylamino]-acetylamino]-3-phenyl-propionsäure,

(E)- und/oder (Z)-(S)-2-[(R)- und -[(S)-2-(4-Benzyloxy-3-methoxy-phenyl)-2-[4-(Nhydroxycarbamimidoyl)-phenylamino]-acetylamino]-3-(4-hydroxy-phenyl)-propionsäure,

(E)- und/oder (Z)-(S)-1-[(R)- und -[(S)-(4-Benzyloxy-3-methoxy-phenyl)-[4-(Nhydroxycarbamimidoyl)-phenylamino]-acetyl]-pyrrolidin-2-carbonsäure,

(E)- und/oder (Z)-(RS)-2-[2-(4-Benzyloxy-3-methoxy-phenyl)-2-[4-(Nhydroxycarbamimidoyl)-phenylamino]-acetylamino]-2-methyl-propionsäure,

(E)- und/oder (Z)-(S)-[[(R)- und -[[(S)-(4-Benzyloxy-3-methoxy-phenyl)-[4-(Nhydroxycarbamimidoyl)-phenylamino]-acetyl]-methyl-amino]-phenyl-essigsäure,

(E)- und/oder (Z)-(RS)- und -(SR)-1-[(RS)-(4-Benzyloxy-3-methoxy-phenyl)-[4-(Nhydroxycarbamimidoyl)-phenylamino]-acetyl]-pyrrolidin-3-carbonsäure,

(E)- und/oder (Z)-(RS)- und -(SR)-2-[(RS)-(4-Benzyloxy-3-methoxy-phenyl)-[4-(Nhydroxycarbamimidoyl)-phenylamino]-acetyl]-1,2,3,4-tetrahydro-isochinolin-3-carbonsäure,

(E)- und/oder (Z)-(RS)-1-[2-(4-Benzyloxy-3-methoxy-phenyl)-2-[4-(Nhydroxycarbamimidoyl)-phenylamino]-acetylamino]-cyclopentancarbonsäure,

(E)- und/oder (Z)-(RS)-[2-(4-Benzyloxy-3-methoxy-phenyl)-2-[4-(Nhydroxycarbamimidoyl)-phenylamino]-acetylamino]-diphenyl-essigsäure,

(S)-2-[(R)- und -[(S)-2-(4-Benzyloxy-3-methoxy-phenyl)-2-[4-[(E)- und/oder -[(Z)-N-hydroxycarbamimidoyl]-phenylamino]-acetylamino]-4-tert-butoxycarbonylaminobuttersäure,

(S)-2-[(R)- und -[(S)-2-(4-Benzyloxy-3-methoxy-phenyl)-2-[4-[(E)- und/oder -[(Z)-N-hydroxycarbamimidoyl]-phenylamino]-acetylamino]-3-tert-butoxycarbonylaminopropionsäure.

(E)- und/oder (Z)-(RS)-3-[2-(4-Benzyloxy-3-methoxy-phenyl)-2-[4-(Nhydroxycarbamimidoyl)-phenylamino]-acetylamino]-benzoesäure,

(E)- und/oder (Z)-(RS)-4-[2-(4-Benzyloxy-3-methoxy-phenyl)-2-[4-(Nhydroxycarbamimidoyl)-phenylamino]-acetylamino]-benzoesäure.

(E)- und/oder (Z)-(RS)- und -(SR)-3-[(RS)-2-(4-Benzyloxy-3-methoxy-phenyl)-2-[4-(N-hydroxycarbamimidoyl)-phenylamino]-acetylamino]-3-phenyl-propionsäure,

(S)-4-Amino-2-[(R)- und - [(S)-2-(4-benzyloxy-3-methoxy-phenyl)-2-(4-carbamimidoyl-phenylamino)-acetylamino]-buttersäure,

(S)-3-Amino-2-[(R)- und -[(S)-2-(4-benzyloxy-3-methoxy-phenyl)-2-(4-carbamimidoyl-phenylamino)-acetylamino]-propionsäure,

(E)- und/oder (Z)-(S)-[(R)- oder -[(S)-2-(4-Benzyloxy-3-methoxy-phenyl)-2-[4-(Nhydroxycarbamimidoyl)-phenylamino]-acetylamino]-phenyl-essigsäure,

(S)-[(S)-2-(4-Benzyloxy-3-methoxy-phenyl)-2-(4-carbamimidoyl-phenylamino)-acetylamino]-phenyl-essigsäure,

(R)-[(R)-2-(4-Benzyloxy-3-methoxy-phenyl)-2-(4-carbamimidoyl-phenylamino)-acetylamino]-phenyl-essigsäure,

(R)-[(S)-2-(4-Benzyloxy-3-methoxy-phenyl)-2-(4-carbamimidoyl-phenylamino)-acetylamino]-phenyl-essigsäure,

(E)- und/oder (Z)-(S)-3-(4-Amino-phenyl)-2-[(R)- und -[(S)-2-(4-benzyloxy-3-methoxy-phenyl)-2-[4-(N-hydroxy-carbamimidoyl)-phenylamino]-acetylamino]-propionsäure-ethylester,

(S)-3-(4-Amino-phenyl)-2-[(R)- und -[(S)-2-(4-benzyloxy-3-methoxy-phenyl)-2-(4-carbamimidoyl-phenylamino)-acetylamino]-propionsäure-ethylester,

(S)-3-(4-Amino-phenyl)-2-[(R)-2-(4-benzyloxy-3-methoxy-phenyl)-2-(4-carbamimidoyl-phenylamino)-acetylamino]-propionsäure,

(S)-3-(4-Amino-phenyl)-2-[(S)-2-(4-benzyloxy-3-methoxy-phenyl)-2-(4-carbamimidoyl-phenylamino)-acetylamino]-propionsäure,

(S)-[(R)-2-(4-Carbamimidoyl-phenylamino)-2-(3,5-dimethoxy-phenyl)-acetylamino]-phenyl-essigsäure,

(RS)-2-(4-Benzyloxy-3-methoxy-phenyl)-2-(4-carbamimidoyl-phenytamino)-N-[2-(3,4-dihydroxy-phenyl)-ethyl]-acetamid,

(RS)-N-Benzyl-2-(4-benzyloxy-3-methoxy-phenyl)-2-(4-carbamimidoylphenylamino)-N-methyl-acetamid,

(RS)-2-(4-Benzyloxy-3-methoxy-phenyl)-2-(4-carbamimidoyl-phenylamino)-N-[2-(4-sulfamoyl-phenyl)-ethyl]-acetamid,

(RS)-2-(4-Benzyloxy-3-methoxy-phenyl)-2-(4-carbamimidoyl-phenylamino)-N-(2-pyridin-2-yl-ethyl)-acetamid,

(RS)-N-[2-(4-Amino-phenyl)-ethyl]-2-(4-benzyloxy-3-methoxy-phenyl)-2-(4-carbamimidoyl-phenylamino)-acetamid,

(R)-2-(4-Benzyloxy-3-methoxy-phenyl)-2-(4-carbamimidoyl-phenylamino)-N-[(R)-2-hydroxy-1-phenyl-ethyl]-acetamid,

(S)-2-(4-Benzyloxy-3-methoxy-phenyl)-2-(4-carbamimidoyl-phenylamino)-N-[(R)-2-hydroxy-1-phenyl-ethyl]-acetamid,

(R)-2-(4-Benzyloxy-3-methoxy-phenyl)-2-(4-carbamimidoyl-phenylamino)-N-[(S)-2-hydroxy-1-phenyl-ethyl]-acetamid,

(S)-2-(4-Benzyloxy-3-methoxy-phenyl)-2-(4-carbamimidoyl-phenylamino)-N-[(S)-2-hydroxy-1-phenyl-ethyl]-acetamid,

(R)-2-(4-Benzyloxy-3-methoxy-phenyl)-2-(4-carbamimidoyl-phenylamino)-N-[(R)-1-phenyl-ethyl]-acetamid,

(S)-2-(4-Benzyloxy-3-methoxy-phenyl)-2-(4-carbamimidoyl-phenylamino)-N-[(R)-1-phenyl-ethyl]-acetamid,

(R)-2-(4-Benzyloxy-3-methoxy-phenyl)-2-(4-carbamimdoyl-phenylamino)-N-[(S)-1-phenyl-ethyl]-acetamid,

(S)-2-(4-Benzyloxy-3-methoxy-phenyl)-2-(4-carbamimidoyl-phenylamino)-N-[(S)-1 -phenyl-ethyl]-acetamid,

(E)-und/oder (Z)-(RS)-N-Benzyl-2-(4-benzyloxy-3-methoxy-phenyl)-2-[4-(Nhydroxycarbamimidoyl)-phenylamino] -N-methyl-acetamid,

(E)- und/oder (Z)-(RS)-2-(4-Benzyloxy-3-methoxy-phenyl)-2-[4-(Nhydroxycarbamimidoyl)-phenylamino] -N-[2-(4-sulfamoyl-phenyl)-ethyl]-acetamid,

(E)-und/oder(Z)-(RS)-2-(4-Benzyloxy-3-methoxy-phenyl)-2-[4-(Nhydroxycarbamimidoyl)-phenylamino]-N-(2-pyridin-2-yl-ethyl)-acetamid,

(E)-und/oder(Z)-(RS)-2-(4-Benzyloxy-3-methoxy-phenyl)-2-[4-(Nhydroxycarbamimidoyl)-phenylamino]-N-[2-(4-nitro-phenyl)-ethyl]-acetamid,

(E)- und/oder (Z)-(R)-2-(4-Benzyloxy-3-methoxy-phenyl)-2-[4-(Nhydroxycarbamimidoyl)-phenylamino]-N-[(R) -(2-hydroxy-1-phenyl-ethyl]-acetamid,

(E)- und/oder (Z)-(S)-2-(4-Benzyloxy-3-methoxy-phenyl)-2-[4-(Nhydroxycarbamimidoyl)-phenylamino]-N-[(R) -(2-hydroxy-1-phenyl-ethyl]-acetamid,

(E)- und/oder (Z)-(R)-2-(4-Benzyloxy-3-methoxy-phenyl)-2-[4-(Nhydroxycarbamimidoyl)-phenylamino]-N-[(S)-2-hydroxy-1-phenyl-ethyl]-acetamid,

(E)- und/oder (Z)-(S)-2-(4-Benzyloxy-3-methoxy-phenyl)-2-[4-(Nhydroxycarbamimidoyl)-phenylamino]-N-[(S)-2-hydroxy-1-phenyl-ethyl]-acetamid,

(E)- und/oder (Z)-(R)-2-(4-Benzyloxy-3-methoxy-phenyl)-2-[4-(Nhydroxycarbamimidoyl)-phenylamino]-N-[(R)-1-phenyl-ethyl]-acetamid,

(E)- und/oder (Z)-(S)-2-(4-Benzyloxy-3-methoxy-phenyl)-2-[4-(Nhydroxycarbamimidoyl)-phenylamino]-N-[(R)-1-phenyl-ethyl]-acetamid,

(E)- und/oder (Z)-(R)-2-(4-Benzyloxy-3-methoxy-phenyl)-2-[4-(Nhydroxycarbamimidoyl)-phenylamino]-N-[(S)-1-phenyl-ethyl]-acetamid,

(E)- und/oder (Z)-(S)-2-(4-Benzyloxy-3-methoxy-phenyl)-2-[4-(Nhydroxycarbamimidoyl)-phenylamino]-N-[(S)-1-phenyl-ethyl]-acetamid.

(RS)-N-[2-[Benzylcarbamoyl-(4-carbamimidoyl-phenylamino)-methyl]-5-benzyloxy-4-methoxy-phenyl]-benzamid-acetat,

(RS)-[2-[Benzylcarbamoyl-(4-carbamimidoyl-phenylamino)-methyl]-5-benzyloxy-4-methoxy-phenyl]-carbamidsäure-methylester-acetat,

(RS)-2-(2-Acetylamino-4-benzyloxy-5-methoxy-phenyl)-N-benzyl-2-(4-carbamimidoyl-phenylamino)-acetamid,

(RS)-N-Benzyl-2-(4-benzyloxy-5-methoxy-2-phenylacetylamino-phenyl)-2-(4-carbamimidoyl-phenylamino)-acetamid,

(RS)-N-Benzyl-2-(4-carbamimidoyl-phenylamino)-2-(4,5-dimethoxy-2-phenylacetylamino-phenyl)-acetamid,

(RS)-[2-[Benzylcarbamoyl-(4-carbamimidoyl-phenylamino)-methyl]-4,5-dimethoxy-phenyl]-carbamidsäure-methylester,

(RS)-N-[2-[Benzylcarbamoyl-(4-carbamimidoyl-phenylamino)-methyl]-4,5-dimethoxy-phenyl]-benzamid,

(RS)-N-[2-[Benzylcarbamoyl-(4-carbamimidoyl-phenylamino)-methyl]-4-methoxyphenyl]-benzamid,

(RS)-2-(2-Benzenesulfonylamino-5-methoxy-phenyl)-N-benzyl-2-(4-carbamimidoyl-phenylamino)-acetamid,

(RS)-N-(2-{Benzylcarbamoyl-[4-(N-hydroxycarbamimidoyl)-phenylamino]-methyl}-5-benzyloxy-4-methoxy-phenyl)-benzamid,

(RS)-(2-{Benzylcarbamoyl-[4-(N-hydroxycarbamimidoyl)-phenylamino]-methyl}-5-benzyloxy-4-methoxy-phenyl)-carbamidsäure-methylester,

(E)- und/oder (Z)-(RS)-2-(2-Acetylamino-4-benzyloxy-5-methoxy-phenyl)-N-benzyl-2-[4-(N-hydroxycarbamimidoyl)-phenylamino]-acetamid,

(E)- und/oder(Z)-(RS)-N-Benzyl-2-(4-benzyloxy-5-methoxy-2-phenylacetylaminophenyl)-2-[4-(N-hydroxycarbamimidoyl)-phenylamino]-acetamid,

(E)- und/oder (Z)-(RS)-N-Benzyl-2-(4,5-dimethoxy-2-phenylacetylamino-phenyl)-2-[4-(N-hydroxycarbamimidoyl)-phenylamino]-acetamid,

(E)- und/oder (Z)-(RS)-[2-[Benzylcarbamoyl-[4-(N-hydroxycarbamimidoyl)-phenylamino]-methyl]-4,5-dimethoxy-phenyl]-carbamidsäure-methylester,

(E)- und/oder (Z)-(RS)-N-[2-[Benzylcarbamoyl-[4-(N-hydroxy-carbamimidoyl)-phenylamino]-methyl]-4,5-dimethoxy-phenyl)-benzamid,

(E)- und/oder (Z)-(RS)-N-[2-[Benzylcarbamoyl-[4-(N-hydroxycarbamimidoyl)-phenylamino]-methyl]-4-methoxy-phenyl]-benzamid,

(E)- und/oder (Z)-(RS)-2-(2-Phenylsulfonylamino-5-methoxy-phenyl)-N-benzyl-2-[4-(N-hydroxycarbamimidoyl)-phenylamino]-acetamid,

(RS)-N-Benzyl-2-[4-benzyloxy-5-methoxy-2-(2-methoxy-acetylamino)-phenyl]-2-[4-(N-hydroxycarbamimidoyl)-phenylamino]-acetamid,

(RS)-N-Benzyl-2-[4-benzyloxy-5-methoxy-2-(2-methoxy-acetylamino)-phenyl]-2-(4-carbamimidoyl-phenylamino)-acetamid,

(RS)-[(2-{Benzylcarbamoyl-[4-(N-hydroxycarbamimidoyl)-phenylamino]-methyl}-5-benzyloxy-4-methoxy-phenylcarbamoyl)-methyl]-carbamidsäure-tert-butyl ester,

(RS)-2-[2-(2-Amino-acetylamino)-4-benzyloxy-5-methoxy-phenyl]-N-benzyl-2-(4-carbamimidoyl-phenylamino)-acetamid,

(RS)-4-[2-[Benzylcarbamoyl-(4-carbamimidoyl-phenylamino)-methyl]-4,5-dimethoxy-phenoxymethyl]-benzoesäure,

(RS)-4-[2-[Benzylcarbamoyl-(4-carbamimidoyl-phenylamino)-methyl]-4,5-dimethoxy-phenoxy]-butersäure,

(RS)-[2- [Benzylcarbamoyl-(4-carbamimidoyl-phenylamino)-methyl]-4-methoxyphenoxy]-essigsäure,

(RS)-[2-[Benzylcarbamoyl-(4-carbamimidoyl-phenylamino)-methyl]-4,6-dimethylphenoxy]-essigsäure,

(E)- und/oder (Z)-(RS)-3-[2-[Benzylcarbamoyl-[4-(N-hydroxycarbamimidoyl)-phenylamino]-methyl]-4,5-dimethoxy-phenoxymethyl]-benzoesäure,

(E)- und/oder (Z)-(RS)-[2-[Benzylcarbamoyl-[4-(N-hydroxycarbamimidoyl)-phenylamino]-methyl]-4,5-dimethoxy-phenoxy]-essigsäure,

(E)- und/oder (Z)-(RS)-4-[2-[Benzylcarbamoyl-[4-(N-hydroxycarbamimidoyl)-phenylaminol-methyl]-4,5-dimethoxy-phenoxymethyl]-benzoesäure,

(E)- und/oder (Z)-(RS)-4-[2-[Benzylcarbamoyl-[4-(N-hydroxycarbamimidoyl)-phenylamino]-methyl]-4,5-dimethoxy-phenoxy]-buttersäure,

(E)- und/oder (Z)-(RS)-[2-[Benzylcarbamoyl-[4-(N-hydroxycarbamimidoyl)-phenylamino]-methyl]-4-methoxy-phenoxy]-essigsäure,

(E)- und/oder (Z)-(RS)-[2-[Benzylcarbamoyl-[4-(N-hydroxycarbamimidoyl)-phenylamino]-methyl]-4,6-dimethyl-phenoxy]-essigsäure,

(E)- und/oder (Z)-(R)-2-[2-[(R)-Benzylcarbamoyl-[4-(N-hydroxy-carbamimidoyl)-phenylamino]-methyl] -4,5-dimethoxy-phenoxy]-propionsäure,

(E)- und/oder (Z)-(R)-2-[2-[(S)-Benzylcarbamoyl-[4-(N-hydroxy-carbamimidoyl)-phenylamino]-methyl]-4,5-dimethoxy-phenoxy]-propionsäure,

(R)-2-[2-[(R)-Benzylcarbamoyl-(4-carbamimidoyl-phenylamino)-methyl]-4,5-dimethoxy-phenoxy]-propionsäure,

(R)-2-[2-[(S)-Benzylcarbamoyl-(4-carbamimidoyl-phenylamino)-methyl]-4,5-dimethoxy-phenoxy]-propionsäure,

(E)- und/oder (Z)-(S)-2-[2-[(R)-Benzylcarbamoyl-[4-(N-hydroxycarbamimidoyl)-phenylamino]-methyl]-4,5-dimethoxy-phenoxy]-propionsäure,

(E)- und/oder (Z)-(S)-2-[2-[(S)-Benzylcarbamoyl-[4-(N-hydroxycarbamimidoyl)-phenylamino]-methyl]-4,5-dimethoxy-phenoxy]-propionsäure,

(S)-2-[2-[(R)-Benzylcarbamoyl-(4-carbamimidoyl-phenylamino)-methyl]-4,5-dimethoxy-phenoxy]-propionsäure,

(S)-2-[2-[(S)-Benzylcarbamoyl-(4-carbamimidoyl-phenylamino)-methyl]-4,5-dimethoxy-phenoxy]-propionsäure,

(RS)-3-{4-[Benzylcarbamoyl-(4-carbamimidoyl-phenylamino)-methyl]-2-methoxyphenoxy}-benzoesäure,

(RS)-2-{4-[Benzylcarbamoyl-(4-carbamimidoyl-phenylamino)-methyl]-2-methoxyphenoxy}-benzoesäure,

(RS)-4-{4-[Benzylcarbamoyl-(4-carbamimidoyl-phenylamino)-methyl]-2-methoxyphenoxy}-benzoesäure.

(RS)-3-(4-{Benzylcarbamoyl-[4-(N-hydroxycarbamimidoyl)-phenylamino]-methyl}-2-methoxy-phenoxy)-benzoesäure,

(RS)-4-(4-{Benzylcarbamoyl-[4-(N-hydroxycarbamimidoyl)-phenylamino]-methyl}-2-methoxy-phenoxy)-benzoesäure,

(RS)-5-{Benzylcarbamoyl-[4-(N-hydroxycarbamimidoyl)-phenylamino]-methyl}-2,3-dimethoxy-benzoesäure-methylester,

(RS)-5-[Benzylcarbamoyl-(4-carbamimidoyl-phenylamino)-methyl]-2,3-dimethoxy-benzoesäure-methylester,

(RS)-5-[Benzylcarbamoyl-(4-carbamimidoyl-phenylamino)-methyl]-2,3-dimethoxy-benzoesäure,

(RS)-2-(4-{Benzylcarbamoyl-[4-(N-hydroxycarbamimidoyl)-phenylamino]-methyl}-2-methoxy-phenoxy)-benzoesäure,

(E)- und/oder (Z)-(RS)-3-[2-[Benzylcarbamoyl-[4-(N-hydroxycarbamimidoyl)-phenylamino]-methyl]-4,5-dimethoxy-phenyl]-propionsäure,

(RS)-3-[2-[Benzylcarbamoyl-(4-carbamimidoyl-phenylamino)-methyl]-4,5-dimethoxy-phenyl]-propionsäure,

(E)- und/oder (Z)-(RS)-2-(3-Acetylamino-phenyl)-N-benzyl-2-[4-(Nhydroxycarbamimidoyl)-phenylamino]-acetamid,

(RS)-2-(3-Acetylamino-phenyl)-N-benzyl-2-(4-carbamimidoyl-phenylamino)-acetamid,

(RS)-N-Benzyl-2-(4-carbamimidoyl-phenylamino)-2-(3-nitro-phenyl)-acetamid,

(RS)-N-Benzyl-2-(4-carbamimidoyl-phenylamino)-2-(2,6-dimethoxy-pyridin-4-yl)-acetamid,

(RS)-N-Benzyl-2-(4-carbamimidoyl-phenylamino)-2-(4,6-dimethoxy-pyridin-2-yl)-acetamid,

(RS)-N-Benzyl-2-(4-benzyloxy-3,5-dimethyl-phenyl)-2-(4-carbamimidoylphenylamino)-acetamid,

(RS)-N-Benzyl-2-(3-benzyloxy-5-propoxy-phenyl)-2-(4-carbamimidoylphenylamino)-acetamid-hydrochlorid,

(RS)-N-Benzyl-2-(3,5-bis-benzyloxy-phenyl)-2-(4-carbamimidoyl-phenylamino)-acetamid-hydrochlorid,

(RS)-N-Benzyl-2-(4-carbamimidoyl-phenylamino)-2-(2,6-diethoxy-pyridin-4-yl)-acetamid,

(RS)-N-Benzyl-2-(4-benzyloxy-3-ethoxy-phenyl)-2-[3-hydroxy-4-(Nhydroxycarbamimidoyl)-phenylamino]-acetamid,

(RS)-N-Benzyl-2-(4-benzyloxy-3-ethoxy-phenyl)-2-(4-carbamimidoyl-3-hydroxyphenylamino)-acetamid,

(S)-[(R)- und -[(S)-2-(2-Phenylsulfonylamino-4-benzyloxy-5-methoxy-phenyl)-2-[4-[(E)- und/oder -[(Z)-N-hydroxy-carbamimidoyl)-phenylamino]-acetylamino]-phenyl-essigsäure,

(S)-[(R)- und -[(S)-2-(4-Benzyloxy-5-methoxy-2-phenylacetylamino-phenyl)-2-[4-[(E)- und/oder -[(Z)-N-Hydroxycarbamimidoyl]-phenylamino]-acetylaimno]-phenyl-essigsäure,

(S)-[(R)-2-(2-Acetylamino-4-benzyloxy-5-methoxy-phenyl)-2-(4-carbamimidoylphenylamino)-acetylamino]-phenyl-essigsäure-acetat,

(S)-[(S)-2-(2-Acetylamino-4-benzyloxy-5-methoxy-phenyl)-2-(4-carbamimidoylphenylamino)-acetylamino]-phenyl-essigsäure,

(RS)-N-[Amino-(4-{[benzylcarbamoyl-(4-benzyloxy-3-ethoxy-phenyl)-methyl]-amino}-phenyl)-methylen]-4-fluoro-benzamid,

(RS)-Benzoesäure-2-{[amino-(4-{[benzylcarbamoyl-(4-benzyloxy-3-ethoxy-phenyl)-methyl]-amino}-phenyl)-methylen]-carbamoyl}-benzylester,

(RS)-[Amino-(4-{[benzylcarbamoyl-(4-benzyloxy-3-ethoxy-phenyl)-methyl]-amino}phenyl)-methylene]-carbamidsäure-2,2,2-trichloro-ethylester,

(RS)-[Amino-(4-{[benzylcarbamoyl-(4-benzyloxy-3-ethoxy-phenyl)-methyl]-amino}phenyl)-methylene]-carbamidsäure-methylester.

Die erfindungsgemässen Verbindungen der Formel I können dadurch hergestellt werden, dass man

a) einen Aldehyd der Formel

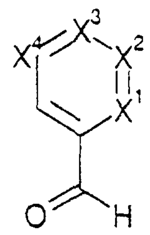

II

mit einem Isonitril der Formel $R^1NC$ und einem 4-Aminobenzamidin der Formel

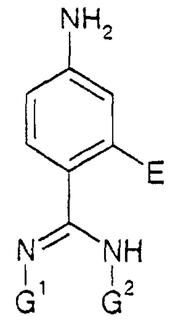

III

umsetzt oder

b) die in einem entsprechenden Nitril der nachstehenden Formel

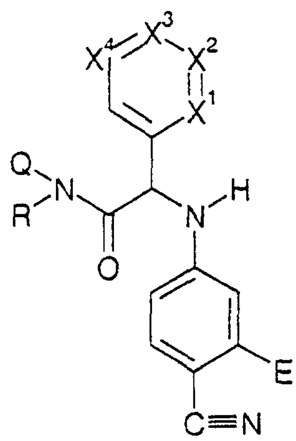

IV

enthaltene Cyanogruppe CN in eine Amidinogruppe $C(N\text{-}G^1)NH\text{-}G^2$ umwandelt,

c) gewünschtenfalls eine in einer Verbindung I enthaltene reaktionsfähige Gruppe funktionell abwandelt und

d) gewünschtenfalls eine Verbindung der Formel I in ein physiologisch verträgliches Salz überführt oder ein Salz einer Verbindung I in die freie Säure oder Base überführt.

Die Umsetzung eines substituierten Benzaldehyds der Formel II, eines Isonitrils der Formel RNC und eines 4-Amino-benzamidins der Formel III wird zweckmässig in einem Lösungsmittel, wie Methanol, Isopropanol, Ethanol, Dioxan, Tetrahydrofuran, oder einem Lösungsmittelgemisch, wie Tetrahydrofuran und Wasser oder Isopropanol und

Wasser, zweckmässig mit einer anorganischen Säure, wie Salzsäure, Schwefelsäure oder Bortrifluoridetherat, oder einer organischen Säure, wie Toluolsulfonsäure, oder einem sauren Ionenaustauscher, wie Amberlyst K10, als Katalysator, bei einer Temperatur zwischen 0°C und 100°C durchgeführt, wobei man Verbindungen der Formel I erhält. Die Umsetzung eines substituierten Aldehyds der Formel II, eines Isonitrils der Formel $R^1NC$ und 4-Aminobenzonitril unter analogen Reaktionsbedingungen führt zu Verbindungen der Formel IV, worin Q Wasserstoff ist.

Verbindungen der Formel IV erhält man auch a) durch die Reaktion eines Aldehyds der Formel II, des Benzylisonitrils und eines gegebenenfalls 2-hydroxylierten 4-Aminobenzonitrils in wasserfreiem Methanol und Bortrifluoridetherat,

b) anschliessender Behandlung mit Wasser, c) Hydrolyse einer erhaltenen Verbindung der Formel

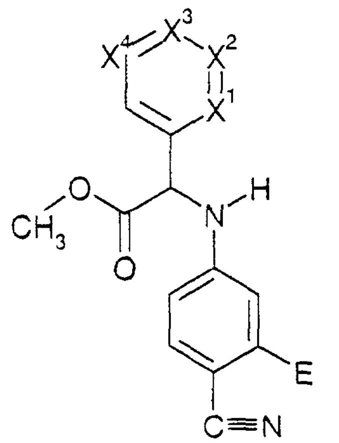

V

z.B. vorteilhaft in THF mit Lithiumhydroxid, und danach

d) Ueberführung einer erhaltenen Säure der Formel

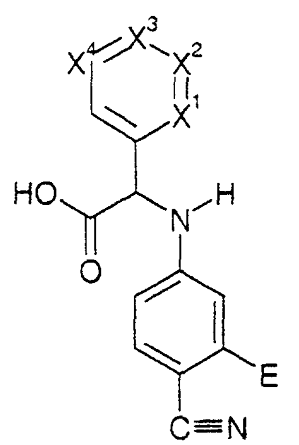

VI

in ein entsprechendes Nitril der Formel IV, durch Reaktion mit einem Amin der Formel HN(R,Q) in einem Lösungsmittel, wie DMF oder Dichlormethan, unter Zugabe eines Kopplungsreagenzes, wie Benzotriazol-1-yloxy-tris(dimethylamino)-phosphonium-hexafluoro-phoshat (BOP), in Gegenwart eines Carbodiimides, wie Diisopropylcarbodiimid oder Dicyclohexylcarbodiimid und gegebenenfalls eines tertiären Amins, wie Diisopropylamin.

Verbindungen der Formel IV können auch über die Umwandlung einer Verbindung IV, in der eine der Gruppen $X^2$, $X^3$ und $X^4$ für C-O-Allyl oder C-Obenzyl steht, in das entsprechende Phenol und gewünschtenfalls anschliessender Alkylierung erhalten werden. Die Abspaltung der Allylgruppe führt man vorzugsweise in einem aprotischen Lösungsmittel, wie Tetrahydrofuran (THF), mit einem Palladium(0) Katalysator wie Tetrakis(triphenylphosphin)-palladium in

THF, in Gegenwart eines Reduktionsmittels, wie Natriumborohydrid durch. Die Abspaltung der Benzylgruppe wird vorzugsweise durch katalytische Hydrierung (Pd/C) in einem Lösungsmittel, z.B. Ethanol und Dioxan, durchgeführt. Das so erhaltene Phenol kann dann gewünschtenfalls in einem Lösungsmittel, wie THF, mit Triphenylphosphin und Diethylazodicarboxylat als Katalysator und einem entsprechenden Alkohol alkyliert werden. Die Alkylierung kann man auch mit dem geeigneten Alkylbromid in Aceton oder DMF in Gegenwart von $K_2CO_3$ bewerkstelligen.

In den Verbindungen der Formeln I und IV bis VI enthaltene reaktionsfähige Gruppen können z.B. wie folgt funktionell abgewandelt werden:

Eine C-$NO_2$ Gruppe $X^1$, $X^2$, $X^3$ oder $X^4$ kann durch katalytische Hydrierung (Pd, Pt oder Ni) in einem Lösungsmittel, z.B. Ethylacetat und Ethanol zur Gruppe C-$NH_2$ reduziert werden.

Eine C-$NH_2$ Gruppe $X^1$, $X^2$, $X^3$ oder $X^4$ kann man z.B. in THF in Gegenwart von Diisopropylethylamin mit einem Acylchlorid, wie Benzoylchlorid, zur Gruppe C-NH-Acyl acylieren. Anstelle des Acylchlorids kann die entsprechende Säure eingesetzt werden. In diesem Fall wird wie bei der weiter oben beschriebenen Reaktion eines Amins HN (R,Q) mit einer Säure der Formel VI vorgegangen.

Eine C-Br Gruppe $X^1$,$X^2$,$X^3$ oder $X^4$ kann in eine C-Alkyl oder in eine über Alkyl gebundene Gruppe $X^1$, $X^2$, $X^3$ oder $X^4$ umgewandelt werden. In einer ersten Stufe wird z.B. ein Bromid IV in Dimethyl-acetamid mit einer endungesättigten Verbindung, wie $CH_2$ = CH-Alkyl-COOH, Triethylamin, Pd-Acetat und Tri-o-tolylphosphin in die entsprechende Verbindung IV übergeführt, in der $X^1$, $X^2$, $X^3$ oder $X^4$ fü CCH = CH-Alkyl-COOH steht. Die erhaltene ungesättigte Säure IV wird z.B. in Ethanol und THF mit Salzsäure, und Pd/C zur entsprechenden Säure IV, worin $X^1$,$X^2$,$X^3$ oder $X^4$ $CCH_2CH_2$- Alkyl-COOH ist, hydriert.

Eine in einer Gruppe R vorliegende Gruppe COO-Alkyl kann in THF mit LiOH zur COOH hydrolisiert werden.

Zur Umwandlung von CN in eine Gruppe C(N-$G^1$)NH-$G^2$, worin eins von $G^1$ und $G^2$ H und das andere OH ist, wird das Ausgangsnitril der Formel IV in einem Lösungsmittel, wie DMF, Ethanol oder Methanol, gelöst und zu einer Reaktionslösung aus einer Base, wie Diisopropylethylamin oder Triethylamin, Natriumhydrid, Natriummethanolat oder Natriumhydroxid, und Hydroxylamin oder einem Salz von Hydroxylamin mit einer anorganischen Säure, wie Hydroxylamin-hydrochlorid gegeben, zweckmässig bei einer Temperatur bis 80°C.

Zur Umwandlung von CN in C(NH)$NH_2$ kann man das Ausgangsnitril in einem Lösungsmittel, wie Ethanol oder Methanol, oder einem Lösungsmittelgemisch, wie Chloroform und Methanol oder Chloform und Ethanol, mit einem trockenen Strom von Chlorwasserstoff, zweckmässig bei einer Temperatur unter 10°C, begasen. Die Reaktionslösung wird mit einem Lösungsmittel, wie Diethylether, versetzt und das so ausgefallene Zwischenprodukt abfiltriert. Danach kann man das Zwischenprodukt in Wasser lösen, mit einer Base wie Natriumcarbonat oder Natriumhydroxid, neutralisieren und aus der wässrigen Phase mit einem Lösungsmittel, wie Dichlormethan, Chloroform oder Essigester, extrahieren. Das so erhaltene Material wird in einem Lösungsmittel, wie Methanol oder Ethanol, entweder mit gasförmigem Ammoniak oder einem Ammoniaksalz, wie Ammoniumchlorid, behandelt, zweckmässig bei einer Temperatur bis 80°C. Alternativ kann das abfiltrierte Zwischenprodukt gleich mit gasförmigem Ammoniak oder einem Ammoniaksalz in Methanol oder Ethanol behandelt werden.

Zur Umwandlung von CN in C(NH)$NH_2$ kann man auch nach der Umwandlung von CN in eine Gruppe C (N-$G^1$)NH-$G^2$, worin eins von $G^1$ und $G^2$ H und das andere OH ist, die erhaltenen Amidoxime der Formel I in einem Lösungsmittel, wie Ethanol, Methanol, Dioxan, THF oder Eisessig, oder einem Lösungsmittelgemisch, wie Ethanol und Eisessig, mit Wasserstoff und einem Katalysator, wie Palladium, Platin oder Nickel, hydrieren. Dabei wird eine in einer Verbindung der Formel I enthaltene Nitrogruppe $R^a$,$R^b$,$R^c$ oder $R^d$ zur Aminogruppe reduziert. Bei Hydrierung mit Palladium wird eine in einem Amidoxim der Formel I enthaltene Benzyloxygruppe $R^a$, $R^b$, $R^c$ oder $R^d$ in die Hydroxygruppe umgewandelt.

Durch Umsetzen einer Verbindung der Formel I, worin $G^1$ und $G^2$ Wasserstoff sind, in einem Lösungsmittel, wie Dichlormethan, Dioxan oder DMF, oder einem Lösungsmittelgemisch wie Dichlormethan und Wasser oder Essigester und Wasser, in Gegenwart einer organischen Base, wie Pyridin oder Triethylamin, oder einer anorganischen Base, wie Natriumhydroxid, Natriumcarbonat oder Kaliumhydrogencarbonat, mit einem Chlorameisensäureester der Formel ClC(O)O-A oder einem Ameisensäure-2,4-dinitrophenylester der Formel [2,4$(NO_2)_2$-$C_6H_3$]OC(O)O-A erhält man die entsprechende Verbindung, worin $G^1$ oder $G^2$ die Gruppe C(O)O-A ist und A für Alkyl steht.

Durch Umsetzen einer Verbindung I, worin $G^1$ und $G^2$ Wasserstoff sind, mit einem Acylchlorid wie einem Aroylchlorid, erhält man die entsprechende Verbindung I, worin eins von $G^1$ und $G^2$ Wasserstoff und das andere Acyl ist. Analog lässt sich eine Verbindung I, worin $G^1$ und $G^2$ Wasserstoff sind, mit einem p-Nitrophenylcarbonat der Formel p-$NO_2C_6H_4OCOO$-$R^g$ in die entsprechende Verbindung I überführen, in der eins von $G^1$ und $G^2$ für COO-$R^g$ steht. Zur Durchführung der Reaktion wird das Acylchlorid oder das p-Nitrophenylcarbonat in THF und DMF zunächst mit N, N-Diisopropylethylamin und dann mit dem unsubstituierten Amidin der Formel I versetzt.

Eine in einer Gruppe R enthaltene geschützte Aminogruppe kann in die freie Aminogruppe umgewandelt werden. So kann man eine eine Boc-geschützte Aminogruppe enthaltende Verbindung I in Methylenchlorid mit Trifluoressigsäure in das freie Amin I überführen.

Zudem enthalten manche der nachfolgenden Beispiele detaillierte Angaben betreffend die Herstellung bestimmter Ausgangsmaterialien und Zwischenprodukte.

Die Verbindungen der Formeln IV, V und VI sind neu und als solche ebenfalls Gegenstand der vorliegenden Erfindung. Die Verbindungen der Formeln II und III sind bekannt bzw. lassen sich in Analogie zu den bekannten Verbindungen herstellen. So erhält man die gegebenenfalls 2-substituierten 4-Aminobenzamidine III aus den entsprechenden 4-Aminobenzonitrile über die 4-Nitrobenzonitrile.

Aus WO-A-97 30971 sind Phenyl Derivate bekannt, welche Faktor Xa inhibieren und einen Einfluss auf die Blutgerinnung aufweisen. Die vorliegende Erfindung stellt neue Verbindungen der Formel I bereit. Die Verbindungen der Formel I, ihre Solvate und Salze hemmen die durch Faktor VIIa und den Gewebsfaktor induzierte Bildung der Gerinnungsfaktoren Xa, IXa und Thrombin. Die besagten Verbindungen beeinflussen dadurch sowohl die durch diese Faktoren induzierte Plättchenaggregation als auch die plasmatische Blutgerinnung. Sie verhindern damit die Entstehung von Thromben und können bei der Bekämpfung bzw. Verhütung von Krankheiten, wie Thrombose, Apoplexie, Herzinfakt, Entzündung und Arteriosklerose verwendet werden. Ferner haben diese Verbindungen einen Effekt auf Tumorzellen und verhindern Metastasen. Somit können sie auch als Antitumormittel eingesetzt werden.

Die Hemmung der amidolytischen Aktivität des Faktor VIIa/Gewebsfaktor-Komplexes durch die erfindungsgemässen Verbindungen wurde wie nachstehend beschrieben mit Hilfe eines chromogenen Peptidsubstrates nachgewiesen.

Die Messungen wurden auf Mikrotiterplatten bei Raumtemperatur durchgeführt. Dafür wurden pro Vertiefung der Platte zu 25 µl einer Lösung des Inhibitors in einem Puffer [pH 7.5, 100mM, bestehend aus 0,14M NaCl, 0.1 M N-(2-Hydroxyethyl)piperazin-N'-(2-ethansulfonsäure) (HEPES), 0,5 mg/l fettsäurefreies BSA (Bovine Serum Albumin) und 0,05% $NaN_3$], 100 µl einer Lösung mit 26 nM Gewebsfaktor, 9 nM löslicher Faktor VIIa und 8 mM Calciumchlorid gegeben. Nach 15 Minuten Inkubationszeit wurde die Reaktion durch Zugabe von 50 µl chromogenem Substrat Chromozym-tPA (3.5 mM, $MeSO_2$-D-Phe-Gly-Arg-Paranitroanilid) gestartet und die Hydrolyse des Substrates spektrophotometrisch auf einem kinetischen Mikrotiter-Plattenleser während 10 Minuten verfolgt. Nach der graphischen Darstellung der Hemmkurven wurden die Ki-Werte nach der in Biochem. J. 55, 1953, 170-171 beschriebenen Methode bestimmt. Die Resultate sind aus folgender Tabelle ersichtlich (Ki in microM):

| Beispiel | 1 | 2a | 5a | 5b | 5c | 5d | 5g | 6 | 7a |
|---|---|---|---|---|---|---|---|---|---|
| Ki | 0.057 | 0.283 | 0.281 | 0.195 | 0.169 | 0.116 | 0.044 | 0.22 | 0.19 |

| Beispiel | 7e | 7f | 7h | 7n | 7s | 8 | 10 |
|---|---|---|---|---|---|---|---|
| Ki | 0.11 | 0.26 | 0.21 | 0.17 | 0.171 | 0.159 | 0.112 |

Wie eingangs erwähnt, sind Arzneimittel enthaltend eine Verbindung der Formel I, ein Solvat oder ein Salz davon ebenfalls Gegenstand der vorliegenden Erfindung, weiterhin auch ein Verfahren zur Herstellung derartiger Arzneimittel, welches dadurch gekennzeichnet, ist, dass man eine oder mehrere solcher Verbindungen, Solvate oder Salze und gewünschtenfalls andere therapeutisch wertvolle Stoffe in galenische Darreichungsform bringt. Diese Arzneimittel können oral, z.B. in Form von Dragees, Hart- und Weichgelatinekapseln, Lösungen, Emulsionen oder Suspensionen, oder rektal, z.B. in Form von Suppositorien, oder als Spray verabreicht werden. Die Verabreichung kann aber auch parenteral, z.B. in Form von Injektionslösungen, erfolgen.

Zur Herstellung von Tabletten, Lacktabletten, Dragées und Hartgelatinekapseln kann der Wirkstoff mit pharmazeutisch inerten, anorganischen oder organischen Excipientien vermischt werden. Als solche Excipientien kann man für Tabletten, Lacktabletten, Dragées und Hartgelatinekapseln z.B. Lactose, Maisstärke oder Derivate davon, Talk, Stearinsäure oder deren Salze verwenden. Für Weichgelatinekapseln eignen sich als Excipientien z.B. vegetabile Oele, Wachse, Fette, halbfeste und flüssige Polyole; je nach Beschaffenheit des Wirkstoffs sind jedoch bei Weichgelatinekapseln überhaupt keine Excipientien erforderlich. Zur Herstellung der Lösungen und Sirupe eignen sich als Excipientien z.B. Wasser, Polyole, Saccharose, Invertzucker und Glucose, für Injektionslösungen eignen sich z.B.

Wasser, Alkohole, Polyole, Glycerin und vegetabile Oele, und für Suppositorien eignen sich natürliche und gehärtete Oele, Wachse, Fette, Halbflüssige oder flüssige Polyole. Die pharmazeutischen Präparate können daneben noch Konservierungsmittel, Lösungsvermittler, Stabilisierungsmittel, Netzmittel, Emulgierungsmittel, Süssmittel, Färbemittel, Aromatisierungsmittel, Salze zur Veränderung des osmotischen Druckes, Puffer, Ueberzugsmittel oder Antioxidantien enthalten.

Für die Bekämpfung bzw. Verhütung der weiter oben genannten Krankheiten kann die Dosierung des Wirkstoffes innerhalb weiter Grenzen variieren und ist natürlich in jedem Einzelfall den individuellen Gegebenheiten anzupassen. Im allgemeinen dürfte bei oraler oder parenteraler, z.B. intravenöser oder subkutaner Verabreichung eine Dosis von etwa 0,1 bis 20 mg/kg, vorzugsweise von etwa 0,5 bis 4 mg/kg, pro Tag für den Erwachsenen angemessen sein, wobei aber die soeben angegebene obere Grenze auch überoder unterschritten werden kann, wenn sich dies als angezeigt erweisen sollte.

Beispiel 1

1 mmol 4-Aminobenzamidin Dihydrochlorid (208 mg), 1 mmol Triethylamin (101 mg), 1 mmol Benzylisonitril (117 mg) und 1 mmol 4-Benzyloxy-3-methoxybenzaldehyd (260 mg) werden in einem Lösungsmittelgemisch aus 1.2 ml Isopropanol und 0.8 ml Wasser über 5 Tage bei Raumtemperatur gerührt. Danach versetzt man mit 0.5 ml 25%-iger Salzsäure, rührt 1 Stunde bei Raumtemperatur, filtriert die ausgefallenen Kristalle ab und wäscht mit 5 ml Wasser. Man erhält 218 mg (41%) (R,S)-N-Benzyl-2-(4-benzyloxy-3-methoxyphenyl)-2-(4-carbamimidoyl-phenylamino)-acetamid-hydrochlorid als farblose Kristalle. ISP-MS: 495.5 ($[M+H]^+$, 100).

Beispiel 2

Analog Beispiel 1 stellt man folgende Verbindungen her:

2.a) Aus 4-Aminobenzamidin Dihydrochlorid, Triethylamin, Benzylisonitril und 3,4-Dimethoxy-benzaldehyd erhält man (R,S)-N-Benzyl-2-(4-carbamimidoylphenylamino)-2-(3,4-dimethoxy-phenyl)-acetamid-hydrochlorid als farblose Kristalle. ISP-MS: 455 ($[M+H]^+$, 100).

2.b) Aus 4-Aminobenzamidin Dihydrochlorid, Triethylamin, Benzylisonitril und Piperonylbenzaldehyd erhält man (R,S)-2-Benzo[1,3]dioxol-5-yl-N-benzyl-2-(4-carbamimidoyl-phenylamino)-acetamid-hydrochlorid als farblose Kristalle. ISP-MS: 439 ($[M+H]^+$, 100).

2.c) Aus 4-Aminobenzamidin Dihydrochlorid, Triethylamin, Benzylisonitril und 2-Hydroxy-4-methoxy-benzaldehyd erhält man (R,S)-N-Benzyl-2-(4-carbamimidoyl-phenylamino)-2-(2-hydroxy-4-methoxy-phenyl)-acetamidhydrochlorid als farblose Kristalle. ISP-MS: 405 ($[M+H]^+$, 100).

2.d) Aus 4-Aminobenzamidin Dihydrochlorid, Triethylamin, Benzylisonitril und 4-Hydroxy-3-methoxy-5-nitro-benzaldehyd erhält man (R,S)-N-Benzyl-2-(4-carbamimidoyl-phenylamino)-2-(4-hydroxy-3-methoxy-5-nitro-phenyl)-acetamidhydrochlorid als farblose Kristalle. ISP-MS: 450 ($[M+H]^+$, 100).

2.e) Aus 4-Aminobenzamidin Dihydrochlorid, Triethylamin, Benzylisonitril und 4-Hydroxy-3,5-dimethoxy-benzaldehyd erhält man (R,S)-N-Benzyl-2-(4-carbamimidoyl-phenylamino)-2-(4-hydroxy-3,5-dimethoxy-phenyl)-acetamidhydrochlorid als farblose Kristalle. ISP-MS: 435 ($[M+H]^+$, 100).

2.f) Aus 4-Aminobenzamidin Dihydrochlorid, Triethylamin, Benzylisonitril und 4-Formylbenzoesäuremethylester erhält man (R,S)-4-[Benzylcarbamoyl-(4-carbamimidoyl-phenylamino)-methyl]-benzoesäuremethylester-hydrochlorid als farblose Kristalle. ISP-MS: 417 ($[M+H]^+$, 100).

2.g) Aus 4-Aminobenzamidin Dihydrochlorid, Triethylamin, Benzylisonitril und 2,3,4-Trimethoxy-benzaldehyd erhält man (R,S)-N-Benzyl-2-(4-carbamimidoyl-phenylamino)-2-(2,3,4-trimethoxy-phenyl)-acetamid-hydrochlorid als farblose Kristalle. ISP-MS: 449 ($[M+H]^+$, 100).

2.h) Aus 4-Aminobenzamidin Dihydrochlorid, Triethylamin, Benzylisonitril und 3,4-Dimethyl-benzaldehyd erhält man (R,S)-N-Benzyl-2-(4-carbamimidoylphenylamino)-2-(3,4-dimethyl-phenyl)-acetamid-hydrochlorid als farblose Kristalle. ISP-MS: 387 ($[M+H]^+$, 100).

2.i) Aus 4-Aminobenzamidin Dihydrochlorid, Triethylamin, Benzylisonitril und 4-Methoxy-3-methyl-benzaldehyd

erhält man (R,S)-N-Benzyl-2-(4-carbamimidoyl-phenylamino)-2-(4-methoxy-3-methyl-phenyl)-acetamidhydrochlorid als farblose Kristalle. ISP-MS: 403 ([M+H]$^+$, 100).

2.j) Aus 4-Aminobenzamidin Dihydrochlorid, Triethylamin, Benzylisonitril und 4,5-Dimethoxy-2-nitro-benzaldehyd erhält man (R,S)-N-Benzyl-2-(4-carbamimidoyl-phenylamino)-2-(4,5-dimethoxy-2-nitro-phenyl)-acetamidhydrochlorid als farblose Kristalle. ISP-MS: 464 ([M+H]$^+$, 100).

2.k) Aus 4-Aminobenzamidin Dihydrochlorid, Triethylamin, Benzylisonitril und 2,3-Dimethoxy-benzaldehyd erhält man (R,S)-N-Benzyl-2-(4-carbamimidoylphenylamino)-2-(2,3-dimethoxy-phenyl)-acetamid-hydrochlorid als farblose Kristalle. ISP-MS: 419 ([M+H]$^+$, 100).

2.1) Aus 4-Aminobenzamidin Dihydrochlorid, Triethylamin, Benzylisonitril und 2,4,5-Trimethoxy-benzaldehyd erhält man (R,S)-N-Benzyl-2-(4-carbamimidoyl-phenylamino)-2-(2,4,5-trimethoxy-phenyl)-acetamid-hydrochlorid als farblose Kristalle. ISP-MS: 449 ([M+H]$^+$, 100).

2.m) Aus 4-Aminobenzamidin Dihydrochlorid, Triethylamin, Benzylisonitril und 3-Hydroxy-4-methoxy-benzaldehyd erhält man (R,S)-N-Benzyl-2-(4-carbamimidoyl-phenylamino)-2-(3-hydroxy-4-methoxy-phenyl)-acetamidhydrochlorid als farblose Kristalle. ISP-MS: 405 ([M+H]$^+$, 100).

2.n) Aus 4-Aminobenzamidin Dihydrochlorid, Triethylamin, Benzylisonitril und 2,4-Dimethoxy-benzaldehyd erhält man N-Benzyl-2-(4-carbamimidoylphenylamino)-2-(2,4-dimethoxy-phenyl)-acetamid-hydrochlorid als farblose Kristalle. ISP-MS: 419 ([M+H]$^+$, 100).

2.o) Aus 4-Aminobenzamidin Dihydrochlorid, Triethylamin, Benzylisonitril und 2-Fluoro-4,5-dimethoxy-benzaldehyd erhält man (R,S)-N-Benzyl-2-(4-carbamimidoyl-phenylamino)-2-(2-fluoro-4,5-dimethoxy-phenyl)-acetamidhydrochlorid als farblose Kristalle. ISP-MS: 437 ([M+H]$^+$, 100).

2.p) Aus 4-Aminobenzamidin Dihydrochlorid, Triethylamin, Cyclopropylmethylisonitril und 3,4,5-Trimethoxy-benzaldehyd erhält man (R,S)-2-(4-Carbamimidoyl-phenylamino)-N-cyclopropylmethyl-2-(3,4,5-trimethoxy-phenyl)-acetamid-hydrochlorid als farblose Kristalle. ISP-MS: 413 ([M+H]$^+$, 100).

2.q) Aus 4-Aminobenzamidin Dihydrochlorid, Triethylamin, 3,4-Dimethoxybenzylisonitril und 3,4,5-Trimethoxy-benzaldehyd erhält man (R,S)-2-(4-Carbamimidoyl-phenylamino)-N-(3,4-dimethoxy-benzyl)-2-(3,4,5-trimethoxy-phenyl)-acetamid-hydrochlorid als farblose Kristalle. ISP-MS: 509 ([M+H]$^+$, 100).

2.r) Aus 4-Aminobenzamidin Dihydrochlorid, Triethylamin, Benzylisonitril und 2-(Formyl-2-methoxy-phenoxy)-benzoesäuremethylester erhält man (R,S)-2-{4-[Benzyl-carbamoyl-(4-carbamimidoyl-phenylamino)-methyl]-2-methoxyphenoxy}-benzoesäuremethylester-hydrochlorid als farblose Kristalle. ISP-MS: 539.4 ([M+H]$^+$, 100).

2.s) Aus 4-Aminobenzamidin Dihydrochlorid, Triethylamin, 2-Phenylethylisonitril und 4-Dimethylamino-3-nitrobenzaldehyd erhält man (R,S)-N-(2-Phenyl-ethyl)-2-(4-carbamimidoyl-phenylamino)-2-(4-dimethylamino-3-nitrophenyl)-acetamid-hydrochlorid als farblose Kristalle. ISP-MS: 461.7 ([M+H]$^+$, 100).

2.t) Aus 4-Aminobenzamidin Dihydrochlorid, Triethylamin, Benzylisonitril und 3,5-Dimethoxy-4-methyl-benzaldehyd erhält man (R,S)-N-Benzyl-2-(4-carbamimidoyl-phenylamino)-2-(3,5-dimethoxy-4-methyl-phenyl)-acetamidhydrochlorid als farblose Kristalle. ISP-MS: 433 ([M+H]$^+$, 100).

2.u) Aus 4-Aminobenzamidin Dihydrochlorid, Triethylamin, Benzylisonitril und 3-Phenoxy-benzaldehyd erhält man (R,S)-N-Benzyl-2-(3-phenoxy-phenyl)-2-(4-carbamimidoyl-phenylamino)-acetamid-hydrochlorid als farblose Kristalle. ISP-MS: 451.6 ([M+H]$^+$, 100).

2.v) Aus 4-Aminobenzamidin Dihydrochlorid, Triethylamin, Benzylisonitril und 3,5-bis-Benzyloxy-benzaldehyd erhält man (R,S)-N-Benzyl-2-(3,5-bisbenzyloxy-phenyl)-2-(4-carbamimidoyl-phenylamino)-acetamid-hydrochlorid als farblose Kristalle. ISP-MS: 571 ([M+H]$^+$, 100).

2.w) Aus 4-Aminobenzamidin Dihydrochlorid, Triethylamin, Benzylisonitril und 3-Benzyloxy-4-hydroxy-benzaldehyd erhält man (R,S)-N-Benzyl-2-(3-benzyloxy-4-hydroxy-phenyl)-2-(4-carbamimidoyl-phenylamino)-acetamidhydrochlorid als farblose Kristalle. ISP-MS: 481 ([M+H]$^+$, 100).

2.x) Aus 4-Aminobenzamidin Dihydrochlorid, Triethylamin, Benzylisonitril und 4-Bromo-3,5-dimethoxy-benzaldehyd erhält man (R,S)-N-Benzyl-2-(4-bromo-3,5-dimethoxy-phenyl)-2-(4-carbamimidoyl-phenylamino)-acetamid-hydrochlorid als farblose Kristalle. ISP-MS: 498 ($[M+H]^+$, 100).

2.y) Aus 4-Aminobenzamidin Dihydrochlorid, Triethylamin, Benzylisonitril und 3,5-Dimethoxy-4-nitro-benzaldehyd erhält man (R,S)-N-Benzyl-2-(4-carbamimidoyl-phenylamino)-2-(3,5-dimethoxy-4-nitro-phenyl)-acetamidhydrochlorid als farblose Kristalle. ISP-MS: 464 ($[M+H]^+$, 100).

Beispiel 3

10 mmol 4-Aminobenzamidin Dihydrochlorid (2.8 g), 10 mmol Triethylamin (1.01 g), 0.5 ml Wasser, 10 mmol 4-Benzyloxy-3-methoxy-benzaldehyd (2.6 g) und 10 mmol Benzylisonitril (1.2 ml) werden in 40 ml Methanol vorgelegt, danach tropft man unter Eisbadkühlung während 2 Stunden 3.8 ml Bortrifluorid Etherat (30 mmol) dazu. Nach 1 weiteren Stunde Rühren bei Raumtemperatur wird das Lösungsmittel unter Vakuum entfernt und das Rohprodukt über eine RP-18 Säule mit Wasser/Methanol als Eluent chromatographiert. Alternativ versetzt man das Rohprodukt unter Eiskühlung mit 5 ml 25%-iger Salzsäure, gibt 10 ml Dichlormethan und 10 ml Wasser dazu und saugt nach 5 Stunden die ausgefallenen Kristalle ab. Man erhält 1.6 g (30%) (R,S)-N-Benzyl-2-(4-benzyloxy-3-methoxy-phenyl)-2-(4-carbamimidoyl-phenylamino)-acetamidhydrochlorid als farblose Kristalle. ISP-MS: 495.5 ($[M+H]^+$, 100). Durch Chromatographie des Rohproduktes über eine RP-18 Säule mit einem Methanol/Wasser Eluenten mit 0.1% Trifluoressigsäure erhält man 0.8 g (15%) (R,S)-N-Benzyl-2-(4-benzyloxy-3-methoxy-phenyl)-2-(4-carbamimidoylphenylamino)-acetamid Trifluoroacetat als farblose Kristalle. ISP-MS: 495.2 ($[M+H]^+$, 100).

Beispiel 4

10 mmol 4-Aminobenzonitril (1.2 g), 0.5 ml Wasser und 10 mmol 4-Benzyloxy-3-methoxy-benzaldehyd (2.6 g) werden in 40 ml Methanol vorgelegt und eine Stunde bei Raumtemperatur gerührt, danach gibt man 10 mmol Benzylisonitril (1.2 ml) dazu und tropft unter Eisbadkühlung und Rühren während 2 Stunden 3.8 ml Bortrifluoridetherat (30 mmol) dazu. Nach 1 weiteren Stunde Rühren bei Raumtemperatur wird das Lösungsmittel unter Vakuum entfernt und das Rohprodukt über eine Kieselgelsäule mit Hexan/Essigester 1/1 als Eluent filtriert. Die Hauptfraktion wird eingedampft und das so erhaltene rohe (R,S)-N-Benzyl-2-(4-benzyloxy-3-methoxy-phenyl)-2-(4-cyano-phenyl-amino)-acetamid wird in einem Lösungsmittelgemisch aus 20 ml Chloroform und 4 ml Methanol gelöst und unter Rühren bei einer Temperatur unter 5°C 20 Minuten mit trockenem Chlorwasserstoff begast. Danach lässt man das Reaktionsgemisch 24 Stunden bei 5°C stehen, gibt dann 20 ml Diethylether dazu und saugt die ausgefallenen Kristalle ab, wäscht mit Diethylether nach und nimmt den Rückstand in mit Ammoniak gesättigtem Methanol auf. Die ammoniakalische Lösung wird über 2 Stunden am Rückfluss gekocht, danach wird das Lösungsmittel im Wasserstrahlvakuum entfernt. Zu dem Rückstand gibt man 5 ml einer 1 N Salzsäurelösung und entfernt das Lösungsmittel erneut am Wasserstrahlvakuum. Das so erhaltene Rohprodukt wird über eine RP-18 Säule mit Wasser/Methanol als Eluent chromatographiert. Man erhält 228 mg (43%) (R,S)-N-Benzyl-2-(4-benzyloxy-3-methoxy-phenyl)-2-(4-carbamimidoylphenylamino)-acetamid-hydrochlorid als farblose Kristalle. ISP-MS: 495.5 ($[M+H]^+$, 100).

Beispiel 5

Analog Beispiel 4 stellt man folgende Verbindungen her:

5.a) Aus 4-Aminobenzonitril, Benzylisonitril une 3,4,5-Trimethoxy-benzaldehyd erhält man (R,S)-N-Benzyl-2-(4-carbamimidoyl-phenylamino)-2-(3,4,5-trimethoxy-phenyl)-acetamid-hydrochlorid als farblose Kristalle. ISP-MS: 449.2 ($[M+H]^+$, 100).

5.b) Aus 4-Aminobenzonitril, Benzylisonitril und 3,5-Dimethoxybenzaldehyd erhält man (R,S)-N-Benzyl-2-(4-carbamimidoyl-phenylamino)-2-(3,5-dimethoxy-phenyl)-acetamid-hydrochlorid als farblose Kristalle. ISP-MS: 419 ($[M+H]^+$, 100).

5.c) Aus 4-Aminobenzonitril, Benzhydrylisonitril und 3,4-Dimethoxybenzaldehyd erhält man (R,S)-N-Benzhydryl-2-(4-carbamimidoyl-phenylamino)-2-(3,4-dimethoxy-phenyl)-acetamid-hydrochlorid als farblose Kristalle. ISP-MS: 495 ($[M+H]^+$, 100).

5.d) Aus 4-Aminobenzonitril, Benzylisonitril und 3,4-Diethoxy-benzaldehyd erhält man (R,S)-N-Benzyl-2-(4-carbamimidoyl-phenylamino)-2-(3,4-diethoxyphenyl)-acetamid-hydrochlorid als farblose Kristalle. ISP-MS: 447.2

([M+H]$^+$, 100).

5.e) Aus 4-Aminobenzonitril, Benzhydrylisonitril und 4-Benzyloxy-3-methoxy-benzaldehyd erhält man (R,S)-N-Benzhydryl-2-(4-benzyloxy-3-methoxyphenyl)-2-(4-carbamimidoyl-phenylamino)-acetamid-hydrochlorid als farblose Kristalle. ISP-MS: 571.4 ([M+H]$^+$, 100).

5.f) Aus 4-Aminobenzonitril, Benzhydrylisonitril und 4-Benzyloxy-3-ethoxybenzaldehyd erhält man (R,S)-N-Benzhydryl-2-(4-benzyloxy-3-ethoxy-phenyl)-2-(4-carbamimidoyl-phenylamino)-acetamid-hydrochlorid als farblose Kristalle. ISP-MS: 585.4 ([M+H]$^+$, 100).

5.g) Aus 4-Aminobenzonitril, Benzylisonitril und 4-Benzyloxy-3-ethoxybenzaldehyd erhält man (R,S)-N-Benzyl-2-(4-benzyloxy-3-ethoxy-phenyl)-2-(4-carbamimidoyl-phenylamino)-acetamid-hydrochlorid als farblose Kristalle. ISP-MS: 509.4 ([M+H]]$^+$, 100).

5.h) Aus 4-Aminobenzonitril, 4-Methoxy-benzylisonitril und 4-Benzyloxy-3-methoxy-benzaldehyd erhält man (R,S)-2-(4-Benzyloxy-3-methoxy-phenyl)-2-(4-carbamimidoyl-phenylamino)-N-(4-methoxy-benzyl)-acetamid-hydrochlorid als farblose Kristalle. ISP-MS: 525.2 ([M+H]$^+$, 100).

5.i) Aus 4-Aminobenzonitril, Naphthyl-1-yl-methylisonitril und 4-Benzyloxy-3-methoxy-benzaldehyd erhält man (R,S)-2-(4-Benzyloxy-3-methoxyphenyl)-2-(4-carbamimidoyl-phenylamino)-N-naphthalen-1-yl-methyl-acetamidhydrochlorid als farblose Kristalle. ISP-MS: 545.3 ([M+H]$^+$, 100).

5.j) Aus 4-Aminobenzonitril, Benzylisonitril und 3-Butoxy-5-methoxybenzaldehyd erhält man (R,S)-N-Benzyl-2-(3-butoxy-5-methoxy-phenyl)-2-(4-carbamimidoyl-phenylamino)-acetamid-hydrochlorid als farblose Kristalle. ISP-MS: 461.3 ([M+H]$^+$, 100).

5.k) Aus 4-Aminobenzonitril, Benzylisonitril und 3-Benzyloxy-5-ethoxybenzaldehyd erhält man (R,S)-N-Benzyl-2-(3-benzyloxy-5-ethoxy-phenyl)-2-(4-carbamimidoyl-phenylamino)-acetamid-hydrochlorid als farblose Kristalle. ISP-MS: 509.4 ([M+H]$^+$, 100).

5.1) Aus 4-Aminobenzonitril, Benzylisonitril und 3-Benzyloxy-5-methoxybenzaldehyd erhält man (R,S)-N-Benzyl-2-(3-benzyloxy-5-methoxy-phenyl)-2-(4-carbamimidoyl-phenylamino)-acetamid-hydrochlorid als farblose Kristalle. ISP-MS: 495.3 ([M+H]$^+$, 100).

Beispiel 6

Aus 130 mg (5,6 mmol) Natrium und 10 ml MeOH wurde unter Argonatmosphäre eine NaOMe-Lösung frisch hergestellt und mit 474 mg (6.8 mmol) Hydroxylamin-hydrochlorid versetzt. Die Suspension wurde während 30 min bei Raumtemperatur gerührt. Nach Zugabe von 286 mg (0.6 mmol) (R,S)-N-Benzyl-2-(4-cyano-phenylamino)-2-[3-methoxy-4-(3-methoxy-benzyloxy)-phenyl]-acetamid (Beispiel 18) wurde das Reaktionsgemisch über Nacht unter Rückfluss erhitzt. Anschliessend wurde das Lösungsmittel im Vakuum abdestilliert. Der Rückstand wurde in Wasser aufgenommen und mit EtOAc extrahiert. Die Organische Phase wurde mit gesättigter NaCl-Lösung gewaschen, über $MgSO_4$ getrocknet, filtriert und im Vakuum eingedampft.

Der Rückstand wurde in 10 ml Ethanol, 1 ml THF und 1 ml HOAc aufgenommen und mit einer Spatelspitze Raney-Nickel versetzt. Das Reaktionsgemisch wurde während 5 h hydriert. Der Katalysator wurde abfiltriert. Das Filtrat wurde im Vakuum eingedampft. Der Rückstand wurde chromatographisch an Kieselgel gereinigt (EtOAc/Aceton/ $H_2O$/ HOAc 6:2:1:1). Man erhielt 191 mg (53%) (R,S)-N-Benzyl-2-(4-carbamimidoyl-phenylamino)-2-[3-methoxy-4-(3-methoxy-benzyloxy)-phenyl]-acetamid Acetat als farblose Kristalle. ISP-MS: 525.2 ([M+H]).

Beispiel 7

In Analogie zu Beispiel 6 erhielt man:

7.a) aus (R,S)-N-Benzyl-2-(4-cyano-phenylamino)-2-[3-methoxy-4-(pyridin-4-yl-methoxy)-phenyl]-acetamid (Beispiel 19.a) das N-Benzyl-2-(4-carbamimidoylphenylamino)-2-[3-methoxy-4-(pyridin-4-ylmethoxy)-phenyl]-acetamid Acetat in 18% Ausbeute. Farbloser Festkörper. ISP-MS: 496.2 ([M+H]).

7.b) aus (R,S)-N-Benzyl-2-(4-cyano-phenylamino-2-[3-methoxy-4-(2-morpholin-4-yl-ethoxy)-phenyl]-acetamid

(Beispiel 19.b) das (R,S)-N-Benzyl-2-(4-carbamimidoyl-phenylamino)-2-[3-methoxy-4-(2-morpholin-4-yl-ethoxy)-phenyl]-acetamid Acetat in 15% Ausbeute. Farbloser Festkörper. ISP-MS: 518.3 ([M+H]).

7.c) aus (R,S)-N-Benzyl-2-(4-cyano-phenylamino)-2-[3-methoxy-4-(2-thiomorpholin-4-yl-ethoxy)-phenyl]-acetamid (Beispiel 19.c) das (R,S)-N-Benzyl-2-(4-carbamimidoyl-phenylamino)-2-[3-methoxy-4-(2-thiomorpholin-4-yl-ethoxy)-phenyl]-acetamid Acetat in 22% Ausbeute. Gelb-grüner Festkörper. ISP-MS: 534.3 ([M+H]).

7.d) aus (R,S)-N-Benzyl-2-(4-cyano-phenylamino)-2-[3-methoxy-4-(3-phenoxy-benzyloxy)-phenyl]-acetamid (Beispiel 19.d) das (R,S)-N-Benzyl-2-(4-carbamimidoyl-phenylamino)-2-[3-methoxy-4-(3-phenoxy-benzyloxy)-phenyl]-acetamid Acetat in 28% Ausbeute. Farbloser Festkörper. ISP-MS: 587.4 ([M+H]).

7.e) aus (R,S)-N-Benzyl-2-(4-cyano-phenylamino)-2-[4-(1H-indol-5-ylmethoxy)-3-methoxy-phenyl]-acetamid (Beispiel 19.e) das (R,S)-N-Benzyl-2-(4-carbamimidoyl-phenylamino)-2-[4-(1H-indol-5-ylmethoxy)-3-methoxy-phenyl]acetamid Acetat in 23% Ausbeute. Farbloser Festkörper. ISP-MS: 534.3 ([M+H]).

7.f) aus (R,S)-N-Benzyl-2-(4-cyano-phenylamino)-2-[3-methoxy-4-(quinolin-7-yl-methoxy)-phenyl]-acetamid (Beispiel 19.f) das (R,S)-N-Benzyl-2-(4-carbamimidoyl-phenylamino)-2-[3-methoxy-4-(1,2,3,4-tetrahydro-chinolin-7-ylmethoxy)-phenyl]-acetamid Acetat in 13% Ausbeute. Farbloser Festkörper. ISP-MS: 550.2 ([M+H]).

7.g) aus (R,S)-N-Benzyl-2-(4-cyano-phenylamino)-2-[3-methoxy-4-(chinolin-7-ylmethoxy)-phenyl]-acetamid (Beispiel 19.f) das (R,S)-N-Benzyl-2-(4-carbamimidoyl-phenyl-amino)-2-[3-methoxy-4-(chinolin-7-ylmethoxy)-phenyl]-acetamid Acetat in 16% Ausbeute. Leicht rosa Festkörper. ISP-MS: 546.3 ([M+H]).

7.h) aus (R,S)-N-Benzyl-2-(4-cyano-phenylamino)-2-(3-methoxy-4-phenethyloxy-phenyl)-acetamid (Beispiel 19.g) das (R,S)-N-Benzyl-2-(4-carbamimidoyl-phenylamino)-2-(3-methoxy-4-phenethyl-oxy-phenyl)-acetamid Acetat in 21% Ausbeute. Farbloser Festkörper. ISP-MS: 509.4 ([M+H]).

7.i) aus (R,S)-N-Benzyl-2-[4-(biphenyl-4-ylmethoxy)-3-methoxy-phenyl]-2-(4-cyano-phenylamino)-acetamid (Beispiel 19 h) das (R,S)-N-Benzyl-2-[4-(biphenyl-4-ylmethoxy)-3-methoxy-phenyl] -2-(4-carbamimidoyl-phenylamino)-acetamid Acetat in 58% Ausbeute. Farbloser Festkörper. ISP-MS: 571.3 ([M+H]).

7.j) aus (R,S)-N-Benzyl-2-(4-cyano-phenylamino)-2-[4-(2,6-dichlorobenzyloxy)-3-methoxy-phenyl]-acetamid (Beispiel 19 i) das (R,S)-N-Benzyl-2-(4-carbamimidoyl-phenylamino)-2- [4-(2,6-dichloro-benzyloxy)-3-methoxy-phenyl]-acetamid Acetat in 58% Ausbeute. Farbloser Festkörper. ISP-MS: 563.3 ([M+H]).

7.k) aus (R,S)-N-Benzyl-2-(4-cyano-phenylamino)-2-[4-(3,5-dichlorobenzyloxy)-3-methoxy-phenyl]-acetamid (Beispiel 19 j) das (R,S)-N-Benzyl-2-(4-carbamimidoyl-phenylamino)-2-[4-(3,5-dichloro-benzyloxy)-3-methoxy-phenyl]-acetamid Acetat in 47% Ausbeute. Farbloser Festkörper. ISP-MS: 563.3 ([M+H]).

7.1) aus (R,S)-N-Benzyl-2-[4-(3-bromo-benzyloxy)-3-methoxy-phenyl]-2-(4-cyano-phenyl-amino)-acetamid (Beispiel 19 k) das (R,S)-N-Benzyl-2-[4-(3-bromobenzyloxy)-3-methoxy-phenyl]-2-(4-carbamimidoyl-phenylamino)-acetamid Acetat in 35% Ausbeute. Farbloser Festkörper. ISP-MS: 573 ([M+H]).

7.m) aus (R,S)-N-Benzyl-2-(4-cyano-phenylamino)-2-[3-methoxy-4-(pyridin-2-ylmethoxy)-phenyl]-acetamid (Beispiel 19 l) das (R,S)-N-Benzyl-2-(4-carbamimidoyl-phenylamino)-2-[3-methoxy-4-(pyridin-2-ylmethoxy)-phenyl]-acetamid Acetat in 34% Ausbeute. Farbloser Festkörper. ISP-MS: 496.3 ([M+H]).

7.n) aus (R,S)-N-Benzyl-2-(4-cyano-phenylamino)-2-[4-(isochinolin-6-ylmethoxy)-3-methoxy-phenyl]-acetamid (Beispiel 19 m) das (R,S)-N-Benzyl-2-(4-carbamimidoyl-phenylamino)-2-[4-(isochinolin-6-ylmethoxy)-3-methoxy-phenyl]-acetamid Acetat in 11% Ausbeute. Farbloser Festkörper. ISP-MS: 546.3 ([M+H]).

7.o) aus (R,S)-2-(4-Benzyloxy-3-methoxy-phenyl)-2-(4-cyano-phenylamino)-N-(3,4-dimethoxy-benzyl)-acetamid (Beispiel 24.a) das (R,S)-2-(4-Benzyloxy-3-methoxy-phenyl)-2-(4-carbamimidoyl-phenyl-amino)-N-(3,4-dimethoxy-benzyl)-acetamid Acetat in 5% Ausbeute. Farbloser Festkörper. ISP-MS: 555.3 ([M+H]).

7.p) aus (R,S)-2-(4-Benzyloxy-3-methoxy-phenyl)-2-(4-cyano-phenylamino)-N-phenethyl-acetamid (Beispiel 23) das (R,S)-2-(4-Benzyloxy-3-methoxy-phenyl)-2-(4-carbamimidoyl-phenylamino)-N-phenethyl-acetamid Acetat in 30% Ausbeute. Farbloser Festkörper. ISP-MS: 555.3 ([M+H]).

7.q) aus (R,S)-2-(4-Benzyloxy-3-methoxy-phenyl)-2-(4-cyano-phenylamino)-N-(4-nitro-benzyl)-acetamid (Beispiel 24.b) das (R,S)-N-(4-Amino-benzyl)-2-(4-benzyloxy-3-methoxy-phenyl)-2-(4-carbamimidoyl-phenylamino)-acetamid Acetat in 52% Ausbeute. Hellgelber Festkörper. ISP-MS: 510.4 ([M+H]).

7.r) aus (R,S)-2-(4-Benzyloxy-3-methoxy-phenyl)-N-(4-cyano-phenyl)-2-(4-cyano-phenyl-amino)-acetamid (Beispiel 24 d) das (R,S)-2-(4-Benzyloxy-3-methoxy-phenyl)-N-(4-carbamimidoyl-phenyl)-2-(4-carbamimidoyl-phenylamino)-acetamid Acetat in 30% Ausbeute. Farbloser Festkörper. ISP-MS: 523.3 ([M+H]).

7.s) aus (R,S)-2-(4-Benzyloxy-3-methoxy-phenyl)-2-(4-cyano-phenylamino)-N-phenyl-acetamid (Beispiel 24 c) das (R,S)-2-(4-Benzyloxy-3-methoxy-phenyl)-2-(4-carbamimidoyl-phenyl-amino)-N-phenyl-acetamid Acetat in 12% Ausbeute. Farbloser Festkörper. ISP-MS: 481.4 ([M+H]).

7.t) aus (R,S)-2-(4-Benzyloxy-3-methoxy-phenyl)-2-(4-cyano-phenylamino)-N-[2-(3H-imidazol-4-yl)-ethyl]-acetamid (Beispiel 24 e) das (R,S)-2-(4-Benzyloxy-3-methoxy-phenyl)-2-(4-carbamimidoyl-phenylamino)-N-[2-(3H-imidazol-4-yl)-ethyl]-acetamid Acetat in 9% Ausbeute. Farbloser Festkörper. ISP-MS: 482.4 ([M+H]).

7.u) aus (R,S)-2-(4-Benzyloxy-3-methoxy-phenyl)-2-(4-cyano-phenylamino-N-[2-(1H-indol-3-yl)-ethyl]-acetamid (Beispiel 24 f) das (R,S)-2-(4-Benzyloxy-3-methoxy-phenyl)-2-(4-carbamimidoyl-phenylamino)-N-[2-(1H-indol-3-yl)-ethyl]-acetamid Acetat in 68% Ausbeute. Farbloser Festkörper. ISP-MS: 553.3 ([M+Na]), 548.3 ([M+NH4]), 531.3 ([M+H]).

Beispiel 8

440 mg N-Benzyl-2-(4-benzyloxy-2-phenylsulfonylamino-5-methoxy-phenyl)-2-[4-(N-hydroxycarbamimidoyl)-phenylamino]-acetamid (0.66 mmol) (Beispiel 14) wurden in 10 ml MeOH gelöst, mit einem Tropfen AcOH versetzt und mit 200 mg 5% Pt/C versetzt. Das Reaktionsgemisch wurde über Nacht hydriert. Der Katalysator wurde abfiltriert. Das Filtrat wurde im Vakuum eingedampft. Der Rückstand wurde chromatographisch an Kieselgel gereinigt (EtOAc/Aceton/$H_2O$/HOAc). Man erhielt 263 mg 2-(2-Phenylsulfonylamino-4-benzyloxy-5-methoxy-phenyl)-N-benzyl-2-(4-carbamimidoyl-phenylamino)-acetamid Acetat als farblosen Festkörper. ISP-NIS: 650 (M+H).

Beispeil 9

Analog zu Beispiel 8 erhielt man aus N-Benzyl-2-(4-benzyloxy-2-methansulfonylamino-5-methoxy-phenyl)-2-[4-(N-hydroxycarbamimidoyl)-phenylamino]-acetamid (Beispiel 15) nach Reinigung mit HPLC über eine RP-18 Säule mit einem $CH_3CN$/Wasser Eluenten mit 0,1% Trifluoressigsäure das N-Benzyl-2-(4-benzyloxy-2-methansulfonylamino-5-methoxy-phenyl)-2-(4-carbamimidoyl-phenylamino)-acetamid Trifluoroacetat als farblosen Festkörper. ISP-MS: 588 (M+H).

Beispiel 10

350 mg N-Benzyl-2-(4-benzyloxy-2-phenylsulfonylamino-5-methoxy-phenyl)-2-[4-(N-hydroxycarbamimidoyl)-phenylamino]-acetamid (0.53 mmol) (Beispiel 14) wurden in 3 ml AcOH gelöst, mit 100 ml $Ac_2O$ versetzt und in Gegenwart von 150 mg 5% Pd/C über Nacht hydriert. Der Katalysator wurde abfiltriert. Das Filtrat wurde im Vakuum eingedampft. Der Rückstand wurde chromatographisch an Kieselgel gereinigt (EtOAc/Aceton/$H_2O$/ HOAc). Man erhielt 188 mg (64%) 2-(2-Phenylsulfonylamino-4-hydroxy-5-methoxy-phenyl)-N-benzyl-2-(4-carbamimidoyl-phenylamino)-acetamid Acetat als farblosen Festkörper. ISP-MS: 560 (M+H).

Beispiel 11

In Analogie zu Beispiel 10 erhielt man aus N-Benzyl-2-(4-benzyloxy-2-methansulfonylamino-5-methoxy-phenyl)-2- [4-(N-hydroxycarbamimidoyl)-phenylamino]-acetamid (Beispiel 15) nach Reinigung durch HPLC über eine RP-18 Säule mit einem $CH_3CN$/Wasser-Eluenten mit 0.1% Trifluoressig-säure das N-Benzyl-2-(4-carbamimidoyl-phenylamino)-2-(4-hydroxy-2-methansulfonylamino-5-methoxy-phenyl)-acetamid Trifluoroacetat als farblosen Festkörper. ISP-MS: 498 (M+H).

Beispiel 12

Aus 115 mg (5 mmol) Natrium und 15 ml MeOH wurde unter Argonatmosphäre eine NaOMe-Lösung frisch

hergestellt und mit 420 mg (6 mmol) Hydroxylamin-hydrochlorid versetzt. Die Suspension wurde während 30 min bei Raumtemperatur gerührt. Nach Zugabe von 261 mg (0.5 mmol) (R,S)-N-Benzyl-2-(4-cyanophenylamino)-2-[3-methoxy-4-(4-nitro-benzyloxy)-phenyl]-acetamid (Beispiel 19 n) wurde das Reaktionsgemisch über Nacht unter Rückfluss erhitzt. Anschliessend wurde das Lösungsmittel im Vakuum abdestilliert. Der Rückstand wurde in Wasser aufgenommen und mit EtOAc extrahiert. Die organische Phase wurde mit gesättigter NaCl-Lösung gewaschen, über $MgSO_4$ getrocknet, filtriert und im Vakuum eingedampft. Der Rückstand wurde chromatographisch an Kieselgel gereinigt (EtOAc). Man erhielt 166 mg (60%) (R,S)-N-Benzyl-2-[4-(N-hydroxy-carbamimidoyl)-phenylamino]-2-[3-methoxy-4-(4-nitrobenzyloxy)-phenyl]-acetamid als farblosen, festen Schaum. ISP-MS: 556.3 ([M+H]).

Beispiel 13

In Analogie zu Beispiel 12 erhielt man

13.a) aus (R,S)-N-Benzyl-2-(4-cyano-phenylamino)-2-[3-methoxy-4-(pyridin-4-yl-methoxy)-phenyl]-acetamid (Beispiel 19.a) das (R,S)-N-Benzyl-2-[4-(Nhydroxycarbamimidoyl)-phenyl-amino]-2-[3-methoxy-4-(pyridin-4-yl-methoxy)-phenyl]-acetamid in 35% Ausbeute. Farbloser, fester Schaum. ISP-MS: 512.4 ([M+H]).

13.b) aus (R,S)-N-Benzyl-2-(4-cyano-phenylamino)-2-[3-methoxy-4-(2-morpholin-4-yl-ethoxy)-phenyl]-acetamid (Beispiel 19.b) das (R,S)-N-Benzyl-2-[4-(N-hydroxycarbamimidoyl)-phenylamino]-2-[3-methoxy-4-(2-morpholin-4-ylethoxy)-phenyl]-acetamid in 28% Ausbeute. Farbloser, fester Schaum. ISP-MS: 534.4 ([M+H]).

13.c) aus (R,S)-N--Benzyl-2-(4-cyano-phenylamino)-2-[3-methoxy-4-(3-phenoxy-benzyloxy)-phenyl]-acetamid (Beispiel 19.d) das (R,S)-N-Benzyl-2-[4-(Nhydroxycarbamimidoyl)-phenyl-amino]-2-[3-methoxy-4-(4-phenoxy-benzyloxy)-phenyl]-acetamid in 40% Ausbeute. Leicht grauer, fester Schaum. ISP-MS: 603.2 ([M+H]).

13.d) aus (R,S)-2-(4-Benzyloxy-3-methoxy-phenyl)-2-(4-cyano-phenylamino)-N-(4-nitrobenzyl)-acetamid (Beispiel 24.b) das (R,S)-2-(4-Benzyloxy-3-methoxyphenyl)-2-[4-(N-hydroxycarbamimidoyl)-phenylamino]-N-(4-nitro-benzyl)-acetamid in 98% Ausbeute. Gelbes Oel. ISP-MS: 545.2 ([M+Na]), 540.3 ([M+$NH_4$]), 523.3 ([M+H]).

13.e) aus (R,S)-2-(4-Benzyloxy-3-methoxy-phenyl)-2-(4-cyano-phenylamino-N-[2-(3H-imidazol-4-yl)-ethyl]-acetamid (Beispiel 24.e) das (R,S)-2-(4-Benzyloxy-3-methoxy-phenyl)-2-[4-(N-hydroxycarbamimidoyl)-phenylamino] -N-[2-(3Himidazol-4-yl)-ethyl]-acetamid in 85% Ausbeute. Gräulich-grüner, fester Schaum. ISP-MS: 515.3 ([M+H]).

13.f) aus (R,S)-2-(4-Benzyloxy-3-methoxy-phenyl)-2-(4-cyano-phenylamino)-N-[2-(1H-indol-3-yl)-ethyl]-acetamid (Beispiel 24.f) das (R,S)-2-(4-Benzyloxy-3-methoxy-phenyl)-2-[4-(N-hydroxycarbamimidoyl)-phenylamino] -N-[2-(1H-indol-3-yl)-ethyl]-acetamid in 85% Ausbeute. Gräulich-grüner, fester Schaum. ISP-NIS: 564.4 ([M+H]).

13.g) aus (R,S)-N-Benzyl-2-(4-cyano-phenylamino)-2-(3,4,5-trimethoxyphenyl)-acetamid (Beispiel 17.a) das (R, S)-N-Benzyl-2-[4-(N-hydroxycarbamimidoyl)-phenylamino]-2-(3,4,5-trimethoxy-phenyl)-acetamid als farblosen Feststoff. ISP-MS: 465.6 ($[M+H]^+$,100).

13.h) aus (R,S)-N-Benzyl-2-(3,5-dimethoxy-phenyl)-2-(4-cyanophenylamino)-acetamid (Beispiel 17.b), das (R,S) -N-Benzyl-2-(3,5-dimethoxyphenyl)-2-[4-(N-hydroxycarbamimidoyl)-phenylamino]-acetamid als farbloser Feststoff. ISP-MS: 435.5 ($[M+H]^+$, 100).

13.i) aus (R,S)-N-Benzyl-2-(4-benzyloxy-3-methoxy-phenyl)-2-(4-cyanophenylamino)-acetamid (Beispiel 17.c) das (R,S)-N-Benzyl-2-(4-benzyloxy-3-methoxy-phenyl)-2-[4-(N-hydroxycarbamimidoyl)-phenylamino]-acetamid als farbloser Feststoff. ISP-NIS: 511.6 ($[M+H]^+$, 100).

13.j) aus (R,S)-N-Benzyl-2-(4-benzyloxy-3-ethoxy-phenyl)-2-(4-cyanophenylamino)-acetamid (Beispiel 17.d) erhält man (R,S)-N-Benzyl-2-(4-benzyloxy-3-ethoxy-phenyl)-2- [4-(N-hydroxy-carbamimidoyl)-phenylamino]-acetamid als farbloser Feststoff. ISP-MS: 525.5 ($[M+H]^+$, 100).

Beispiel 14

995 mg 2-(2-Phenylsulfonylamino-4-benzyloxy-5-methoxy-phenyl)-N-benzyl-2-(4-cyanophenylamino)-acetamid (1.57 mmol) (Beispiel 28), 547 mg Hydroxylamin-hydrochlorid (7.87 mmol) und 2.2 ml Triaethylamin (15.7 mmol) wurden in 5 ml EtOH vorgelegt. Das Reaktionsgemisch wurde während 5 Stunden unter Rückfluss erhitzt. Anschliessend wurde das Lösungsmittel im Vakuum abdestilliert. Der Rückstand wurde in Wasser aufgenommen und mit EtOAc extrahiert. Die organische Phase wurde mit gesättigter NaCl-Lösung gewaschen, über $MgSO_4$ getrocknet, filtriert und im Vakuum eingedampft. Man erhielt 885 mg (85%) 2-(2-Phenylsulfonylamino-4-benzyloxy-5-methoxy-phenyl)-N-benzyl-2-[4-(N-hydroxycarbamimidoyl)-phenylamino]-acetamid als gelben öligen Festkörper. ISP-MS: 666 (M+H).

Beispiel 15

In Analogie zu Beispiel 14 erhielt man aus N-Benzyl-2-(4-benzyloxy-2-methansulfonylamino-5-methoxy-phenyl)-2-(4-cyano-phenylamino)-acetamid (Beispiel 29) das N-Benzyl-2-(4-benzyloxy-2-methansulfonylamino-5-methoxyphenyl)-2-[4-(N-hydroxycarbamimidoyl)-phenylamino]-acetamid als gelbes Oel in 95% Ausbeute. ISP-MS: 604 (M+H).

Beispiel 16

10 g 4-Benzyloxy-5-methoxy-2-nitro-benzaldehyd (34.8 mmol) und 4.11 g 4-Aminobenzonitril (34.8 mmol) wurden in 140 ml Methanol gelöst und während einer Stunde bei Raumtemperatur gerührt. Dann wurde mit 4.25 ml Benzylisonitril (34.8 mol) versetzt. Anschliessend wurden 12.9 ml Bortrifluoridetherat (104.4 mmol) unter Eisbadkühlung innert einer halben Stunde dazugetropft. Nach 30 Min. wurde das Eisbad entfernt. Das Reaktionsgemisch wurde während 2 Tagen bei Raumtemperatur nachgerührt. Anschliessend wurde das Lösungsmittel im Vakuum abdestilliert. Der Rückstand wurde in EtOAc aufgenommen und mit Wasser gewaschen. Die organische Phasewurde über $MgSO_4$ getrocknet, filtriert und im Vakuum eingedampft. Das Rohprodukt wurde chromatographisch an Kieselgel mit Aceton/Toluol (3/97) gereinigt. Man erhielt 8.9 g (48%) N-Benzyl-2-(4-benzyloxy-5-methoxy-2-nitrophenyl)-2-(4-cyano-phenylamino)-acetamid als orangen öligen Festkörper. ISP-MS: 523 (M+H).

Beispiel 17

Analog zu Beispiel 16 erhielt man:

17.a) Aus 4-Aminobenzonitril, 3,4,5-Trimethoxy-benzaldehyd und Benzylisonitril erhält man (R,S)-N-Benzyl-2-(4-cyano-phenylamino)-2-(3,4,5-trimethoxy-phenyl)-acetamid als farblose Kristalle. NMR-$^1$H (DMSO-$D_6$): 8.80 (t;NH;1H) 5.02 (d;CH;1H) 4.28 (d;$CH_2$;2H), 3.75 (s;$CH_3$;6H), 2.61 (s;$CH_3$;3H).

17.b) aus 4-Aminobenzonitril, 3,5-Dimethoxy-benzaldehyd und Benzylisonitril erhält man (R,S)-N-Benzyl-2-(3,5-dimethoxy-phenyl)-2-(4-cyanophenylamino)-acetamid als farblose Kristalle. NMR-$^1$H (DMSO-$D_6$): 8.82 (t;NH;1H) 5.15 (d;CH;1H), 4.28 (d;$CH_2$;2H), 3.72 (s;$CH_3$;6H).

17.c) Aus 4-Aminobenzonitril, 4-Benzyloxy-3-methoxy-benzaldehyd und Benzylisonitril erhält man (R,S)-N-Benzyl-2-(4-benzyloxy-3-methoxy-phenyl)-2-(4-cyano-phenylamino)-acetamid als farblose Kristalle. NMR-$^1$H (DMSO-$D_6$): 8.80 (t;NH;1H), 5.07 (d;CH;2H), 4.99 (d;CH;2H), 4.28 (s;$CH_2$;2H), 3.73 (s;$CH_3$;3H).

17.d) Aus 4-Aminobenzonitril, 4-Benzyloxy-3-ethoxy-benzaldehyd und Benzylisonitril erhält man (R,S)-N-Benzyl-2-(4-benzyloxy-3-ethoxy-phenyl)-2-(4-cyano-phenylamino)-acetamid als farblose Kristalle. NMR-$^1$H (DMSO-$D_6$): 8.75 (t;NH;1H), 5.10 (s;$CH_2$;2H), 4.95 (d;CH;1H) 4.28 (d;$CH_2$;2H), 4.05 (q;$CH_2$;2H), 1.32 (t;$CH_3$;3H).

Beispiel 18

Eine Lösung von 290 mg (0.75 mmol) (R,S)-N-Benzyl-2-(4-cyanophenylamino)-2-(4-hydroxy-3-methoxy-phenyl)-acetamid (Beispiel 20), 110 ml (0.9 mmol) 3-Methoxybenzylalkohol und 236 mg (0.9 mmol) Triphenylphosphin in 15 ml THF wurde unter Argonatmosphäre mit 140 ml (0.9 mmol) Diethylazodicarboxylat versetzt und über Nacht bei Raumtemperatur gerührt. Die Reaktionslösung wurde im Vakuum eingedampft. Der Rückstand wurde durch Chromatographie an Kieselgel gereinigt ($CH_2Cl_2$/EtOAc 9:1). Man erhielt 326 mg (86%) (R,S)-N-Benzyl-2-(4-cyano-phenylamino)-2-[3-methoxy-4-(3-methoxy-benzyloxy)-phenyl]-acetamid als Farblosen Festkörper. ISP-MS: 530.2 ([M+Na]), 525.2 ([M+$NH_4$]), 508.4 ([M+H]), 390.2.

Beispiel 19

In Analogie zu Beispiel 18 erhielt man aus (R,S)-N-Benzyl-2-(4-cyanophenylamino)-2-(4-hydroxy-3-methoxy-phenyl)-acetamid

19.a) und 4-Hydroxymethylpyridin das (R,S)-N-Benzyl-2-(4-cyanophenylamino-2-[3-methoxy-4-(pyridin-4-ylmethoxy)-phenyl]-acetamid in 72% Ausbeute. ISP-MS: 479.3 [M+H].

19.b) und N-(2-Hydroxyethyl)-morpholin das (R,S)-N-Benzyl-2-(4-cyanophenylamino)-2-[3-methoxy-4-(2-morpholin-4-yl-ethoxy)-phenyl]-acetamid in 66% Ausbeute. Farbloses Harz. ISP-MS: 501.3 [M+H].

19.c) und N-(2-Hydroxyethyl)-thiomorpholin (L.A. Burrows, E.E. Reid, Journal of American Chemical Society (1934) 56, 1720) das (R,S)-N-Benzyl-2-(4-cyano-phenylamino)-2-[3-methoxy-4-(2-thiomorpholin-4-ylethoxy)-phenyl]-acetamid in 84% Ausbeute. Farbloser, fester Schaum. ISP-MS 517.3 [M+H].

19.d) und 3-Phenoxybenzylalkohol das (R,S)-N-Benzyl-2-(4-cyanophenylamino)-2-[3-methoxy-4-(3-phenoxybenzyloxy)-phenyl]-acetamid in 44% Ausbeute. Farbloser, fester Schaum. ISN-MS: 628.3 ([M+AcOH-H]), 568.3 [M-H].

19.e) und 5-Hydroxymethylindol (M. Somei, Y. Saida, N. Komura, Chem. Pharm. Bull. (1986) 34, 4116) das (R,S) -N-Benzyl-2-(4-cyano-phenylamino)-2-[4-(1H-indol-5-ylmethoxy)-3-methoxy-phenyl]-acetamid in 67% Ausbeute. Hellgelber, fester Schaum. ISP-MS: 539.3 ([M+Na]), 534.3 ([$M+NH_4$]), 517.2 ([M+H], 399.3.

19.f) und 7-Hydroxymethylchinolin (C.E. Kaslow, W.R. Clark, Journal of Organic Chemistry (1953) 18, 55) das (R, S)-N-Benzyl-2-(4-cyano-phenylamino)-2-[3-methoxy-4-(chinolin-7-ylmethoxy)-phenyl]-acetamid in 100% Ausbeute. Farbloser Festkörper. ISP-MS: 529.2 [M+H].

19.g) und 2-Phenylethylalkohol das (R,S)-N-Benzyl-2-(4-cyanophenylamino)-2-(3-methoxy-4-phenethyloxy-phenyl)-acetamid in 90% Ausbeute. Hellgrünes Harz. ISP-MS: 492.2 [M+H].

19.h) und 4-Hydroxymethylbiphenyl das (R,S)-N-Benzyl-2-[4-(biphenyl-4-ylmethoxy)-3-methoxy-phenyl]-2-(4-cyano-phenylamino)-acetamid in 25% Ausbeute. Farbloser Festkörper. ISN-MS: 612.3 ([M+AcOH-H]), 552.2 [M-H].

19.i) und 2,6-Dichlorbenzylalkohol das (R,S)-N-Benzyl-2-(4-cyanophenylamino)-2-[4-(2,6-dichloro-benzyloxy)-3-methoxy-phenyl]-acetamid in 70% Ausbeute. Farblose Kristalle. ISN-MS: 604.1 ([M+AcOH-H]), 544.1 [M-H].

19.j) und 3,5-Dichlorbenzylalkohol das (R,S)-N-Benzyl-2-(4-cyanophenylamino)-2-[4-(3,5-dichloro-benzyloxy)-3-methoxy-phenyl]-acetamid in 74% Ausbeute. Farblose Kristalle. ISN-MS: 604.1 ([M+AcOH-H]), 544.1 [M-H].

19.k) und 3-Brombenzylalkohol das (R,S)-N-Benzyl-2-[4-(3-bromobenzyloxy)-3-methoxy-phenyl]-2-(4-cyano-phenylamino)-acetamid in 51% Ausbeute. Farblose Kristalle. ISN-MS: 614.2 ([M+AcOH-H]), 556.0 [M-H].

19.1) und 2-Hydroxymethylpyridin das (R,S)-N-Benzyl-2-(4-cyanophenylamino)-2-[3-methoxy-4-(pyridin-2-ylmethoxy)-phenyl]-acetamid in 89% Ausbeute. Farbloses Harz. ISP-MS: 479.3 [M+H].

19.m) und 6-Hydroxymethylisochinolin (EP 385 662) das (R,S)-N-Benzyl-2-(4-cyano-phenylamino)-2-[4-(isochinolin-6-ylmethoxy)-3-methoxy-phenyl]-acetamid in 100% Ausbeute. Farbloser Festkörper. ISP-MS: 529.3 [M+H].

19.n) und 4-Nitrobenzylalkohol das (R,S)-N-Benzyl-2-(4-cyanophenylamino)-2-[3-methoxy-4-(4-nitro-benzyloxy)-phenyl]-acetamid in 71% Ausbeute. Leicht gelber, fester Schaum ISN-MS: 581.2 ([M+AcOH-H]), 521.2 [M+H].

Beispiel 20

Zu einer Lösung von 8.5 g (20 mmol) (R,S)-2-(4-Allyloxy-3-methoxy-phenyl)-N-benzyl-2-(4-cyano-phenylamino)-acetamid (Beispiel 21) in 250 ml THF wurden 460 mg (0.4 mmol) Tetrakis(triphenylphosphin)-palladium gegeben. Die Lösung wurde während 10 min bei Raumtemperatur gerührt, dann mit 1.13 g (30 mmol) $NaBH_4$ versetzt. Nach 2 h Rühren bei Raumtemperatur wurde im Vakuum eingedampft. Der Rückstand wurde in $CH_2Cl_2$/EtOAc 9:1 aufgenommen und während 30 min in Gegenwart von Aktivkohle gerührt. Die Suspension wurde über Dicalit filtriert. Das Filtrat wurde im Vakuum eingedampft und der Rückstand aus EtOAc/Hexan umkristallisiert. Man erhielt 6.81 g (88%) (R,S) -N-Benzyl-2-(4-cyano-phenylamino)-2-(4-hydroxy-3-methoxy-phenyl)-acetamid als farblose Kristalle. ISN-MS: 386.1 ([M-H]), 268.3.

Beispiel 21

Eine Lösung von 17.8 g (35 mmol) (R,S)-2-(4-Allyloxy-3-methoxy-phenyl)-N-benzyl-2-(4-cyano-phenylamino)-acetamidosäuremethylester (Beispiel 22) in 35 ml DMSO wurde über Nacht bei Raumtemperatur gerührt. Das Lösungsmittel wurde im Hochvakuum abgedampft. Der Rückstand wurde durch Chromatographie an Kieselgel gereinigt ($CH_2Cl_2$/EtOAc 19:1). Man erhielt 10,6 g (71%) (R,S)-2-(4-Allyloxy-3-methoxy-phenyl)-N-benzyl-2-(4-cyano-phenylamino)-acetamid als farblosen Festkörper. ISN-MS: 486.1 ([M+AcOH-H]), 426.1 [M-H].

Beispiel 22

Eine Lösung von 17.72 g (150 mmol) 4-Aminobenzonitril und 28.8 g 4-Allyloxy-3-methoxybenzaldehyd (W. A. Ayer, P.A. Craw, Can. J. Chem. (1991) 69, 1909) wurde unter Argonatmosphäre während 1 h bei Raumtemperatur gerührt. Es wurden 18.3 ml (150 mmol) Benzylisonitril zugegeben. Anschliessend wurden bei 0°C 56 ml (450 mmol) Bortrifluorid-ethyletherat langsam zugetropft. Nach kurzer Zeit bildeten sich Kristalle, welche unter Argonatmosphäre abfiltriert und im Hochvakuum getrocknet wurden. Man erhielt 74.1 g (87%) (R,S)-2-(4-Allyloxy-3-methoxy-phenyl)-N-benzyl-2-(4-cyano-phenylamino)-acetimidosäuremethylester als leicht gelben Festkörper. ISP-MS: 442.3 [M+H].

Beispiel 23

Zu einer Lösung aus 194 mg (0.5 mmol) (R,S)-(4-Benzyloxy-3-methoxyphenyl)-(4-cyanophenylamino)-essigsäure (Beispiel 25), 75 ml (0.6 mmol) Phenethylamin, 115 mg (0.75 mmol) 1-Hydroxybenzotriazol und 128 ml (0.75 mmol) N,N-Diisopropylethylamin in 2.5 ml THF wurden unter Argonatmosphäre 144 mg (0.75 mmol) N-(3-Dimethylaminopropyl)-N'-ethyl-carbodiimidhydrochlorid gegeben. Das Reaktionsgemisch wurde während 5 h bei Raumtemperatur gerührt und dann im Vakuum eingeengt. Der Rückstand wurde in EtOAc aufgenommen und mit gesättigter $KHCO_3$-Lösung, mit 2%iger Zitronensäurelösung, mit $H_2O$ und dann mit gesättigter NaCl-Lösung gewaschen. Die organische Phase wurde über $MgSO_4$ getrocknet, filtriert und im Vakuum eingedampft. Das Rohprodukt wurde durch Chromatographie an Kieselgel gereinigt (Cyclohexan/EtOAc 2:1). Man erhielt 210 mg (86%) (R,S)-2-(4-Benzyloxy-3-methoxy-phenyl)-2-(4-cyano-phenylamino)-N-phenethyl-acetamid als farblosen, festen Schaum. ISP-MS: 492.3 ([M+Na]), 509.4 ([M+NH4]), 523.3 ([M+H]).

Beispiel 24

In Analogie zu Beispiel 23 erhielt man aus (R,S)-(4-Benzyloxy-3-methoxyphenyl)-(4-cyano-phenylamino)-essigsäure

24 a) und Veratrylamin das (R,S)-2-(4-Benzyloxy-3-methoxy-phenyl)-2-(4-cyano-phenylamino)-N-(3,4-dimethoxy-benzyl)-acetamid in 80% Ausbeute. Leicht gelber, fester Schaum. ISP-MS: 560.4 ([M+Na]), 555.3 ([M+NH4]), 538.4 ([M+H]).

24 b) und 4-Nitrobenzylamin-hydrochlorid das (R,S)-2-(4-Benzyloxy-3-methoxy-phenyl)-2-(4-cyano-phenylamino) -N-(4-nitro-benzyl)-acetamid in 43% Ausbeute. Farbloser Festkörper. ISP-MS: 545.2 ([M+Na]), 540.3 ([$M+NH_4$]), 523.2 ([M+H]).

24 c) und Anilin das (R,S)-2-(4-Benzyloxy-3-methoxy-phenyl)-2-(4-cyanophenylamino)-N-phenyl-acetamid in 52% Ausbeute. Farbloser Festkörper. ISP-MS: 486.2 ([M+Na]), 464.2 ([M+H]).

24 d) und 4-Aminobenzonitril das (R,S)-2-(4-Benzyloxy-3-methoxy-phenyl)-N-(4-cyano-phenyl)-2-(4-cyano-phenylamino)-acetamid in 10% Ausbeute. Farbloser Festkörper, ISN-MS:547.2 ([M+AcOH-H]), 487.2 [M-H]).

24 e) und Histamin-dihydrochlorid das (R,S)-2-(4-Benzyloxy-3-methoxyphenyl)-2-(4-cyano-phenylamino) -N-[2-(3H-imidazol-4-yl)-ethyl]-acetamid in 74% Ausbeute. Farbloser, fester Schaum. ISP-MS: 482.4 [M+H].

24 f) und Tryptamin das (R,S)-2-(4-Benzyloxy-3-methoxy-phenyl)-2-(4-cyano-phenylamino)-N-[2-(1H-indol-3-yl)-ethyl]-acetamid in 92% Ausbeute. Oranger, fester Schaum. ISP-MS: 553.3 ([M+Na]), 548.3 [M+NH4], 531.3 [M+H].

Beispiel 25

Eine Lösung von 1.17 g (2.9 mol) (R,S)-(4-Benzyloxy-3-methoxy-phenyl)-(4-cyano-phenylamino)-essigsäuremethylester in 6 ml THF wurden auf 0°C gekühlt und mit 14.5 ml (14.5 mmol) IN LiOH-Lösung versetzt. Man liess 30 min bei 0°C und 2 h bei Raumtemperatur rühren. Das THF wurde im Vakuum abdestilliert. Die Verbleibende Lösung wurde mit 1N HCl auf pH 3 gebracht. Dabei bildete sich ein farbloser Niederschlag, welcher abfiltriert und mit Wasser gewaschen wurde. Der Festkörper wurde in EtOH/$H_2O$ umkristallisiert. Man erhielt 788 mg (70%) (R,S)-(4-Benzyloxy-3-methoxy-phenyl)-(4-cyano-phenylamino)-essigsäure als farblosen Festkörper. ISN-MS: 387.1 [M-H]).

Beispiel 26

Eine Lösung von 970 mg (4 mmol) 4-Benzyloxy-3-methoxybenzaldehyd und 473 mg (4 mmol) 4-Aminobenzonitril in 16 ml MeOH wurden unter Argonatmosphäre während 1 h bei Raumtemperatur gerührt. Dabei bildete sich ein leicht gelber Niederschlag. Die Suspension wurde mit 488 ml (4 mmol) Benzylisonitril versetzt und auf 0°C gekühlt. Dann wurden 1.52 ml (12 mmol) Bortrifluorid-ethyletherat langsam zugetropft. Nach 2 h liess man auf Raumtemperatur aufwärmen und dampfte im Vakuum ein. Der Rückstand wurde in MeOH aufgenommen. Die Lösung wurde langsam mit Wasser versetzt, bis Kristallisation einsetzte. Mann liess über Nacht bei 4°C stehen. Der Feststoff wurde abfiltriert und im Hochvakuum getrocknet. Der Rückstand wurde aus Cyclohexan/EtOAc umkristallisiert. Man erhielt 1.17 g (73%) (R,S)-(4-Benzyloxy-3-methoxy-phenyl)-(4-cyano-phenylamino)-essigsäuremethylester als farblosen Festkörper. ISP-MS: 425 ([M+Na]), 420.2 ([M+NH4]).

Beispiel 27

2.0 g N-Benzyl-2-(4-benzyloxy-5-methoxy-2-nitro-phenyl)-2-(4-cyanophenylamino)-acetamid (3.8 mmol) (Beispiel 16) wurden in 50 ml EtOAc und 50 ml EtOH gelöst, mit 1.2 g 5% Pt/C versetzt und während 3 h hydriert. Der Katalysator wurde abfiltriert. Das Filtrat wurde im Vakuum eingedampft. Man erhielt 1.42 g (76%) 2-(2-Amino-4-benzyloxy-5-methoxy-phenyl)-N-benzyl-2-(4-cyanophenylamino)-acetamid als gelben öligen Festkörper. ISP-MS: 493 (M+H).

Beispiel 28

1.7 g 2-(2-Amino-4-benzyloxy-5-methoxy-phenyl)-N-benzyl-2-(4-cyanophenylamino)-acetamid (3.4 mmol) (Beispiel 27) wurden in 60 ml $CH_2Cl_2$ und 20 ml DMF gelöst und mit 0.7 ml Hünigsbase versetzt. Anschliessend wurden bei 0°C 0.7 ml Phenylsulfonylchlorid (3.8 mmol) zugetropft. Das Reaktionsgemisch wurde während 4 Stunden bei Raumtemperatur gerührt, anschliessend mit 100 ml $CH_2Cl_2$ verdünnt und mit 5% $NaHCO_3$-Lösung gewaschen. Die organische Phase wurde mit gesättigter NaCl-Lösung gewaschen, über $MgSO_4$ getrocknet, filtriert und im Vakuum eingedampft. Der Rückstand wurde chromatographisch an Kieselgel mit Aceton/Toluol (10/90) gereinigt. Man erhielt 995 mg (46%) 2-(2-Phenylsulfonylamino-4-benzyloxy-5-methoxy-phenyl)-N-benzyl-2-(4-cyano-phenylamino)-acetamid als orangen öligen Festkörper. ISN-MS: 631 (M-H).

Beispiel 29

In Analogie zu Beispiel 28 erhielt man aus 2-(2-Amino-4-benzyloxy-5-methoxy-phenyl)-N-benzyl-2-(4-cyanophenylamino)-acetamid (Beispiel 27) und Methansulfonylchlorid das N-Benzyl-2-(4-benzyloxy-2-methansulfonylamino-5-methoxy-phenyl)-2-(4-cyano-phenylamino)-acetamid als orangen Festkörper in 36% Ausbeute. ISN-MS: 569 (M-H).

Beispiel 30

0.2 mmol (R,S)-N-Benzyl-2-(4-benzyloxy-3-methoxy-phenyl)-2-(4-carbamimidoyl-phenylamino)-acetamid Trifluoroacetat (Beispiel 3) (120 mg) werden in 1.3 ml Tetrahydrofuran unter Argon bei 0°C mit 0.335 mmol O-Methyl-O'-(4-nitrophenyl)-kohlensäureester (66 mg) versetzt. Danach lässt man das Reaktionsgemisch über Nacht auf Raumtemperatur aufwärmen, entfernt das Lösungsmittel im Vakuum und extrahiert den Rückstand mit Essigester und Wasser. Die vereinigten organischen Phasen werden über Natriumsulfat getrocknet. Nach Filtrieren und Entfernen des Lösungsmittels schlemmt man in Diethylether auf und filtriert ab. Man erhält 88 mg (82%) [Amino-(4-{[benzylcarbamoyl-(4-benzyloxy-3-methoxy-phenyl)-methyl]-amino}phenyl)-methylen]-carbaminsäure Methylester als farblosen Feststoff. ISP-MS: 553.3 ($[M+H]^+$, 100).

Beispiel 31

Analog Beispiel 30 stellt man folgende Verbindungen her:

31 a) Aus (R,S)-N-Benzyl-2-(4-carbamimidoyl-phenylamino)-1-(3,4-dimethoxy-phenyl)-acetamid-hydrochlorid (Beispel 2) erhält man [Amino-(4-{[benzylcarbamoyl-(3,4-dimethoxy-phenyl)-methyl]-amino}-phenyl)-methylen]-carbaminsäure Methylester als farbloser Feststoff. ISP-MS: 477.5 ($[M+H]^+$, 100).

31 b) Aus (R,S)-N-Benzyl-2-(4-carbamimidoyl-phenylamino)-2-(3,5-dimethoxy-phenyl)-acetamid-hydrochlorid (Beispiel 5b) erhält man [Amino-(4-{[benzylcarbamoyl-(3,5-dimethoxy-phenyl)-methyl]-amino}-phenyl)-methylen]-carbaminsäure Methylester als farbloser Feststoff. ISP-MS: 477.5 ($[M+H]^+$, 100).

31 c) Aus (R,S)-N-Benzyl-2-(4-carbamimidoyl-phenylamino)-2-(3,4,5-trimethoxy-phenyl)-acetamid-hydrochlorid (Beispiel 5a) erhält man [Amino-(4-{[benzylcarbamoyl-(3,4,5-trimethoxy-phenyl)-methyl]-amino}-phenyl)-methylen]-carbaminsäure Methylester als farbloser Feststoff. ISP-MS: 507.6 ($[M+H]^+$, 100).

Eine Verbindung der Formel I, ein Hydrat, ein Solvat oder ein Salz davon kann als Wirkstoff zur Herstellung von pharmazeutischen Präparaten, wie die folgenden, verwendet werden.

Beispiel 32

Eine entgaste Lösung von 369 mg des nach Beispiel 36.3 hergestellten Materials in 5 ml EtOH, 5 ml THF, 2 ml $H_2O$ und 1 ml HOAc wurde mit einer Spatelspitze Raney-Nickel versetzt und während 5 Std hydriert. Der Katalysator wurde abfiltriert. Das Filtrat wurde eingeengt. Der Rückstand wurde chromatographisch an Kieselgel gereinigt (EtOAc/Aceton/$H_2O$/HOAc 6:2:1:1). Man erhielt so die beiden Epimeren:

1. (S)-2-[(R)-2-(4-Benzyloxy-3-methoxy-phenyl)-2-(4-carbamimidoylphenylamino)-acetylamino]-propionsäure-acetat (1:1) und

2. (S)-2-[(S)-2-(4-Benzyloxy-3-methoxy-phenyl)-2-(4-carbamimidoylphenylamino)-acetylamino]-propionsäure-acetat (1:1), beide als farblose Lyophilisate, ISP-MS: 477.3 [M+H].

Beispiel 33

Analog Bsp. 32 erhielt man

33.a
aus dem Epimerengemisch aus Bsp. 37.a.1:

1. das (S)-2-[(R)-2-(4-Benzyloxy-3-methoxy-phenyl)-2-(4-carbamimidoylphenylamino)-acetylamino]-3-phenyl-propionsäure-acetat (1:1) und aus dem Epimerengemisch aus Bsp. 37.a.

2. das (S)-2- [(S)-2-(4-Benzyloxy-3-methoxy-phenyl)-2-(4-carbamimidoylphenylamino)-acetylanuno]-3-phenyl-propionsäure-acetat (1:1), ISP-MS: 553.3 [M+H],

33.b
aus dem Produkt von Bsp. 37.b nach chromatografischer Trennung die beiden Epimeren:

1. (S)-2-[(R)-2-(4-Benzyloxy-3-methoxy-phenyl)-2-(4-carbamimidoylphenylamino)-acetylamino]-3-(4-hydroxy-phenyl)-propionsäure-acetat (1:2) und

2. (S)-2-[(S)-2-(4-Benzyloxy-3-methoxy-phenyl)-2-(4-carbamimidoylphenylamino)-acetylamino]-3-(4-hydroxy-phenyl)-propionsäure-acetat (1:1), ISP-MS: 569.3 [M+H],

33.c
aus dem Produkt von Bsp. 37.c eine Mischung von (S)-1-[(R)- und -[(S)-(4-Benzyloxy-3-methoxy-phenyl)-(4-carbamimidoyl-phenylamino)-acetyl]-pyrrolidin-2-carbonsäure-acetat, ISP-MS: 503.3 [M+H],

33.d
aus dem Produkt von Bsp. 37.d das (RS)-2-[2-(4-Benzyloxy-3-methoxy-phenyl)-2-(4-carbamimidoyl-phenylamino)-acetylamino]-2-methyl-propionsäure-acetat (1:0.5), ISP-MS: 491.5 [M+H], 513.5 [M+Na],

33.e
aus dem Produkt von Bsp. 37.e nach chromatografischer Trennung die beiden Epimeren:

1. (S)-[[(R)-(4-Benzyloxy-3-methoxy-phenyl)-(4-carbamimidoyl-phenylamino)-acetyl]-methyl-amino]-phenyl-essigsäure-acetat (1:2) und

2. (S)-[[(S)-(4-Benzyloxy-3-methoxy-phenyl)-(4-carbamimidoyl-phenylamino)-acetyl]-methyl-amino]-phenyl-essigsäure-acetat (1:1), ISP-MS: 553.3 [M+H], 575.1 [M+Na],

33.f
aus dem Produkt von Bsp. 37.f das Gemisch von (RS)- und (SR)-1-[(RS)-(4-Benzyloxy-3-methoxy-phenyl)-(4-carbamimidoyl-phenylamino)-acetyl]-pyrrolidin-3-carbonsäure, ISP-MS: 503.3 [M+H],

33.g
aus dem Produkt von Bsp. 37.g nach chromatografischer Trennung die beiden diastereomeren Racemate von (RS)- oder (SR)-2-[(RS)-(4-Benzyloxy-3-methoxyphenyl)-(4-carbamimidoyl-phenylamino)-acetyl]-1,2,3,4-tetrahydro-isochinolin-3-carbonsäure-acetat (1:1), ISP-MS: 565.3 [M+H], 587.2 [M+Na],

33.h
aus dem Produkt von Bsp. 37.h das (RS)-1-[2-(4-Benzyloxy-3-methoxy-phenyl)-2-(4-carbamimidoyl-phenylamino)-acetylamino]-cyclopentancarbonsäure-acetat (1:2), ISP-MS: 517.2 [M+H], 539.3 [M+Na],

33.i
aus dem Produkt von Bsp. 37.i das (RS)-[2-(4-Benzyloxy-3-methoxy-phenyl)-2-(4-carbamimidoyl-phenylamino)-acetylamino]-diphenyl-essigsäure-acetat (1:1), ISP-MS: 615.3 [M+H],

33.j
aus dem Produkt von Bsp. 37.j nach chromatografischer Trennung die beiden Epimeren:

1. (S)-2-[(R)-2-(4-Benzyloxy-3-methoxy-phenyl)-2-(4-carbamimidoylphenylamino)-acetylamino]-4-tert-butoxycarbonylamino-buttersäure-acetat (1:3) und

2. (S)-2-[(S)-2-(4-Benzyloxy-3-methoxy-phenyl)-2-(4-carbamimidoylphenylamino)-acetylamino]-4-tert-butoxycarbonylamino-buttersäure-acetat (1:3), ISP-MS: 606.1 [M+H], 628.2 [M+H],

33.k
aus dem Produkt von Bsp. 37.k nach chromatografischer Trennung die beiden Epimeren:

1. (S)-2-[(R)-2-(4-Benzyloxy-3-methoxy-phenyl)-2-(4-carbamimidoylphenylamino)-acetylamino]-3-tert-butoxycarbonylamino-propionsäure-acetat (1:1) und
2. (S)-2-[(S)-2-(4-Benzyloxy-3-methoxy-phenyl)-2-(4-carbamimidoylphenylamino)-acetylamino]-3-tert-butoxycarbonylamino-propionsäure-acetat (1:1), ISP-MS: 592.2 [M+H].

<u>Beispiel 34</u>

Analog Bsp. 32 erhielt man

34.a
aus dem Produkt von Bsp. 38.a das (RS)-2-[2-(4-Benzyloxy-3-methoxy-phenyl)-2-(4-carbamimidoyl-phenylamino)-acetylamino]-benzoesäure-acetat (1:1), ISP-MS: 525.1 [M+H],

34.b
aus dem Produkt von Bsp. 38.b das (RS)-3-[2-(4-Benzyloxy-3-methoxy-phenyl)-2-(4-carbamimidoyl-phenylamino)-acetylamino]-benzoesäure-acetat (1:1), ISP-MS: 525.1 [M+H],

34.c
aus dem Produkt von Bsp. 38.c das (RS)-4-[2-(4-Benzyloxy-3-methoxy-phenyl)-2-(4-carbamimidoyl-phenylamino)-acetylamino]-benzoesäure-acetat (1:1), ISP-MS: 525.1 [M+H].

Beispiel 35

Analog Bsp. 32 erhielt man aus dem Produkt von Bsp. 39 das Gemisch aus (RS)- und (SR)-3-[(RS)-2-(4-Benzyloxy-3-methoxy-phenyl)-2-(4-carbamimidoylphenylamino)-acetylamino]-3-phenyl-propionsäure.

Beispiel 36

36.1
Zu einer Lösung von 777 mg des in Beispiel 25 hergestellten Materials in 10 ml DMF wurden 335 mg L-Alaninmethylester-hydrochlorid, 1.03 ml Diisopropylethylamin und 1.06 g (Benzotriazol-1-yloxy)-tris-(dimethylamino)-phosphonium-hexafluoro-phosphat gegeben. Die Reaktionslösung wurde während 2 Std. bei RT gerührt und anschliessend im Hochvakuum eingedampft. Der Rückstand wurde in Essigester aufgenommen und dann mit 10%iger $KHCO_3$-Lösung, mit 2%iger Zitronensäure, Wasser und gesättigter NaCl-Lösung gewaschen. Die organische Phase wurde über $MgSO_4$ getrocknet und im Vakuum eingeengt. Der Rückstand wurde chromatographisch an Kieselgel gereinigt. Man erhielt 823 mg (87%) einer 1:1 Mischung von (S)-2-[(R)- und -[(S)-2-(4-benzyloxy-3-methoxy-phenyl)-2-(4-cyano-phenylamino)-acetylamino]-propionsäuremethylester als festen, farblosen Schaum. ISP-MS: 474.3 [M+H], 496.1 [M+Na], 512.2 [M+K].

36.2
Zu einer auf 0°C gekühlten Lösung von 792 mg des nach Bsp 36.1 hergestellten Materials in 10 ml THF wurden 8.4 ml 1N LiOH-Lösung gegeben. Es wurde während 30 min bei 0°C und während 2 Std. bei RT gerührt. Das THF wurde im Vakuum abdestilliert. Die verbleibende wässrige Lösung wurde mit 1N HCl angesäuert, wobei sich ein farbloser Niederschlag bildete. Dieser wurde abfiltriert und im Hochvakuum getrocknet. Man erhielt 712 mg (91%) eines 1:1 Gemisches von (S)-2-[(R)- und -[(S)-2-(4-benzyloxy-3-methoxy-phenyl)-2-(4-cyanophenylamino)-acetylamino]-propionsäure als farblosen Feststoff. ISN-MS: 458.3 [M-H].

36.3
Zu einer Suspension von 1.04 g Hydroxylamin-hydrochlorid in 25 ml EtOH wurden 4.1 ml Triethylamin und dann 683 mg des nach Bsp 36.2 hergestellten Materials gegeben. Das Reaktionsgemisch wurde über Nacht unter Rückfluss erhitzt und anschliessend im Vakuum eingedampft. Der Rückstand wurde chromatographisch an Kieselgel gereinigt (EtOAc/Aceton/$H_2O$/HOAc 6:2:1:1). Man erhielt 625 mg (86%) eines 1:1 Gemisches von (E)- und/oder (Z)-(S)-2-[(R)-and -[(S)-2-(4-Benzyloxy-3-methoxy-phenyl)-2-[4-(N-hydroxycarbamimidoyl)-phenylamino]-acetylamino]-propionsäure als leicht rosa gefärbtes Lyophilisat. ISP-MS: 493.3 [M+H], 515.3 [M+Na].

Beispiel 37

Analog Bsp. 36 erhielt man aus dem in Bsp 25 hergestellten Material

37.a
und L-Phenylalaninmethylester-hydrochlorid eine 1:1 Mischung von (S)-2-[(R)-und -[(S)-2-(4-Benzyloxy-3-methoxy-phenyl)-2-(4-cyano-phenylamino)-acetylamino]-3-phenyl-propionsäure-methylester, daraus das 1:1 Gemisch (S)-2-[(R)- und -[(S)-2-(4-Benzyloxy-3-methoxy-phenyl)-2-(4-cyano-phenylamino)-acetylamino]-3-phenylpropionsäure und daraus nach chromatografischer Trennung die beiden Produkte:

1. (E)- und/oder (Z)-(S)-2-[(R)-2-(4-Benzyloxy-3-methoxy-phenyl)-2-[4-(Nhydroxycarbamimidoyl)-phenylamino]-acetylamino]-3-phenyl-propionsäure und

2. (E)- und/oder (Z)-(S)-2-[(S)-2-(4-Benzyloxy-3-methoxy-phenyl)-2-[4-(Nhydroxycarbamimidoyl)-phenylamino]-acetylamino]-3-phenyl-propionsäure,

37.b
und L-Tyrosinethylester-hydrochlorid das 1:1 Gemisch von (S)-2-[(R)- und -[(S)-2-(4-Benzyloxy-3-methoxy-phenyl)-2-(4-cyano-phenylamino)-acetylamino]-3-(4-hydroxy-phenyl)-propionsäure-ethylester, daraus ein 1:1 Gemisch von (S)-2-[(R)-und -[(S)-2-(4-Benzyloxy-3-methoxy-phenyl)-2-(4-cyano-phenylamino)-acetylamino]-3-(4-hydroxy-phenyl)-propionsäure und daraus das 1:1 Gemisch von (E)- und/oder (Z)-(S)-2-[(R)- und -[(S)-2-(4-Benzyloxy-3-methoxy-phenyl)-2-[4-(Nhydroxycarbamimidoyl)-phenylamino]-acetylamino]-3-(4-hydroxy-phenyl)-propionsäure,

37.c

und L-Prolinmethylester ein 1:1 Gemisch von (S)-1-[(R)- und -[(S)-(4-Benzyloxy-3-methoxy-phenyl)-(4-cyano-phenylamino)-acetyl]-pyrrolidin-2-carbonsäuremethylester, daraus das Gemisch aus (S)-1-[(R)- und -[(S)-(4-Benzyloxy-3-methoxy-phenyl)-(4-cyano-phenylamino)-acetyl]-pyrrolidin-2-carbonsäure und daraus das 1:1 Gemisch aus (E)- und/oder (Z)-(S)-1-[(R)- und -[(S)-(4-Benzyloxy-3-methoxy-phenyl)- [4-(N-hydroxycarbamimidoyl)-phenylamino]-acetyl] -pyrrolidin-2-carbonsäure,

37.d

und 2-Amino-isobuttersäure-methylester-hydrochlorid den (RS)-2-[2-(4-Benzyloxy-3-methoxy-phenyl)-2-(4-cyano-phenylamino)-acetylamino] -2-methylpropionsäure-methylester, daraus die (RS)-2-[2-(4-Benzyloxy-3-methoxy-phenyl)-2-(4-cyano-phenylamino)-acetylamino]-2-methyl-propionsäure und daraus die (E)- und/oder (Z) -(RS)-2-[2-(4-Benzyloxy-3-methoxy-phenyl)-2-[4-(Nhydroxycarbamimidoyl)-phenylamino]-acetylamino]-2-methyl-propionsäure,

37.e

und N-Methyl-L-phenylglycin-ethylester-hydrochlorid ein 1:1 Gemisch von (S)-[[(R)- und -[[(S)-(4-Benzyloxy-3-methoxy-phenyl)-(4-cyano-phenylamino)-acetyl]-methyl-amino]-phenyl-essigsäure-ethylester, daraus die 1:1 Mischung von (S)-[[(R)- und [[(S)-(4-Benzyloxy-3-methoxy-phenyl)-(4-cyano-phenylamino)-acetyl]-methyl-aminol-phenyl-essigsäure und daraus das Gemisch (E)- und/oder (Z)-(S)-[[(R)- und -[[(S)-(4-Benzyloxy-3-methoxy-phenyl)-[4-(N-hydroxycarbamimidoyl)-phenylamino]-acetyl]-methyl-amino]-phenyl-essigsäure,

37.f

und beta-D,L-Prolin-ethylester-hydrochlorid eine Mischung von (RS)- und (SR)-1-[(RS)-(4-Benzyloxy-3-methoxy-phenyl)-(4-cyano-phenylamino)-acetyl]-pyrrolidin-3-carbonsäure-ethylester, daraus das Gemisch von (RS)- und (SR)-1-[(RS)-(4-Benzyloxy-3-methoxy-phenyl)-(4-cyano-phenylamino)-acetyl]-pyrrolidine-3-carbonsäure und daraus das Gemisch (E)- und/oder (Z)-(RS)- und -(SR)-1-[(RS)-(4-Benzyloxy-3-methoxy-phenyl)-[4-(N-hydroxy-carbamimidoyl)-phenylamino]-acetyl]-pyrrolidin-3-carbonsäure,

37.g

und 1,2,3,4-Tetrahydro-3-isochinolin-carbonsäure-ethylester-hydrochlorid ein Gemisch von (RS)- und (SR)-2-[(RS)-(4-Benzyloxy-3-methoxy-phenyl)-(4-cyanophenylamino)-acetyl]-1,2,3,4-tetrahydro-isochinolin-3-carbonsäure-ethylester, daraus das Gemisch (RS)- und (SR)-2-[(RS)-(4-Benzyloxy-3-methoxy-phenyl)-(4-cyano-phenylamino)-acetyl]-1,2,3,4-tetrahydro-isochinolin-3-carbonsäure und daraus das Gemisch (E)- und/oder (Z)-(RS)- und -(SR)-2-[(RS)-(4-Benzyloxy-3-methoxy-phenyl)-[4-(N-hydroxycarbamimidoyl)-phenylamino]-acetyl]-1,2,3,4-tetrahydro-isochinolin-3-carbonsäure,

37.h

und 1-Amino-1-cyclopentan-carbonsäure-ethylester-hydrochlorid den (RS)-1-[2-(4-Benzyloxy-3-methoxy-phenyl)-2-(4-cyano-phenylamino)-acetylamino]-cyclopentanecarbonsäure-ethylester,darausdie(RS)-1-[2-(4-Benzyloxy-3-methoxy-phenyl)-2-(4-cyano-phenylamino)-acetylamino]-cyclopentancarbonsäure und daraus die (E)- und/oder (Z)-(RS)-1-[2-(4-Benzyloxy-3-methoxy-phenyl)-2-[4-(N-hydroxycarbamimidoyl)-phenylamino]acetylamino]-cyclopentancarbonsäure,

37.i

und $\alpha,\alpha$-Diphenylglycin-methylester-hydrochlorid den (RS)-[2-(4-Benzyloxy-3-methoxy-phenyl)-2-(4-cyano-phenylamino)-acetylamino]-diphenyl-essigsäuremethylester, daraus die (RS)-[2-(4-Benzyloxy-3-methoxy-phenyl)-2-(4-cyanophenylamino)-acetylamino]-diphenyl-essigsäure und daraus die (E)- und/oder (Z)-(RS)-[2-(4-Benzyloxy-3-methoxy-phenyl)-2-[4-(N-hydroxycarbamimidoyl)-phenylamino]-acetylamino]-diphenyl-essigsäure,

37.j

und N-$\gamma$-Boc-L-$\alpha,\gamma$-Diaminobuttersäure-methylester-hydrochlorid eine 1:1 Mischung aus (S)-2-[(R)- und -[(S)-2-(4-Benzyloxy-3-methoxy-phenyl)-2-(4-cyanophenylamino)-acetylamino]-4-tert-butoxycarbonylamino-buttersäuremethylester, daraus das 1:1 Gemisch (S)-2-[(R)- und -[(S)-2-(4-Benzyloxy-3-methoxy-phenyl)-2-(4-cyano-phenylamino)-acetylamino]-4-tert-butoxycarbonylamino-butersäure und daraus das 1:1 Gemisch (S)-2-[(R)- und -[(S)-2-(4-Benzyloxy-3-methoxy-phenyl)-2-[4-[(E)- und/oder -[(Z)-N-hydroxycarbamimidoyl]-phenylamino]-acetylamino] -4-tert-butoxycarbonylamino-buttersäure,

37.k
und N-β-Boc-L-α,β-Diaminopropionsäure-methylester-hydrochlorid ein 1:1 Gemisch von (S)-2-[(R)- und -[(S)-2-(4-Benzyloxy-3-methoxy-phenyl)-2-(4-cyanophenylamino)-acetylamino]-3-tert-butoxycarbonylamino-propionsäuremethylester, daraus das 1:1 Gemisch (S)-2-[(R)- und -[(S)-2-(4-Benzyloxy-3-methoxy-phenyl)-2-(4-cyano-phenylamino)-acetylamino]-3-tert-butoxycarbonylamino-propionsäure und daraus das 1:1 Gemisch (S)-2-[(R)- und -[(S)-2-(4-Benzyloxy-3-methoxy-phenyl)-2-[4-[(E)- und/oder -[(Z)-N-hydroxycarbamimidoyl]-phenylamino]-acetylamino] -3-tert-butoxycarbonylaminopropionsäure.

Beispiel 38

Analog Bsp. 36 erhielt man aus dem in Bsp 25 hergestellten Material

38.a
und 2-Aminobenzoesäure-ethylester den (RS)-2-[2-(4-Benzyloxy-3-methoxyphenyl)-2-(4-cyano-phenylamino)-acetylamino]-benzoesäure-ethylester, daraus die (RS)-2-[2-(4-Benzyloxy-3-methoxy-phenyl)-2-(4-cyano-phenylamino)-acetylamino]-benzoesäure und daraus die (E)- und/oder (Z)-(RS)-2-[2-(4-Benzyloxy-3-methoxy-phenyl)-2-[4-(N-hydroxycarbamimidoyl)-phenylamino]-acetylamino]-benzoesäure,

38.b
und 3-Aminobenzoesäure-ethylester den (RS)-3-[2-(4-Benzyloxy-3-methoxyphenyl)-2-(4-cyano-phenylamino)-acetylamino]-benzoesäure-ethylester, daraus die (RS)-3-[2-(4-Benzyloxy-3-methoxy-phenyl)-2-(4-cyano-phenylamino)-acetylamino]-benzoesäure und daraus die (E)- und/oder (Z)-(RS)-3-[2-(4-Benzyloxy-3-methoxy-phenyl)-2-[4-(N-hydroxycarbamimidoyl)-phenylamino]-acetylamino]-benzoesäure,

38.c
und 4-Aminobenzoesäure-ethylester den (RS)-4-[2-(4-Benzyloxy-3-methoxyphenyl)-2-(4-cyano-phenylamino)-acetylamino]-benzoesäure-ethylester, daraus die (RS)-4-[2-(4-Benzyloxy-3-methoxy-phenyl)-2-(4-cyano-phenylamino)-acetylamino]-benzoesäure und daraus die (E)- und/oder (Z)-(RS)-4-[2-(4-Benzyloxy-3-methoxy-phenyl)-2-[4-(N-hydroxycarbamimidoyl)-phenylamino]-acetylamino]-benzoesäure.

Beispiel 39

Analog Bsp. 36 erhielt man aus dem in Bsp 25 hergestellten Material und D,L-3-Amino-3-phenylpropionsäure-methylester-hydrochlorid die Diastereomerengemische der entsprechenden Nitril-Ester, daraus die entsprechenden Nitril-Säuren und schliesslich das Gemisch (E)- und/oder (Z)-(RS)- und -(SR)-3-[(RS)-2-(4-Benzyloxy-3-methoxy-phenyl)-2-[4-(N-hydroxycarbamimidoyl)-phenylamino]-acetylamino]-3-phenyl-propionsäure.

Beispiel 40

40.a
Eine Suspension von 107 mg des unter Bsp. 33.j erhaltenen Epimerengemisches in 3 ml $CH_2Cl_2$ wurde mit 0.5 ml Trifluoressigsäure versetzt und während 1 Std. bei RT gerührt. Das Reaktionsgemisch wurde eingedampft. Der Rückstand wurde chromatographisch an Kieselgel gereinigt (EtOAc/Aceton/$H_2O$/HOAc 6:2:2:4). Man erhielt 79 mg (85%) einer Mischung von (S)-4-Amino-2-[(R)- und -[(S)-2-(4-benzyloxy-3-methoxy-phenyl)-2-(4-carbamimidoyl-phenylamino)-acetylamino]-buttersäure-acetat (1:1) als beiges Lyophilisat.

40.b
Analog Bsp. 40.a erhielt man aus dem unter Bsp. 33.k erhaltenen Epimerengemisch das eine 1:1 Mischung von (S)-3-Amino-2-[(R)- und -[(S)-2-(4-benzyloxy-3-methoxy-phenyl)-2-(4-carbamimidoyl-phenylamino)-acetylamino]-propionsäureacetat (1:1) in 44% Ausbeute als beiges Lyophilisat.

Beispiel 41

41.a

Analog Bsp. 32 erhielt man via (E)- und/oder (Z)-(S)-[(R)- oder -[(S)-2-(4-Benzyloxy-3-methoxy-phenyl)-2-[4-(N-hydroxycarbamimidoyl)-phenylamino]-acetylamino]-phenyl-essigsäure:

a) die (S)-[(R)-2-(4-Benzyloxy-3-methoxy-phenyl)-2-(4-carbamimidoylphenylamino)-acetylamino]-phenyl-essigsäure und

b) die (S)-[(S)-2-(4-Benzyloxy-3-methoxy-phenyl)-2-(4-carbamimidoylphenylamino)-acetylamino]-phenyl-essigsäure.

41.b
Das in 41.a eingesetzte Amidoxim wurde analog Bsp. 37 ausgehend von nach Bsp. 25 erhaltenem Material und (S)-Phenylglycine-methylester über die entsprechenden Nitril-Ester und daraus den entsprechenden Nitril-Säuren hergestellt.

Beispiel 42

42.a
Analog Bsp. 41, jedoch unter Verwendung von (R)-Phenylglycin-methylesterhydrochlorid anstelle von (S)-Phenylglycin-methylester-hydrochlorid, erhält man die beiden Epimeren

1. (R)-[(R)-2-(4-Benzyloxy-3-methoxy-phenyl)-2-(4-carbamimidoylphenylamino)-acetylamino]-phenyl-essigsäure und

2. (R)-[(S)-2-(4-Benzyloxy-3-methoxy-phenyl)-2-(4-carbamimidoylphenylamino)-acetylamino]-phenyl-essigsäure.

42.b
Analog Bsp. 41, jedoch unter Verwendung von p-Amino-L-phenylalaninethylester-dihydrochlorid anstelle von (S)-Phenylglycin-methylesterhydrochlorid, erhält man das 1:1 Gemisch (S)-3-(4-Amino-phenyl)-2-[(R)- und - [(S)-2-(4-benzyloxy-3-methoxy-phenyl)-2-(4-cyano-phenylamino)-acetylamino]-propionsäure-ethylester, daraus

1. das 1:1 Gemisch (E)- und/oder (Z)-(S)-3-(4-Amino-phenyl)-2-[(R)- und -[(S)-2-(4-benzyloxy-3-methoxy-phenyl)-2-[4-(N-hydroxycarbamimidoyl)-phenylamino]-acetylamino]-propionsäure-ethylester, daraus

2. das 1:1 Gemisch (S)-3-(4-Amino-phenyl)-2-[(R)- und -[(S)-2-(4-benzyloxy-3-methoxy-phenyl)-2-(4-carbamimidoyl-phenylamino)-acetylamino]-propionsäureethylester acetat (1:1) und schliesslich daraus nach chromatografischer Trennung

3. das (S)-3-(4-Amino-phenyl)-2-[(R)-2-(4-benzyloxy-3-methoxy-phenyl)-2-(4-carbamimidoyl-phenylamino)-acetylamino]-propionsäure-acetat (1:3) und

4. das (S)-3-(4-Amino-phenyl)-2-[(S)-2-(4-benzyloxy-3-methoxy-phenyl)-2-(4-carbamimidoyl-phenylamino)-acetylamino]-propionsäure-acetat (1:3), ISP-MS: 568.3 [M+H], 590.3 [M+Na].

Beispiel 43

43.1
Analog Bsp. 26 erhielt man aus 3,5-Dimethoxybenzaldehyd, 4-Aminobenzonitril und Benzylisonitril den (RS)-(4-Cyano-phenylamino)-(3,5-dimethoxy-phenyl)-essigsäure-methylester.

43.2
Analog Bsp. 25 erhielt man aus dem unter 43.1 hergestellten Produkt die (RS)-(4-Cyano-phenylamino)-(3,5-dimethoxy-phenyl)-essigsäure.

43.3
Analog Bsp. 41 erhielt man aus der unter 43.2 hergestellten Säure und dem (S)-Phenylglycin-methylester-hydrochlorid nach chromatografischer Trennung des Epimerengemisches die beiden Produkte

a) (S)-[(R)-2-(4-Carbamimidoyl-phenylamino)-2-(3,5-dimethoxy-phenyl)-acetylamino]-phenyl-essigsäure-acetat (1:1.6) und

b) (S)-[(S)-2-(4-Carbamimidoyl-phenylamino)-2-(3,5-dimethoxy-phenyl)-acetylamino]-phenyl-essigsäure-acetat (1:1.75), ISP-MS: 463.2 [M+H].

Beispiel 44

Analog Bsp. 26 erhielt man aus 3,4-Diethoxybenzaldehyd, 4-Aminobenzonitril und Benzylisonitril die (RS)-(4-Cyano-phenylamino)-(3,4-diethoxy-phenyl)-essigsäure.

Beispiel 45

Eine entgaste Lösung von 147 mg des Produktes aus Bsp. 47.b in 5 ml EtOH, 5 ml THF, 2 ml $H_2O$ und 1 ml HOAc wurde mit einer Spatelspitze Raney-Nickel versetzt und während 5 Std hydriert. Der Katalysator wurde abfiltriert. Das Filtrat wurde eingeengt. Der Rückstand wurde chromatographisch an Kieselgel gereinigt (EtOAc/Aceton/$H_2O$/HOAc 6:2:1:1). Man erhielt 80 mg (56%) (RS)-2-(4-Benzyloxy-3-methoxy-phenyl)-2-(4-carbamimidoyl-phenylamino)-N-[2-(3,4-dihydroxy-phenyl)-ethyl]-acetamid-acetat (1:1) als farbloses Lyophilisat. ISP-MS: 541.2 [M+H].

Beispiel 46

In Analogie zu Bsp. 45 erhielt man

46.a
aus dem Produkt von Bsp. 48.a das (RS)-N-Benzyl-2-(4-benzyloxy-3-methoxyphenyl)-2-(4-carbamimidoyl-phenylamino)-N-methyl-acetamid, ISP-MS: 509.5 [M+H],

46.b
aus dem Produkt von Bsp. 48.b das (RS)-2-(4-Benzyloxy-3-methoxy-phenyl)-2-(4-carbamimidoyl-phenylamino)-N-[2-(4-sulfamoyl-phenyl)-ethyl]-acetamid, ISP-MS: 588.4 [M+H],

46.c
aus dem Produkt von Bsp. 48.c das (RS)-2-(4-Benzyloxy-3-methoxy-phenyl)-2-(4-carbamimidoyl-phenylamino)-N-(2-pyridin-2-yl-ethyl)-acetamid-acetat (1:1), ISP-MS: 510.3 [M+H],

46.d
aus dem Produkt von Bsp. 48.d das (RS)-N-[2-(4-Amino-phenyl)-ethyl]-2-(4-benzyloxy-3-methoxy-phenyl)-2-(4-carbamimidoyl-phenylamino)-acetamid-acetat (1:1), ISP-MS: 523.4 [M+H],

46.e

1. aus dem Diastereomeren aus Bsp. 48.e.1 das entsprechende (R)-2-(4-Benzyloxy-3-methoxy-phenyl)-2-(4-carbamimidoyl-phenylamino)-N-[(R)-2-hydroxy-1-phenyl-ethyl]-acetamid-acetat (1:1) und

2. aus dem Diastereomeren aus Bsp. 48.e.2 das entsprechende (S)-2-(4-Benzyloxy-3-methoxy-phenyl)-2-(4-carbamimidoyl-phenylamino)-N- [(R)-2-hydroxy-1-phenyl-ethyl]-acetamid-acetat (1:1, ISP-MS: 525.2 [M+H],

46.f

1. aus dem Diastereomeren aus Bsp. 48.f.1 das entsprechende (R)-2-(4-Benzyloxy-3-methoxy-phenyl)-2-(4-carbamimidoyl-phenylamino)-N-[(S)-2-hydroxy-1-phenyl-ethyl]-acetamid-acetat (1:1) und

2. aus dem Diastereomeren aus Bsp. 48.f.2 das entsprechende (S)-2-(4-Benzyloxy-3-methoxy-phenyl)-2-(4-carbamimidoyl-phenylamino)-N-[(S)-2-hydroxy-1-phenyl-ethyl]-acetamid-acetat (1:1), ISP-MS: 525.4 [M+H],

46.g

1. aus dem Diastereomeren aus Bsp. 48.g.1 das entsprechende (R)-2-(4-Benzyloxy-3-methoxy-phenyl)-2-(4-carbamimidoyl-phenylamino)-N-[(R)-1-phenylethyl]-acetamid-acetat (1:1) und

2. aus aus dem Diastereomeren aus Bsp. 48.g.2 das entsprechende (S)-2-(4-Benzyloxy-3-methoxy-phenyl)-2-(4-carbamimidoyl-phenylamino)-N- [(R)-1-phenylethyl]-acetamid-acetat (1:1), ISP-MS: 509.4 [M+H],

46.h

1. aus dem Diastereomeren aus Bsp. 48.h.1 das entsprechende (R)-2-(4-Benzyloxy-3-methoxy-phenyl)-2-(4-carbamimidoyl-phenylamino)-N-[(S)-1-phenylethyl]-acetamid-acetat (1:1) und

2. aus aus dem Diastereomeren aus Bsp. 48.h.2 das entsprechende (S)-2-(4-Benzyloxy-3-methoxy-phenyl)-2-(4-carbamimidoyl-phenylamino)-N-[(S)-1-phenylethyl]-acetamid-acetat (1:1), ISP-MS: 509.4 [M+H].

Beispiel 47

47.a
Analog Bsp. 36.1 erhielt man aus 388 mg des Produkts aus Bsp. 25 und 184 mg 3-Hydroxythyramin 323 mg (62%) (RS)-2-(4-Benzyloxy-3-methoxy-phenyl)-2-(4-cyano-phenylamino)-N-[2-(3,4-dihydroxy-phenyl)-ethyl]-acetamid als festen farblosen Schaum. ISN-MS: 522.1 [M-H].

47.b
Analog Bsp. 36.3 erhielt man aus 258 mg des unter Bsp. 47.a erhaltenen Produktes 222 mg (81%) des (RS)-2-(4-Benzyloxy-3-methoxy-phenyl)-2-(4-cyanophenylamino)-N-[2-(3,4-dihydroxy-phenyl)-ethyl]-acetamides als farbloses Pulver. ISP-MS: 557.2 [M+H].

Beispiel 48

Analog dem Verfahren beschrieben in Beispiel 36.1 und 36.3 wurden folgende Verbindungen hergestellt:
Aus dem Produkt von Beispiel 25:

48.a
und N-Benzylmethylamin, über das Zwischenprodukt (RS)-N-Benzyl-2-(4-benzyloxy-3-methoxy-phenyl)-2-(4-cyano-phenylamino)-N-methyl-acetamid das (E)- und/oder (Z)-(RS)-N-Benzyl-2-(4-benzyloxy-3-methoxy-phenyl)-2-[4-(Nhydroxycarbamimidoyl)-phenylamino]-N-methyl-acetamid,

48.b
und 4-(2-Aminoethyl)-benzolsulfonsäureamid, über das Zwischenprodukt (RS)-2-(4-Benzyloxy-3-methoxy-phenyl)-2-(4-cyano-phenylamino)-N-[2-(4-sulfamoylphenyl)-ethyl]-acetamid, das (E)- und/oder (Z)-(RS)-2-(4-Benzyloxy-3-methoxyphenyl)-2-[4-(N-hydroxycarbamimidoyl)-phenylamino]-N-[2-(4-sulfamoyl-phenyl)-ethyl]-acetamid,

48.c
und 2-(2-Aminoethylpyridin), über das Zwischenprodukt (RS)-2-(4-Benzyloxy-3-methoxy-phenyl)-2-(4-cyano-phenylamino)-N-(2-pyridin-2-yl-ethyl)-acetamid das (E)-und/oder (Z)-(RS)-2-(4-Benzyloxy-3-methoxy-phenyl)-2-[4-(Nhydroxycarbamimidoyl)-phenylamino]-N-(2-pyridin-2-yl-ethyl)-acetamid,

48.d
und 4-Nitrophenethylamin über das Zwischenprodukt (RS)-2-(4-Benzyloxy-3-methoxy-phenyl)-2-(4-cyano-phenylamino)-N-[2-(4-nitro-phenyl)-ethyl]-acetamid das (E)- und/oder (Z)-(RS)-2-(4-Benzyloxy-3-methoxy-phenyl)-2-[4-(Nhydroxycarbamimidoyl)-phenylamino]-N-[2-(4-nitro-phenyl)-ethyl]-acetamid,

48.e
und (R)-Phenylglycinol und nach chromatografischer Trennung die beiden Diastereomeren (R)-2-(4-Benzyloxy-3-methoxy-phenyl)-2-(4-cyano-phenylamino)-N-[(R)-2-hydroxy-1-phenyl-ethyl]-acetamid und (S)-2-(4-Benzyloxy-3-methoxyphenyl)-2-(4-cyano-phenylamino)-N-[(R)-2-hydroxy-1-phenyl-ethyl]-acetamid;

1. aus dem ersten Diastereomeren erhielt man (E)- und/oder (Z)-(R)-2-(4-Benzyloxy-3-methoxy-phenyl)-2-[4-(N-hydroxycarbamimidoyl)-phenylamino]-N-[(R)-(2-hydroxy-1-phenyl-ethyl]-acetamid, und

2. aus dem zweiten Diastereomeren erhielt man (E)- und/oder (Z)-(S)-2-(4-Benzyloxy-3-methoxy-phenyl)-2-[4-(N-hydroxycarbamimidoyl)-phenylamino] -N-[(R)-(2-hydroxy-1-phenyl-ethyl]-acetamid,

48.f
und (S)-Phenylglycinol und nach chromatografischer Trennung die beiden Diastereomeren (R)-2-(4-Benzyloxy-3-methoxy-phenyl)-2-(4-cyano-phenylamino)-N-[(S)-2-hydroxy-1-phenyl-ethyl]-acetamid und (S)-2-(4-Benzyloxy-3-methoxyphenyl)-2-(4-cyano-phenylamino)-N- [(S)-2-hydroxy-1-phenyl-ethyl]-acetamid;

1. aus dem ersten Diastereomeren erhielt man (E)- und/oder (Z)-(R)-2-(4-Benzyloxy-3-methoxy-phenyl)-2-[4-(N-hydroxycarbamimidoyl)-phenylamino]-N-[(S)-2-hydroxy-1-phenyl-ethyl]-acetamid und

2. aus dem zweiten Diastereomeren erhielt man (E)- und/oder (Z)-(S)-2-(4-Benzyloxy-3-methoxy-phenyl)-2-[4-(N-hydroxycarbamimidoyl)-phenylamino]-N-[(S)-2-hydroxy-1-phenyl-ethyl] -acetamid,

48.g
und (R)-1-Phenylethylamin und nach chromatografischer Trennung die beiden Diastereomeren

1. (E)- und/oder (Z)-(R)-2-(4-Benzyloxy-3-methoxy-phenyl)-2-[4-(Nhydroxycarbamimidoyl)-phenylamino] -N-[(R)-1-phenyl-ethyl]-acetamid und

2. (E)- und/oder (Z)-(S)-2-(4-Benzyloxy-3-methoxy-phenyl)-2-[4-(Nhydroxycarbamimidoyl)-phenylamino] -N-[(R)-1-phenyl-ethyl]-acetamid,

48.h
und (S)-1-Phenylethylamin und nach chromatografischer Trennung die beiden Diastereomeren (R)-2-(4-Benzyloxy-3-methoxy-phenyl)-2-(4-cyano-phenylamino)-N-[(S)-1-phenyl-ethyl)-acetamid und (S)-2-(4-Benzyloxy-3-methoxy-phenyl)-2-(4-cyano-phenylamino)-N-[(S)-1-phenyl-ethyl)-acetamid;

1. aus dem ersten Diastereomeren erhielt man (E)- und/oder (Z)-(R)-2-(4-Benzyloxy-3-methoxy-phenyl)-2-[4-(N-hydroxycarbamimidoyl)-phenylamino]-N-[(S)-1-phenyl-ethyl]-acetamid,

2. aus dem zweiten Diastereomeren erhielt man (E)- und/oder (Z)-(S)-2-(4-Benzyloxy-3-methoxy-phenyl)-2-[4-(N-hydroxycarbamimidoyl)-phenylamino]-N-[(S)-1-phenyl-ethyl]-acetamid.

Beispiel 49

Analog Bsp. 45 erhielt man

49.a
aus dem Produkt von Beispiel 50.2 das (RS)-N-[2-[Benzylcarbamoyl-(4-carbamimidoyl-phenylamino)-methyl]-5-benzyloxy-4-methoxy-phenyl]-benzamidacetat (1:1.5) als farblosen Feststoff,

49.b
aus dem Produkt von Beispiel 51.a das (RS)-[2-[Benzylcarbamoyl-(4-carbamimidoyl-phenylamino)-methyl]-5-benzyloxy-4-methoxy-phenyl]-carbamidsäure-methylester-acetat (1:1.3) als farblosen Festkörper,

49.c
aus dem Produkt von Beispiel 51.b das (RS)-2-(2-Acetylamino-4-benzyloxy-5-methoxy-phenyl)-N-benzyl-2-(4-carbamimidoyl-phenylamino)-acetamid-acetat (1:1als farblosen Festkörper,

49.d
aus dem Produkt von Beispiel 51.c das (RS)-N-Benzyl-2-(4-benzyloxy-5-methoxy-2-phenylacetylamino-phenyl)-2-(4-carbamimidoyl-phenylamino)-acetamid-acetat (1:1) als leicht braunen Festkörper,

49.e
aus dem Produkt von Beispiel 52.3 das (RS)-N-Benzyl-2-(4-carbamimidoylphenylamino)-2-(4,5-dimethoxy-2-phenylacetylamino-phenyl)-acetamid-acetat (1:1) als farblosen Festkörper,

49.f
aus dem Produkt von Beispiel 53.a das (RS)-[2-[Benzylcarbamoyl-(4-carbamimidoyl-phenylamino)-methyl]-4,5-dimethoxy-phenyl]-carbamidsäuremethylester-acetat (1:1) als leicht braunen Festkörper,

49.g
aus dem Produkt von Beispiel 53.b das (RS)-N-[2-[Benzylcarbamoyl-(4-carbamimidoyl-phenylamino)-methyl]-4,5-dimethoxy-phenyl] -benzamid-acetat (1:1) als farblosen Festkörper, ISP-MS: 538.3 [M+H],

49.h
aus dem Produkt von Beispiel 54.3 das (RS)-N-[2-[Benzylcarbamoyl-(4-carbamimidoyl-phenylamino)-methyl]-4-methoxy-phenyl]-benzamid-acetat (1:1) als farbloses Lyophilisat, ISP-MS: 508.3 [M+H],

49.i
aus dem Produkt von Beispiel 55 das (RS)-2-(2-Benzenesulfonylamino-5-methoxy-phenyl)-N-benzyl-2-(4-carbamimidoyl-phenylamino)-acetamid-acetat (1:1) als farbloses Lyophilisat, ISP-MS: 544.1 [M+H].

Beispiel 50

50.1
Zu einer Lösung von 300 mg der nach Beispiel 27 erhaltenen Nitrils in 10 ml THF wurden 0.26 ml Diisopropylethylamin und 0.08 ml Benzoylchlorid gegeben. Die Reaktionslösung wurde während 3 Std. bei RT gerührt. Anschliessend wurde das THF im Vakuum abdestilliert. Der Rückstand wurde in $CH_2Cl_2$ aufgenommen und mit gesättigter NaCl-Lösung gewaschen. Die organische Phase wurde über $Na_2SO_4$ getrocknet, filtriert und im Vakuum eingedampft. Der Rückstand wurde aus Cyclohexan/Toluol/Aceton kristallisiert. Man erhielt 288 mg (79%) (RS) -N-{2-[Benzylcarbamoyl-(4-cyano-phenylamino)-methyl]-5-benzyloxy-4-methoxy-phenyl}-benzamid als leicht braunen Festkörper.

50.2
Analog Bsp. 36.3 erhielt man aus 280 mg des oben hergestellten Materials 254 mg (86%) des (RS)-N-(2-{Benzylcarbamoyl-[4-(N-hydroxycarbamimidoyl)-phenylamino]-methyl}-5-benzyloxy-4-methoxy-phenyl)-benzamid als leicht braunen Festkörper.

Beispiel 51

Analog Bsp. 50.1 erhält man aus dem Produkt von Beispiel 27 und

51.a
Methylchloroformat das (RS)-(2-{Benzylcarbamoyl-[4-(N-hydroxycarbamimidoyl)-phenylamino]-methyl}-5-benzyloxy-4-methoxy-phenyl)-carbamidsäuremethylester,

51.b
Acetylchlorid das (E)- und/oder (Z)-(RS)-2-(2-Acetylamino-4-benzyloxy-5-methoxyphenyl)-N-benzyl-2-[4-(N-hydroxycarbamimidoyl)-phenylamino]-acetamid,

51.c
Phenylacetylchlorid das (E)- und/oder(Z)-(RS)-N-Benzyl-2-(4-benzyloxy-5-methoxy-2-phenylacetylamino-phenyl)-2-[4-(N-hydroxycarbamimidoyl)-phenylamino]-acetamid.

Beispiel 52

52.1
Analog Bsp. 16 erhielt man ausgehend von 6-Nitroveratrumaldehyd, 4-Aminobenzonitril und Benzylisonitril das (RS)-N-Benzyl-2-(4-cyanophenylamino)-2-(4,5-dimethoxy-2-nitro-phenyl)-acetamid in 10% Ausbeute als gelbe Kristalle.

52.2
Analog zu Bsp. 27 erhielt man aus der in 52.1 dargestellten Nitroverbindung das (RS)-2-(2-Amino-4,5-dimethoxy-phenyl)-N-benzyl-2-(4-cyano-phenylamino)-acetamid in 25% Ausbeute. Leicht graue Kristalle.

52.3
Analog Bsp. 50 erhielt man aus der oben dargestellten Anilinverbindung und Phenylacetylchlorid das (E)- und/oder (Z)-(RS)-N-Benzyl-2-(4,5-dimethoxy-2-phenylacetylamino-phenyl)-2-[4-(N-hydroxycarbamimidoyl)-phenyl-amino]-acetamid.

Beispiel 53

Analog Bsp. 50 erhielt man aus der in 52.2 dargestellten Anilinverbindung und:

53.a
Methylchloroformat das (E)- und/oder (Z)-(RS)-[2-[Benzylcarbamoyl-[4-(Nhydroxycarbamimidoyl)-phenylamino]-methyl]-4,5-dimethoxy-phenyl]-carbamidsäure-methylester,

53.b
Benzoylchlorid das (E)- und/oder (Z)-(RS)-N-[2-[Benzylcarbamoyl-[4-(N-hydroxycarbaimidoyl)-phenylamino]-methyl]-4,5-dimethoxy-phenyl)-benzamid.

Beispiel 54

54.1
Analog Bsp. 16 erhält man ausgehend von 6-Nitroveratrumaldehyd, 4-Aminobenzonitril und Benzylisonitril das (RS)-N-Benzyl-2-(4-cyanophenylamino)-2-(5-methoxy-2-nitro-phenyl)-acetamid in 24 % Ausbeute. Gelbe Kristalle.

54.2
Analog Bsp. 27 erhält man aus der oben dargestellten Nitroverbindung das (RS)-N-Benzyl-2-(4-cyano-phenylamino)-2-(2-amino-5-methoxy-phenyl)-acetamid in 79 % Ausbeute. ISP-MS: 387.1 [M+H], 409.2 [M+Na].

54.3
Analog Bsp. 50 erhält man aus dem in Bsp 54.2 erhaltenen Nitril und Benzoylchlorid das (E)- und/oder (Z)-(RS) -N-[2-[Benzylcarbamoyl-[4-(Nhydroxycarbamimidoyl)-phenylamino]-methyl]-4-methoxy-phenyl]-benzamid.

Beispiel 55

Analog zu Bsp. 50 erhielt man aus in Bsp 54.2 erhaltenen Nitril und Phenylsulfonylchlorid das (E)- und/oder (Z)-(RS)-2-(2-Benzenesulfonylamino-5-methoxy-phenyl)-N-benzyl-2- [4-(N-hydroxycarbamimidoyl)-phenylamino]-acetamid.

Beispiel 56

56.1

Analog Bsp. 36.1 erhielt man aus dem nach Bsp. 27 erhaltenen Materials und Methoxyessigsäure das (RS)-N-Benzyl-2-[4-benzyloxy-5-methoxy-2-(2-methoxyacetylamino)-phenyl]-2-(4-cyano-phenylamino)-acetamid in 66% Ausbeute. Festkörper.

56.2
Analog Bsp. 36.3 wurde das in Bsp. 56.1 hergestellte Nitril in 81% Ausbeute zu (RS)-N-Benzyl-2-[4-benzyloxy-5-methoxy-2-(2-methoxy-acetylamino)-phenyl]-2-[4-(N-hydroxycarbamimidoyl)-phenylamino]-acetamid umgesetzt. Leicht brauner Festkörper.

56.3
Analog Bsp. 45 wurde das in Bsp 56.2 erhaltene Amidoxim zu (RS)-N-Benzyl-2-[4-benzyloxy-5-methoxy-2-(2-methoxy-acetylamino)-phenyl]-2-(4-carbamimidoylphenylamino)-acetamid-hydrochlorid (1:2) reduziert.

Beispiel 57

57.1
Analog Bsp. 36.1 erhielt man aus dem Produkt von Beispiel 27 und N-Boc-Glycin den (RS)-({2-[Benzylcarbamoyl-(4-cyano-phenylamino)-methyl]-5-benzyloxy-4-methoxy-phenylcarbamoyl}-methyl)-carbamidsäure-tert-butyl ester in 65% Ausbeute.

57.2
Das oben erhaltene Produkt wurde analog Bsp. 36.3 in 65% Ausbeute zu (RS)-[(2-{Benzylcarbamoyl-[4-(N-hydroxycarbamimidoyl)-phenylamino]-methyl}-5-benzyloxy-4-methoxy-phenylcarbamoyl)-methyl]-carbamidsäure-tert-butyl ester umgesetzt. Leicht brauner Festkörper.

57.3
Eine Lösung von 170 mg des nach Bsp 57.2 erhaltenen Amidoxims in 10 ml EtOH wurde mit einer Spatelspitze Raney-Nickel und ein paar Tropfen Essigsäure versetzt. Dann wurde während 2 Std. hydriert. Anschliessend wurde vom Katalysator abfiltriert. Das Filtrat wurde eingeengt. Der Rückstand wurde chromatographisch an Kieselgel gereinigt (EtOAc/Aceton/ $H_2O$/HOAc 6:2:1:1). Die Produktfraktionen wurden eingeengt. Der Rückstand wurde in $CH_2Cl_2$ gelöst, mit 5 ml Trifluoressigsäure versetzt und während 3 Std. bei 0°C gerührt. Dann wurde im Vakuum eingedampft. Der Rückstand wurde aus $Et_2O$ kristallisiert. Man erhielt 107 mg (76%) (RS)-2-[2-(2-Amino-acetylamino)-4-benzyloxy-5-methoxy-phenyl]-N-benzyl-2-(4-carbamimidoyl-phenylamino)-acetamidtrifluoroacetat (1:2) als leicht braunen Festkörper.

Beispiel 58

In Analogie zu Bsp. 45 erhielt man:

58.a
aus dem in Beispiel 59.5 erhaltenen Produkt das (RS)-3-[2-[Benzylcarbamoyl-(4-carbamimidoyl-phenylamino)-methyl]-4,5-dimethoxy-phenoxymethyl]-benzoesäure-acetat (1:1), ISP-MS: 569.3 [M+H],

58.b
aus dem in Beispiel 60.a erhaltenen Produkt das (RS)-[2-[Benzylcarbamoyl-(4-carbamimidoyl-phenylamino)-methyl]-4,5-dimethoxy-phenoxy]-essigsäure-acetat (1:1),

58.c
aus dem in Beispiel 60.b erhaltenen Produkt das (RS)-4-[2-[Benzylcarbamoyl-(4-carbamimidoyl-phenylamino)-methyl]-4,5-dimethoxy-phenoxymethyl]-benzoesäure-acetat (1:1), ISP-MS: 569.3 [M+H],

58.d
aus dem in Beispiel 60.c erhaltenen Produkt die (RS)-4-[2-[Benzylcarbamoyl-(4-carbamimidoyl-phenylamino)-methyl]-4,5-dimethoxy-phenoxyl-butersäure,

58.e

aus dem in Beispiel 61 erhaltenen Produkt die (RS)-[2-[Benzylcarbamoyl-(4-carbamimidoyl-phenylamino)-methyl]-4-methoxy-phenoxy]-essigsäure, ISP-MS: M+H [463.2],

58.f
aus dem in Beispiel 62 erhaltenen Produkt das (RS)-[2-[Benzylcarbamoyl-(4-carbamimidoyl-phenylamino)-methyl]-4,6-dimethyl-phenoxy]-essigsäure-acetat (1:0.5), ISP-MS: 461.3 [M+H], 483.4 [M+Na].

Beispiel 59

59.1
Zu einer Lösung von 2.78 mg 2-Benzyloxy-4,5-dimethoxybenzaldehyd in 36 ml THF und 4 ml $H_2O$ wurden 1.18 g 4-Aminobenzonitril und 1.22 ml Benzylisonitril gegeben. Man rührte während 5 min bei RT und versetzte dann mit 2.85 g p-Toluolsulfonsäure. Die Reaktionslösung wurde während 24 Std. bei RT gerührt. Dann wurde im Vakuum eingedampft. Der Rückstand wurde in $CH_2Cl_2$ aufgenommen und dann mit gesättigter $NaHCO_3$-Lösung und gesättigter NaCl-Lösung gewaschen. Die organische Phase wurde über $Na_2SO_4$ getrocknet, abfiltriert und im Vakuum eingedampft. Der Rückstand wurde chromatographisch an Kieselgel gereinigt (Toluol/Aceton 9:1). Man erhielt 2.13 g (42%) (RS)-N-Benzyl-2-(2-benzyloxy-4,5-dimethoxy-phenyl)-2-(4-cyanophenylamino)-acetamid als hellgelben Feststoff.

59.2
Eine Lösung von 1.4 g des in 59.1 erhaltenen Materials in 140 ml EtOH wurde mit 420 mg Pd/C 10% versetzt und während 5 Std. hydriert, wobei sich ein farbloser Niederschlag bildete. Dieser wurde durch Zugabe von 75 ml Dioxan wieder in Lösung gebracht. Anschliessend wurde vom Katalysator abfiltriert. Das Filtrat wurde im Vakuum eingedampft. Der Rückstand wurde aus EtOH kristallisiert. Man erhielt 907 mg (79%) (RS)-N-Benzyl-2-(4-cyano-phenylamino)-2-(2-hydroxy-4,5-dimethoxy-phenyl)-acetamid als farblose Kristalle.

59.3
Eine Lösung von 418 mg des in 59.2 erhaltenen Materials in 30 ml Aceton wurde mit 415 mg $K_2CO_3$ und 252 mg Methyl-3-bromomethylbenzoat versetzt. Das Reaktionsgemisch wurde während 3 Std. unter Rückfluss erhitzt und anschliessend filtriert. Das Filtrat wurde eingeengt. Der Rückstand wurde in $CH_2Cl_2$ aufgenommen und mit $H_2O$ gewaschen. Die organische Phase wurde über $Na_2SO_4$ getrocknet, filtriert und im Vakuum eingedampft. Der Rückstand wurde chromatographisch an Kieselgel gereinigt (EtOAc/Hexan 2:3). Man erhielt 365 mg (65%) (RS)-3-[2-[Benzylcarbamoyl-(4-cyano-phenylamino)-methyl]-4,5-dimethoxy-phenoxymethyl]-benzoesäure-methylester als farblosen Festkörper.

59.4
In Analogie zu Bsp. 36.2 erhielt man aus dem in 59.3 erhaltenen Material die (RS)-3-[2-[Benzylcarbamoyl-(4-cyano-phenylamino)-methyl]-4,5-dimethoxyphenoxymethyl]-benzoesäure in quantitativer Ausbeute. Farbloser Feststoff.

59.5
In Analogie zu Bsp. 36.3 erhielt man aus dem unter 59.4 erhaltenen Material die (E)- und/oder (Z)-(RS)-3-[2-[Benzylcarbamoyl-[4-(N-hydroxycarbamimidoyl)-phenylamino]-methyl]-4,5-dimethoxy-phenoxymethyl]-benzoesäure in 70% Ausbeute. Farbloser Feststoff.

Beispiel 60

Analog Bsp. 59.3 - 59.5 erhielt man aus dem nach Bsp. 59.2 erhaltenen Material und

60.a
Bromessigsäure-ethylester über die Nitrile (RS)-[2-[Benzylcarbamoyl-(4-cyanophenylamino)-methyl]-4,5-dimethoxy-phenoxy]-essigsäure-ethylester und (RS)-[2-[Benzylcarbamoyl-(4-cyano-phenylamino)-methyl]-4,5-dimethoxy-phenoxy]-essigsäure die (E)- und/oder (Z)-(RS)-[2-[Benzylcarbamoyl-[4-(Nhydroxycarbamimidoyl)-phenylamino]-methyl]-4,5-dimethoxy-phenoxy]-essigsäure,

60.b
Methyl-4-bromomethylbenzoat über die Nitrile (RS)4-[2-[Benzylcarbamoyl-(4-cyano-phenylamino)-methyl]-4,5-dimethoxy-phenoxymethyl]-benzoesäuremethylester und (RS)-4-[2-[Benzylcarbamoyl-(4-cyano-phenylamino)-methyl]-4,5-dimethoxy-phenoxymethyl]-benzoesäure die (E)- und/oder (Z)-(RS)-4-[2-[Benzylcarbamoyl-[4-(N-hydroxycarbamimidoyl)-phenylamino]-methyl]-4,5-dimethoxy-phenoxymethyl]-benzoesäure,

60.c
4-Brombuttersäure-ethylester über die Nitrile (RS)-4-[2-[Benzylcarbamoyl-(4-cyano-phenylamino)-methyl]-4,5-dimethoxy-phenoxy]-buttersäure-ethylester und (RS)-4-[2-[Benzylcarbamoyl-(4-cyano-phenylamino)-methyl]-4,5-dimethoxyphenoxy]-buttersäure die (E)- und/oder (Z)-(RS)-4-[2-[Benzylcarbamoyl-[4-(Nhydroxycarbamimidoyl)-phenylamino]-methyl]-4,5-dimethoxy-phenoxy]-buttersäure.

Beispiel 61

Analog Bsp. 60 erhielt man aus (RS)-N-Benzyl-2-(4-cyano-phenylamino)-2-(2-hydroxy-5-methoxyphenyl)-acetamid und Bromessigsäure-ethylester über die Nitrile (RS)-[2- [Benzylcarbamoyl-(4-cyano-phenylamino)-methyl]-4-methoxyphenoxy]-essigsäure-ethylester und (RS)-[2-[Benzylcarbamoyl-(4-cyanophenylamino)-methyl]-4-methoxy-phenoxy]-essigsäure die (E)- und/oder (Z)-(RS)-[2-[Benzylcarbamoyl-[4-(N-hydroxycarbamimidoyl)-phenylamino]-methyl]-4-methoxy-phenoxy]-essigsäure.
Das Ausgangsmaterial wurde wie folgt hergestellt:

61.1
Eine Lösung aus 30 g 2-Hydroxy-5-methoxybenzaldehyd in 200 ml Aceton wurde mit 51.2 ml Allylbromid un 81.75 g $K_2CO_3$ versetzt und während 2 Std. unter Rückfluss erhitzt. Anschliessend filtrierte man das Reaktionsgemisch. Das Filtrat wurde im Vakuum eingeengt. Der Rückstand wurde in $CH_2Cl_2$ aufgenommen und mit $H_2O$ gewaschen. Die organischen Phasen wurden über $Na_2SO_4$ getrocknet, filtriert und im Vakuum eingedampft. Der Rückstand wurde im HV destilliert. Man erhielt 24.5 g (65%) 2-Allyloxy-5-methoxy-benzaldehyd als leicht grünes Oel.

61.2
In Analogie zu Bsp. 59.1 erhielt man aus dem oben erhaltenen Aldehyd, 4-Aminobenzonitril und Benzylisonitril das (RS)-2-(2-Allyloxy-5-methoxy-phenyl)-N-benzyl-2-(4-cyano-phenylamino)-acetamid in 35% Ausbeute. ISP-MS: 428.3 [M+H], 450.1 [M+Na].

61.3
Eine Lösung von 4.6 g des oben erhaltenen Nitrils in 140 ml THF wurde mit 249 mg Tetrakis(triphenylphosphin)-palladium(0) versetzt. Man rührte während 10 min bei RT und gab dann portionenweise 626 mg $NaBH_4$ zu. Es wurde während 2 Std. bei RT gerührt und anschliessend im Vakuum eingedampft. Der Rückstand wurde in EtOAc aufgenommen und mit $H_2O$ gewaschen. Die organische Phase wurde über $Na_2SO_4$ getrocknet, filtriert und im Vakuum eingedampft. Der Rückstand wurde in $CH_2Cl_2$/EtOAc 9:1 gelöst und mit Aktivkohle entfärbt. Anschliessend wurde aus $Et_2O/CH_2Cl_2$ kristallisiert. Man erhielt 3.22 g (77%) (RS)-N-Benzyl-2-(4-cyano-phenylamino)-2-(2-hydroxy-5-methoxy-phenyl)-acetamid als leicht braunen, kristallinen Festkörper. ISN-MS: 386.1 [M-H].

Beispiel 62

In Analogie zu Bsp. 60 erhielt man aus (RS)-N-Benzyl-2-(4-cyano-phenylamino)-2-(2-hydroxy-3,5-dimethyl-phenyl)-acetamid und Bromessigsäure-ethylester den (RS)-[2-[Benzylcarbamoyl-(4-cyano-phenylamino)-methyl]-4,6-dimethyl-phenoxy]-essigsäure-ethylester und daraus die (E)- und/oder (Z)-(RS)-[2-[Benzylcarbamoyl-[4-(N-hydroxycarbamimidoyl)-phenylamino]-methyl]-4,6-dimethyl-phenoxy]-essigsäure.
Das Ausgangsmaterial wurde in Analogie zu Bsp. 16 aus 3,5-Dimethyl-2-hydroxybenzaldehyd (G. Casiraghi et al., J. Chem. Soc. (1980), 1862), 4-Aminobenzonitril und Benzylisonitril hergestellt.

Beispiel 63

63.1
Eine Lösung von 1.04 g des in Beispiel 59.2 erhaltenen Nitrils, 0.35 ml (S)-Milchsäure-methylester und 785 mg Triphenylphosphin in 75 ml THF wurden mit 0.475 ml Azo-dicarbonsäure-diethylester versetzt und während 4 Std. bei RT gerührt. Anschliessend wurde das THF im Vakuum abdestilliert. Der Rückstand wurde in EtOAc aufgenommen und mit gesättigter NaCl-Lösung gewaschen. Die organische Phase wurde über $MgSO_4$ getrocknet, filtriert

und im Vakuum eingedampft. Der Rückstand wurde chromatographisch an Kieselgel gereinigt ($CH_2Cl_2$/EtOAc 9: 1). Man erhielt 1.145 g (88%) eines 1:1 Gemisches von (R)-2-[2-[(R)- und -[(S)-Benzylcarbamoyl-(4-cyano-phenylamino)-methyl] -4,5-dimethoxyphenoxy]-propionsäure-ethylester als gelben Schaum. ISP-MS: 518.2 [M+H], 540.3 [M+Na].

63.2
In Analogie zu Bsp.36.2 erhielt man aus dem oben hergestellten Ester nach chromatographischer Trennung an Kieselgel die beiden Diastereomeren (R)-2-[2-[(R)-Benzylcarbamoyl-(4-cyano-phenylamino)-methyl]-4,5-dimethoxy-phenoxy]-propionsäure und (R)-2-[2-[(S)-Benzylcarbamoyl-(4-cyano-phenylamino)-methyl]-4,5-dimethoxy-phenoxy]-propionsäure in 49% Ausbeute, beide als hellgelbe Harze. ISP-MS: 490.3 [M+H], 512.3 [M+Na].

63.3
Analog Bsp. 36.3 erhielt man aus dem oben erhaltenen ersten Diastereomeren

a) die (E)- und/oder (Z)-(R)-2-[2-[(R)-Benzylcarbamoyl-[4-(N-hydroxycarbamimidoyl)-phenylamino]-methyl]-4,5-dimethoxy-phenoxy]-propionsäure und aus dem oben erhaltenen zweiten Diastereomeren

b) die (E)- und/oder (Z)-(R)-2-[2-[(S)-Benzylcarbamoyl-[4-(N-hydroxycarbamimidoyl)-phenylamino]-methyl]-4,5-dimethoxy-phenoxy]-propionsäure, beide als farblose Pulver. ISP-MS: 523.2 [M+H], 545.2 [M+Na], 567.2 [M+2Na].

63.4
In Analogie zu Bsp. 33 erhielt man aus den jeweiligen diastereomeren Amidoximen von Bsp. 63.3 die Amidine

a) (R)-2-[2-[(R)-Benzylcarbamoyl-(4-carbamimidoyl-phenylamino)-methyl]-4,5-dimethoxy-phenoxy]-propionsäure-acetat (1:1) und

b) (R)-2-[2-[(S)-Benzylcarbamoyl-(4-carbamimidoyl-phenylamino)-methyl]-4,5-dimethoxy-phenoxy]-propionsäure-acetat (1:1), ISP-MS: 507.4 [M+H].

Beispiel 64

64.1
In Analogie zu Bsp. 63.1 - 63.3 erhielt man aus dem in Beispiel 59.2 erhaltenen Material und (R)-Milchsäure-ethylester über das Epimerengemisch des entsprechenden Nitril-Esters die beiden diastereomeren Nitril-säuren und daraus die jeweiligen Epimeren

a) (E)- und/oder (Z)-(S)-2-[2-[(R)-Benzylcarbamoyl-[4-(N-hydroxycarbamimidoyl)-phenylamino]-methyl]-4,5-dimethoxy-phenoxy]-propionsäure und

b) (E)- und/oder (Z)-(S)-2-[2-[(S)-Benzylcarbamoyl-[4-(N-hydroxycarbamimidoyl)-phenylamino]-methyl]-4,5-dimethoxy-phenoxy]-propionsäure, beide als farblose Lyophilisate.

64.2
Analog Bsp. 33 erhielt man aus oben erhaltenen Amidoximen die jeweiligen Amidine

a) (S)-2-[2-[(R)-Benzylcarbamoyl-(4-carbamimidoyl-phenylamino)-methyl]-4,5-dimethoxy-phenoxy]-propionsäure und

b) (S)-2-[2-[(S)-Benzylcarbamoyl-(4-carbamimidoyl-phenylamino)-methyl]-4,5-dimethoxy-phenoxy]-propionsäure, beide als farblose Lyophilisate. ISP-MS: 507.4 [M+H], 529.2 [M+Na].

Beispiel 65

Analog Bsp. 45 erhielt man

65.a
aus dem Produkt von Bsp. 66.4) die (RS)-3-{4-[Benzylcarbamoyl-(4-carbamimidoyl-phenylamino)-methyl]-2-methoxy-phenoxy}-benzoesäure in 44% Ausbeute. Farbloser Festkörper, ISP-MS: 525.2 [M+H],

65.b
aus dem Produkt von Bsp. 67.a die (RS)-2-{4-[Benzylcarbamoyl-(4-carbamimidoyl-phenylamino)-methyl]-2-methoxy-phenoxy}-benzoesäure, ISP-MS: 525.2 [M+H],

65.c
aus dem Produkt von Bsp. 67.b die (RS)-4-{4-[Benzylcarbamoyl-(4-carbamimidoyl-phenylamino)-methyl]-2-methoxy-phenoxy}-benzoesäure. Leicht brauner Festkörper. ISP-MS: 525.2 [M+H].

Beispiel 66

66.1
Eine Lösung von 3 g Vanillin, 11 g Ethyl-3-iodbenzoat und 2.9 g $Cu_2O$ in 5 ml Dimethylacetamid wurden während 24 Std. auf 165°C erhitzt. Man liess auf RT abkühlen und filtrierte. Das Filtrat wurde im HV eingedampft. Der Rückstand wurde chromatographisch an Kieselgel gereinigt (EtOAc/Hexan 3:7). Man erhielt 1.93 g (32%) 3-(4-Formyl-2-methoxy-phenoxy)-benzoesäure-ethylester als leicht braunes Oel.

66.2
Analog Bsp. 59.1 erhielt man aus oben erhaltenem Aldehyd den (RS)-3-{4-[Benzylcarbamoyl-(4-cyano-phenylamino)-methyl]-2-methoxy-phenoxy}benzoesäure-ethylester in 58% Ausbeute. Farbloser Festkörper.

66.3
Analog Bsp. 36.2 erhielt man aus dem oben erhaltenen Nitril-Ester die (RS)-3-{4-[Benzylcarbamoyl-(4-cyano-phenylamino)-methyl]-2-methoxy-phenoxy}benzoesäure in 51% Ausbeute. Farbloser Festkörper. ISN-MS: 506.2 [M-H].

66.4
Analog Bsp. 36.3 erhielt man aus der oben erhaltenen Nitril-Säure die (RS)-3-(4-{Benzylcarbamoyl-[4-(N-hydroxycarbamimidoyl)-phenylamino]-methyl}-2-methoxy-phenoxy)-benzoesäure in 94% Ausbeute. Farbloser Festkörper. ISP-MS: 541.2 [M+H], 563.3 [M+Na].

Beispiel 67

Analog Bsp. 66 erhielt man

67.a
unter Verwendung von Methyl-2-iodbenzoat anstelle von Ethyl-3-iodbenzoat über die entsprechenden Zwischenprodukte 4-(2-Carbomethoxy)phenoxy-3-methoxybenzaldehyd, (RS)-2-[4-[Benzylcarbamoyl-(4-cyano-phenylamino)-methyl]-2-methoxy-phenoxy]-benzoesäure-methylester und (RS)-2-{4-[Benzylcarbamoyl-(4-cyano-phenylamino)-methyl]-2-methoxy-phenoxy}-benzoesäure die (RS)-2-(4-{Benzylcarbamoyl-[4-(N-hydroxycarbamimidoyl)-phenylamino]-methyl}-2-methoxy-phenoxy)-benzooesäure,

67.b
unter Verwendung von Methyl-4-iodbenzoat anstelle von Ethyl-3-iodbenzoat über die entsprechenden Zwischenprodukte 4-(4-Formyl-2-methoxy-phenoxy)-benzoesäure-methylester, (RS)-4-{4-[Benzylcarbamoyl-(4-cyanophenylamino)-methyl]-2-methoxy-phenoxy}-benzooesäure-methylester und (RS)-4-{4-[Benzylcarbamoyl-(4-cyano-phenylamino)-methyl]-2-methoxy-phenoxy}-benzoesäure die (RS)-4-(4-{Benzylcarbamoyl-[4-(N-hydroxycarbamimidoyl)-phenylamino]-methyl}-2-methoxy-phenoxy)-benzoesäure.

Beispiel 68

Analog Bsp. 66.2 -66.4 erhielt man aus 5-Formyl-2,3-dimethoxy-benzoesäuremethylester (F.Y. Wu, D.L. Brink, J. Agric. Food Chem. (1977), 25, 692) über die Zwischenstufen (RS)-5-[Benzylcarbamoyl-(4-cyano-phenylamino)-methyl]-2,3-dimethoxy-benzoesäure-methylester,

a) den (RS)-5-{Benzylcarbamoyl-[4-(N-hydroxycarbamimidoyl)-phenylamino]-methyl}-2,3-dimethoxy-benzoesäure-methylester und

b) den (RS)-5-[Benzylcarbamoyl-(4-carbamimidoyl-phenylamino)-methyl]-2,3-dimethoxy-benzoesäure-methylester und

c) die (RS)-5-[Benzylcarbamoyl-(4-carbamimidoyl-phenylamino)-methyl]-2,3-dimethoxy-benzoesäure. Leicht brauner Festkörper.

Beispiel 69

69.1
Analog Bsp. 59.1 erhielt man aus 6-Bromveratrumaldehyd, 4-Aminobenzonitril und Benzylisonitril das (RS)-N-Benzyl-2-(2-bromo-4,5-dimethoxy-phenyl)-2-(4-cyano-phenylamino)-acetamid in 47% Ausbeute. Farbloser Festkörper.

69.2
Eine Lösung von 2.4 g des in Bsp. 69.1 erhaltenen Nitrils in 10 ml Dimethylacetamid wurde mit 0.41 ml Acrylsäure, 1.51 ml Triethylamin, 22 mg Palladiumacetat und 122 mg Tri-o-tolylphosphin versetzt und während 2 Std. auf 120 °C erhitzt. Das Reaktionsgemisch wurde mit Wasser verdünnt, mit 1N HCl auf pH 1 gestellt und dann mit EtOAc extrahiert. Die organische Phase wurde im Vakuum eingedampft. Der Rückstand wurde in MeOH gelöst und filtriert. Das Filtrat wurde im Vakuum eingedampft. Der Rückstand wurde in $Et_2O$ verrührt. Der Feststoff wurde abfiltriert und im HV getrocknet. Man erhielt 1.71 g (73%) (E)-(RS)-3-[2-[Benzylcarbamoyl-(4-cyano-phenylamino)-methyl]-4,5-dimethoxyphenyl]-acrylsäure als beigen Festkörper.

69.3
Eine Lösung von 236 mg des oben erhaltenen Nitrils in 2 ml EtOH und 3 ml THF wurde mit 5 ml 1N HCl und 106 mg Pd/C 10% versetzt und während 2 Std. hydriert. Das Reaktionsgemisch wurde filtriert. Das Filtrat wurde mit EtOAc extrahiert. Die organische Phase wurde über $MgSO_4$ getrocknet und im Vakuum eingedampft. Der Rückstand wurde chromatographisch an Kieselgel gereinigt. Man erhielt 44 mg (18%) (RS)-3-[2-[Benzylcarbamoyl-(4-cyano-phenylamino)-methyl]-4,5-dimethoxy-phenyl]-propionsäure als leicht gelben Festkörper.

69.4
Analog Bsp. 36.3 erhielt man aus dem in Bsp. 69.3 erhaltenen Nitrils die (E)-und/oder (Z)-(RS)-3-[2-[Benzylcarbamoyl-[4-(N-hydroxycarbamimidoyl)-phenylamino]-methyl]-4,5-dimethoxy-phenyl]-propionsäure in 26 % Ausbeute. Beiges Lyophilisat.

69.5
Reduktion des oben erhaltenen Amidoxims analog Bsp. 45 ergab die (RS)-3-[2-[Benzylcarbamoyl-(4-carbamimidoyl-phenylamino)-methyl]-4,5-dimethoxyphenyl]-propionsäure in 62% Ausbeute. Weisses Lyophilisat.

Beispiel 70

70.1
Analog Bsp. 16 erhielt man aus 3-Nitrobenzaldehyd, 4-Aminobenzonitril und Benzylisonitril das (RS)-N-Benzyl-2-(4-cyano-phenylamino)-2-(3-nitro-phenyl)-acetamid in 11% Ausbeute. Hellgelber, kristalliner Festkörper. ISP-MS: 387.2 [M+H], 409.3 [M+Na].

70.2
Eine Lösung von 1.4 g der oben erhaltenen Nitroverbindung in 80 ml THF wurde mit 140 mg Pd/C 10% versetzt und während 6 Std. hydriert. Der Katalysator wurde abfiltriert, das Filtrat wurde im Vakuum eingedampft. Der Rückstand wurde chromatographisch an Kieselgel gereinigt ($CH_2Cl_2$/MeOH 19:1). Man erhielt 1.15 g (89%) (RS)-2-(3-Amino-phenyl)-N-benzyl-2-(4-cyano-phenylamino)-acetamid als gelben Schaum. ISP-MS: 357.2 [M+H], 379.3 [M+Na].

70.3

Analog Bsp. 50.1 wurde das oben erhaltene Amin mit Acetylchlorid in 99% Ausbeute zu farblosem (RS)-2-(3-Acetylamino-phenyl)-N-benzyl-2-(4-cyanophenylamino)-acetamid umgesetzt. ISP-MS: 399.4 [M+H], 421.3 [M+Na], 437.3 [M+K].

70.4

Analog Bsp. 36.3 erhielt man ausgehend vom obigen Nitril das (E)- und/oder (Z)-(RS)-2-(3-Acetylamino-phenyl)-N-benzyl-2-[4-(N-hydroxycarbamimidoyl)-phenylamino]-acetamid in quantitativer Ausbeute. Farbloser Festkörper.

70.5

Analog Bsp. 45 wurde das unter Bsp 70.4 erhaltene Amidoxim in 65% Ausbeute zu (RS)-2-(3-Acetylamino-phenyl)-N-benzyl-2-(4-carbamimidoyl-phenylamino)-acetamid-acetat (1:1)) reduziert. Farbloses Pulver.

Beispiel 71

In eine Lösung von 386 mg des Nitrits aus Bsp 70.1 in 20 ml MeOH und 10 ml $CHCl_3$ wurde während 2 Std. bei 5-10°C trockenes HCl-Gas eingeleitet. Anschliessend wurde die Reaktionslösung im Vakuum eingedampft. Der Rückstand wurde in 30 ml mit $NH_3$ gesättigtem MeOH aufgenommen. Die Lösung wurde über Nacht bei RT stehengelassen, dann während 4 Std. auf 40°C erhitzt und anschliessend im Vakuum eingedampft. Der Rückstand wurde chromatographisch an Kieselgel gereinigt. Man erhielt 160 mg (35%) (RS)-N-Benzyl-2-(4-carbamimidoyl-phenyiamino)-2-(3-nitro-phenyl)-acetamid-acetat (1:1) als gelben Festkörper. ISP-MS: 404.4 [M+H].

Beispiel 72

Eine Lösung von 201 mg 2,6-Dimethoxy-isonicotin-aldehyd (I. Kompis et al., Eur. J. Med. Chem. - Chim. Ther. (1977), 12, 531) und 575 mg 4-Aminobenzamidin-ditoluolsulfonat in 4.8ml THF/$H_2O$ 9:1 wurde mit 0.15 ml Benzylisonitril) versetzt. Das Reaktionsgemisch wurde während 6 Std. bei RT gerührt und anschliessend im Vakuum eingedampft. Der Rückstand wurde in Diethylether suspendiert. Der Feststoff wurde abfiltriert und aus EtOH/Wasser umkristallisiert. Man erhielt 2.02 g (28%) (RS)-N-Benzyl-2-(4-carbamimidoylphenylamino)-2-(2,6-dimethoxy-pyridin-4-yl)-acetamid als farblosen kristallinen Feststoff.

Beispiel 73

Analog zu Bsp. 72 erhielt man

73.a

aus 4,6-Dimethoxy-picolinaldehyd (I. Kompis et al., Eur. J. Med. Chem. - Chim. Ther. (1977), 12, 531), 4-Amidinobenzamidin-ditoluolsulfonat und Benzylisonitril das (RS)-N-Benzyl-2-(4-carbamimidoyl-phenylamino)-2-(4,6-dimethoxy-pyridin-2-yl)-acetamid in 41% Ausbeute, farbloser kristalliner Festkörper,

73.b

aus 4-Benzyloxy-3,5-dimethoxybenzaldehyd, 4-Amidinobenzamidin-ditoluolsulfonat und Benzylisonitril das (RS)-N-Benzyl-2-(4-benzyloxy-3,5-dimethyl-phenyl)-2-(4-carbamimidoyl-phenylamino)-acetamid in 23% Ausbeute. Farbloser kristalliner Festkörper.

Beispiel 74

Analog Bsp. 1 erhielt man

74.a

aus 3-Benzyloxy-5-propoxy-benzaldehyd, 4-Aminobenzamidin-dihydrochlorid und Benzylisonitril das (RS)-N-Benzyl-2-(3-benzyloxy-5-propoxy-phenyl)-2-(4-carbamimidoyl-phenylamino)-acetamid-hydrochlorid.

74.b

aus 3,5-Bis(benzyloxy)-benzaldehyd, 4-Aminobenzamidin-dihydrochlorid und Benzylisonitril das (RS)-N-Benzyl-2-(3,5-bis-benzyloxy-phenyl)-2-(4-carbamimidoyl-phenylamino)-acetamid-hydrochlorid.

Beispiel 75

75.a
Analog Bsp. 59.1 erhielt man aus 2,6-Diethoxy-pyridine-4-carbaldehyd (I. Kompis et al., Eur. J. Med. Chem. - Chim. Ther. (1977), 12, 531), 4-Aminobenzonitril und Benzylisonitril das (RS)-N-Benzyl-2-(4-cyano-phenylamino)-2-(2,6-diethoxypyridin-4-yl)-acetamid.

75.b
Analog Bsp. 71 erhielt man aus dem Produkt von Bsp 75.a das (RS)-N-Benzyl-2-(4-carbamimidoyl-phenylamino)-2-(2,6-diethoxy-pyridin-4-yl)-acetamidhydrochlorid.

Beispiel 76

76.1
Eine Lösung von 5 g 2-Hydroxy-4-nitro-benzonitril (W.Borsche, Ann. Chem. (1912) 390, 1) in 80.5 ml Isopropanol wurde mit 1 g Pd/C 10 % versetzt und während 1.5 Std. hydriert. Anschliessend wurde vom Katalysator abfiltriert und das Filtrat eingedampft. Man erhielt 3.3 g (80%) 4-Amino-2-hydroxy-benzonitril.

76.2
Analog Bsp. 59.1 erhielt man aus dem Nitril von Bsp. 76.1, 4-Benzyloxy-3-ethoxybenzaldehyd und Benzylisonitril das (RS)-N-Benzyl-2-(4-benzyloxy-3-ethoxy-phenyl)-2-(4-cyano-3-hydroxy-phenylamino)-acetamid.

76.3
Analog Bsp. 12 erhielt man aus oben erhaltenen Material das (RS)-N-Benzyl-2-(4-benzyloxy-3-ethoxy-phenyl)-2-[3-hydroxy-4-(N-hydroxycarbamimidoyl)-phenylamino]-acetamid.

76.4
Reduktion des Amidoxims aus Bsp. 76.3 analog Bsp. 45 ergab das (RS)-N-Benzyl-2-(4-benzyloxy-3-ethoxy-phenyl)-2-(4-carbamimidoyl-3-hydroxy-phenylamino)-acetamid.

Beispiel 77

77.1
Eine Lösung von 14.35 g 4-Benzyloxy-5-methoxy-2-nitrobenzaldehyd in 175 ml MeOH wurde mit 5.9 g 4-Aminobenzonitril versetzt und während 1 Std. bei RT gerührt. Dabei entstand eine Suspension, welche mit 6.1 ml Benzylisonitril versetzt und dann auf 0°C gekühlt wurde. Anschliessend wurde innert 15 min 19 ml Bortrifluorid-ethyletherat zugetropft. Man liess auf RT erwärmen und rührte während 1.5 Std. nach, wobei eine Lösung entstand. Diese wurde im Vakuum eingedampft. Der Rückstand wurde in 330 ml MeOH und 3.6 ml $H_2O$ gelöst. Das Reaktionsgemisch wurde über Nacht bei RT gerührt, wobei ein Feststoff auskristallisierte. Dieser wurde abfiltriert, mit MeOH/$H_2O$ 8:2 und $Et_2O$ gewaschen und im HV getrocknet. Man erhielt 16.3 g (61%) (RS)-(4-Benzyloxy-5-methoxy-2-nitro-phenyl)-(4-cyano-phenylamino)-essigsäure-methylester als leicht gelben, kristallinen Feststoff.

77.2
Eine Lösung von 4.47 g des oben erhaltenen Nitrils in 100 ml THF wurde mit 1.79 g Pt/C 5% versetzt und während 1 Tag hydriert. Anschliessend wurde vom Katalysator abfiltriert und im Vakuum eingedampft. Der Rückstand wurde chromatographisch an Kieselgel gereinigt (Toluol/EtOAc 2:1). Man erhielt 2.24 g (50%) (RS)-(2-Amino-4-benzyloxy-5-methoxy-phenyl)-(4-cyano-phenylamino)-essigsäure-methylester als leicht orangen Festkörper. ISP-MS: 440.3 [M+Na].

77.3
Eine Lösung von 1.79 g des in Bsp 77.2 erhaltenen Materials in 60 ml $CH_2Cl_2$ wurde mit 4 ml DMF sowie 2.21 ml Diisopropylethylamin versetzt und auf 0°C gekühlt. Dann wurden innert 20 min 1.21 ml Phenylsulfonylchlorid in 10 ml $CH_2Cl_2$ zugetropft. Man liess innert 3 Std. auf RT aufwärmen. Die Reaktionslösung wurde im Vakuum eingedampft. Der Rückstand wurde chromatographisch an Kieselgel gereinigt (Toluol/EtOAc 4:1). Man erhielt 635 mg (21%) (RS)-[4-Benzyloxy-2-(bis-phenylsulfonyl-amino)-5-methoxy-phenyl]-(4-cyanophenylamino)-essigsäure-methylester als leicht braunen Festkörper. ISP-MS: 698.2 [M+H], 720.2 [M+Na].

77.4
Analog Bsp. 25 erhielt man aus 795 mg) des in Bsp 77.3 erhaltenen Nitrils die (RS)-[4-Benzyloxy-2-(bis-phenylsulfonyl-amino)-5-methoxy-phenyl]-(4-cyanophenylamino)-essigsäure. Diese wurde analog Bsp. 36.1, jedoch unter Verwendung von (S)-Phenylglycin-methylester anstelle von L-Alaninmethylester-hydrochlorid, zu einem 1:1 Gemisch von (S)-[(R)- und -[(S)-2-[4-benzyloxy-2-(bis-phenylsulfonyl-amino)-5-methoxy-phenyl]-2-(4-cyanophenylamino)-acetylamino]-phenyl-essigsäure-methylester umgesetzt. Letzteres wurde wiederum in 20 ml THF gelöst und mit 11 ml 1N LiOH-Lösung versetzt. Das Reaktionsgemisch wurde während 6 Std. auf 60°C und über Nacht auf 40°C erhitzt. Anschliessend wurde das THF im Vakuum abdestilliert. Die verbleibende wässrige Lösung wurde mit 2%iger Zitronensäure verdünnt und mit EtOAc extrahiert. Die organische Phase wurde mit gesättigter NaCl-Lösung gewaschen, über $MgSO_4$ getrocknet, abfiltriert und im Vakuum eingedampft. Der Rückstand wurde in 25 ml EtOH gelöst und mit 3.07 ml Triethylamin sowie 794 mg Hydroxylamin-hydrochlorid versetzt. Die Reaktionslösung wurde über Nacht unter Rückfluss erhitzt, dann im Vakuum eingedampft. Der Rückstand wurde durch zweimalige Chromatographie an Kieselgel (EtOAc/Aceton/$H_2O$/HOAc 6:2:1:1) gereinigt. Man erhielt 256 mg (29%) eines 1:1 Gemisches von (S)-[(R)-und -[(S)-2-(2-phenylsulfonylamino-4-benzyloxy-5-methoxy-phenyl)-2-[4-[(E)-und/oder -[(Z)-N-hydroxycarbamimidoyl)-phenylamino]-acetylamino]-phenylessigsäure als leicht braunes Lyophilisat. ISP-MS: 710.1 [M+H], 732.2 [M+Na].

77.5
Eine Lösung von 190 mg oben erhaltenen Amidoxims in 8 ml EtOH und 1 ml HOAc wurde mit einer Spatelspitze Raney-Nickel versetzt und über Nacht hydriert. Der Katalysator wurde abfiltriert und das Filtrat im Vakuum eingeengt. Der Rückstand wurde chromatographisch an Kieselgel gereinigt. Man erhielt die beiden Epimeren

a) (S)-[(R)-2-(2-Phenylsulfonylamino-4-benzyloxy-5-methoxy-phenyl)-2-(4-carbamimidoyl-phenylamino)-acetylamino]-phenylessigsäure-acetat (1:1) und

b) (S)-[(S)-2-(2-Phenylsulfonylamino-4-benzyloxy-5-methoxy-phenyl)-2-(4-carbamimidoyl-phenylamino)-acetylamino]-phenyl-essigsäure-acetat (1:1), beide als farblose Lyophilisate. ISP-MS: 694.2 [M+H].

<u>Beispiel 78</u>

78.1
Zu einer Lösung von 14.3 g 4-Benzyloxy-5-methoxy-2-nitrobenzaldehyd in 350 ml Allylalkohol wurden 5.9 g 4-Aminobenzonitril gegeben. Die gelbe Suspension wurde während 1.5 Std. bei RT gerührt. Dann wurden 6.1 ml Benzylisonitril zugegeben und das Reaktionsgemisch wurde auf 0°C gekühlt. Es wurden 19 ml Bortrifluorid-etherat zugetropft. Man liess die Suspension auf RT erwärmen. Anschliessend wurde der Festkörper abfiltriert, mit Diethylether nachgewaschen und in 200 ml Allylalkohol sowie 18 ml Wasser suspendiert. Das Reaktionsgemisch wurde über Nacht bei RT gerührt und dann im Vakuum eingedampft. Der Rückstand wurde chromatographisch an Kieselgel gereinigt (Toluol/Aceton 9:1). Das Produkt wurde auf THF/Hexan umkristallisiert. Man erhielt 7.35 g (45%) (RS)-(4-Benzyloxy-5-methoxy-2-nitro-phenyl)-(4-cyanophenylamino)-essigsäure-allylester als hellgelbe Kristalle. ISN-MS: 472.1 [M-H].

78.2
Zu einer Lösung von 7.1 g des unter Bsp 78.1 erhaltenen Nitrils in 75 ml THF wurden 2 g Dimedon (5,5-Dimethyl-1,3-cyclohexandion) und 1.8 g Tetrakis(triphenylphosphin)palladium gegeben. Das Reaktionsgemisch wurde während 30 min bei RT gerührt, anschliessend mit Aktivkohle versetzt, nochmals während 30 min bei RT gerührt und dann filtriert. Das Filtrat wurde im Vakuum eingedampft. Der Rückstand wurde durch zweimalige Chromatographie an Kieselgel gereinigt (erst $CH_2Cl_2$/MeOH 19:1, dann EtOAc). Man erhielt 2.25 g (35%) (RS)-(4-Benzyloxy-5-methoxy-2-nitro-phenyl)-(4-cyano-phenylamino)-essigsäure als gelben Festkörper. ISP-MS: 434.3 [M+H].

78.3

Analog Bsp. 36.1 erhielt man aus 1.67 g des unter Bsp 78.2 erhaltenen Nitrils und (S)-Phenylglycin-methylester anstelle von L-Alaninmethylester-hydrochlorid 1.03 g (46%) einer 1:1 Mischung von (S)-[(R)- und -[(S)-2-(4-Benzyloxy-5-methoxy-2-nitro-phenyl)-2-(4-cyano-phenylamino)-acetylamino]-phenyl-essigsäure-methylester als rotbraunen, festen Schaum. ISP-MS: 581.1 [M+H], 603.0 [M+Na].

78.4

Analog Bsp. 27 erhielt man aus 636 mg der Nitroverbindung aus Bsp 78.3 235 mg (40%) eines 1:1 Gemisches aus (S)-[(R)- und -[(S)-2-(2-Amino-4-benzyloxy-5-methoxy-phenyl)-2-(4-cyano-phenylamino)-acetylamino]-phenyl-essigsäuremethylester als farblosen, kristallinen Feststoff. ISN-MS: 549.1 [M+H].

78.5

Analog Bsp. 50.1 erhielt man aus 100 mg des obigen Amino-Nitrils und Phenylacetylchlorid 118 mg (98%) einer 1:1 Mischung von (S)-[(R)- und -[(S)-2-(4-benzyloxy-5-methoxy-2-phenylacetylamino-phenyl)-2-(4-cyano-phenylamino)-acetylamino]-phenyl-essigsäure-methylester als farblosen, festen Schaum. ISN-MS: 667.2 [M-H].

78.6

Analog Bsp. 36.2 erhielt man aus 108 mg des in Bsp 78.5 erhaltenen Nitrils 104 mg (98%) einer 1:1 Mischung aus (S)-[(R)- und -[(S)-2-(4-Benzyloxy-5-methoxy-2-phenylacetylamino-phenyl)-2-(4-cyano-phenylamino)-acetylamino]-phenylessigsäure als festen, leicht gelben Schaum. ISP-MS: 655.1 [M+H].

78.7

Analog Bsp. 36.3 erhielt man aus 180 mg (des obigen Nitrils 133 mg (70%) eines 1:1 Gemisches von (S)-[(R)- und -[(S)-2-(4-Benzyloxy-5-methoxy-2-phenylacetylamino-phenyl)-2-[4-[(E)- und/oder [(Z)-N-Hydroxycarbamimidoyl]-phenylamino]-acetylamino]-phenyl-essigsäure als farbloses Pulver. ISP-MS: 688.2 [M+H], 710.1 [M+Na].

78.8

Aus dem Epimerengemisch aus Bsp. 78.7 erhielt man analog Bsp. 33 und nach chromatografischer Trennung des Produktes an Kieselgel die beiden Epimeren

a) (S)-[(R)-2-(2-Benzenesulfonylamino-4-benzyloxy-5-methoxy-phenyl)-2-(4-carbamimidoyl-phenylamino)-acetylamino]-phenylessigsäure-acetat (1:1) und

b) (S)-[(S)-2-(2-Benzenesulfonylamino-4-benzyloxy-5-methoxy-phenyl)-2-(4-carbamimidoyl-phenylamino)-acetylamino]-phenyl-essigsäure -acetat (1:1), beide als farblose Pulver. ISP-MS: 672.3 [M+H].

Beispiel 79

Analog Bsp. 78.5 - 78.8 erhielt man aus dem Produkt von Bsp. 78.4, jedoch unter Verwendung von Essigsäureanhydrid anstelle von Phenylacetylchlorid, nach chromatografischer Trennung die beiden Epimeren (S)-[(R)-2-(2-Acetylamino-4-benzyloxy-5-methoxy-phenyl)-2-(4-cyano-phenylamino)-acetylamino]-phenylessigsäure und (S) -[(S)-2-(2-Acetylamino-4-benzyloxy-5-methoxy-phenyl)-2-(4-cyano-phenylamino)-acetylamino]-phenyl-essigsäure. Diese wurden über die jeweiligen Amidoxime zu den beiden Epimeren

a) (S)-[(R)-2-(2-Acetylamino-4-benzyloxy-5-methoxy-phenyl)-2-(4-carbamimidoyl-phenylamino)-acetylamino]-phenyl-essigsäure-acetat und

b) (S)-[(S)-2-(2-Acetylamino-4-benzyloxy-5-methoxy-phenyl)-2-(4-carbamimidoyl-phenylamino)-acetylamino]-phenyl-essigsäure-acetat umgesetzt. ISP-MS: 596.2 [M+H].

Beispiel 80

Eine Lösung von 0.024 ml) 4-Fluorbenzoylchlorid in 1.0ml THF und 0.5ml DMF wurde mit 0.038 ml N,N-Diisoproplyethylamin versetzt und während 5 Minuten bei RT gerührt. Anschliessend wurden 100 mg des Produktes aus Beispiel 5.g zugegeben. Das Reaktionsgemisch wurde über Nacht bei RT gerührt. Es wurden nochmals 0.024 ml 4-Fluorbenzoylchlorid und 0.1 ml N,N-Diisoproplyethylamin zugesetzt und während 3 Std. bei RT gerührt. Das Reaktionsgemisch wurde im Vakuum eingedampft. Der Rückstand wurde in gesättigter $NaHCO_3$-Lösung aufgenommen und mit EtOAc extrahiert. Die organische Phase wurde über $MgSO_4$ getrocknet, filtriert und im Vakuum eingedampft. Der Rückstand wurde chromatographisch an Kieselgel gereinigt (Hexan/EtOAc 2:1). Das Produkt wurde aus $Et_2O$

kristallisiert. Man erhielt 20 mg (17%) (RS)-N-[Amino-(4-{[benzylcarbamoyl-(4-benzyloxy-3-ethoxy-phenyl)-methyl]-amino}-phenyl)-methylen]-4-fluoro-benzamid als kristallinen Feststoff. ISP-MS: 631.2 [M+H].

Beispiel 81

Analog Bsp. 80 erhielt man aus dem Produkt aus Beispiel 5.g und 2-Benzyloxymethylbenzoylchlorid den (RS)-Benzoesäure-2-{[amino-(4-{[benzylcarbamoyl-(4-benzyloxy-3-ethoxy-phenyl)-methyl]-amino}-phenyl)-methylen]-carbamoyl}-benzylester in 21% Ausbeute. Gelber kristalliner Feststoff. ISP-MS: 747.4 [M+H].

Beispiel 82

Analog Bsp. 80 erhielt man aus dem Produkt von Beispiel 5.g und p-Nitrophenyl-(2,2,2-trichlorethyl)-carbonat den (RS)-[Amino-(4-{[benzylcarbamoyl-(4-benzyloxy-3-ethoxy-phenyl)-methyl]-amino}-phenyl)-methylene]-carbamidsäure-2,2,2-trichloro-ethylester in 60% Ausbeute. Kristalliner Festkörper. ISP-MS: 683.1 [M+H].

Beispiel 83

Analog Bsp. 80 erhielt man aus dem Produkt von Beispiel 5.g und Methyl-(4-nitrophenyl)-carbonat den (RS)-[Amino-(4-{[benzylcarbamoyl-(4-benzyloxy-3-ethoxy-phenyl)-methyl]-amino}-phenyl)-methylene]-carbamidsäure-methylester in 91% Ausbeute als Festkörper. ISP-MS: 567.3 [M+H].

Eine Verbindung der Formel I, ein Hydrat, ein Solvat oder ein Salz davon kann als Wirkstoff zur Herstellung von pharmazeutischen Präparaten, wie die folgenden, verwendet werden.

| Beispiel A | |
|---|---|
| | pro Tablette |
| Wirkstoff | 200 mg |
| mikrokristalline Cellulose | 155 mg |
| Maisstärke | 25 mg |
| Talk | 25 mg |
| Hydroxypropylmethylcellulose | 20 mg |
| | 425 mg |

| Beispiel B | |
|---|---|
| | pro Kapsel |
| Wirkstoff | 100,0 mg |
| Maisstärke | 20,0 mg |
| Milchzucker | 95,0 mg |
| Talk | 4,5 mg |
| Magnesiumstearat | 0,5 mg |
| | 220,0 mg |

**Patentansprüche**

1. N-(Carbamimido-phenyl)-glycinamidderivate der Formel

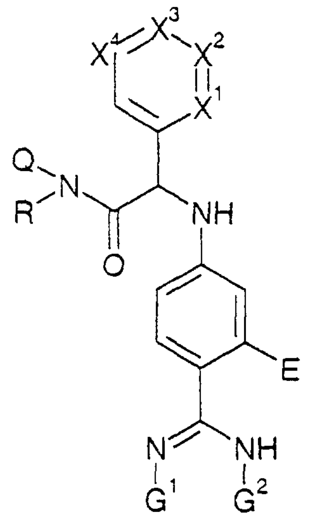 . I

worin

| | |
|---|---|
| E | Wasserstoff oder OH, |
| Q | Wasserstoff oder Alkyl, |
| R | Aryl, Cycloalkyl oder durch $R^1$, $R^2$ und $R^3$ substituiertes Alkyl, |
| $R^1$ | Wasserstoff, COOH, COO-Alkyl oder Aryl, |
| $R^2$ | Wasserstoff, Aryl, Cycloalkyl oder Heteroaryl, |
| $R^3$ | Wasserstoff, Aryl oder (in einer anderen als die $\alpha$-Stellung zum N-Atom an dem die Alkylgruppe R gebunden ist) OH, Alkoxy oder gegebenenfalls geschütztes Amino, |
| N(Q,R) | durch COOH oder COO-Alkyl substituiertes Pyrrolidino,Piperidino oder 1,2,3,4-Tetrahydroisochinolin-3-yl, |
| drei von $X^1$ bis $X^4$ | unabhängig voneinander eine Gruppe $C(R^a)$, $C(R^b)$ oder $C(R^c)$ und das vierte eine Gruppe $C(R^d)$ oder N, |
| $R^a$ bis $R^d$ | unabhängig voneinander H, OH, $NO_2$, Dialkyl-amino, Halogen, Alkyl, Alkoxy, Aryloxy, Aralkyloxy, Heteroaryl-alkyloxy, Heterocyclyl-alkyloxy, COOH, COO-Alkyl, NH-$SO_2$-Alkyl, $NHSO_2$-Aryl, NHCO-Alkyl, NHCO-Aryl, NHCOO-Alkyl, NHCO-Alkyl-$NH_2$, NHCO-Alkyl-NH -G, Aralkyl-CONH, Alkyl-O-alkyl-CONH, Aryl-O-alkyl-CONH, Alkyl-COOH, Alkyl-COO-alkyl, |

O-Alkyl-COOH oder O-Alkyl-COO-alkyl, oder
zwei benachbarte Gruppen $R^a$ bis $R^d$ zusammen Alkylendioxy sind,
wobei nicht mehr als drei von $R^a$ bis $R^d$ die gleiche Bedeutung haben sollen und $X^1$ nicht CCOOH oder CCOO-Alkyl sein soll,

| | |
|---|---|
| G | eine Aminoschutzgruppe und |
| eins von $G^1$ und $G^2$ | Wasserstoff und das andere Wasserstoff, Alkyl, OH, Alkoxy, Aroyl, Alkanoyl-$OCH_2$, Aroyl-$OCH_2$ oder eine Gruppe COO-$R^g$ oder OCO-$R^g$, $R^g$ gegebenenfalls durch Halogen, OH, Alkoxy, COOH oder COO-Alkyl substituiertes Alkyl, |

sowie Hydrate oder Solvate und physiologische verwendbare Salze davon.

**2.** Verbindungen nach Anspruch 1, worin E, $G^2$ und Q Wasserstoff,

R Alkyl, Aryl, Aralkyl, Benzhydryl, Cycloalkyl-alkyl oder Heteroaryl-alkyl,
$G^1$ Wasserstoff, OH oder COO-Alkyl,
$X^1$ bis $X^4$ eine Gruppe $C(R^a)$ bis $C(R^d)$ und
$R^a$ bis $R^d$ unabhängig voneinander H, OH, $NO_2$, Dialkyl-amino, Halogen, Alkyl, Alkoxy, Aryloxy, Aralkyloxy, Heteroaryl-alkyloxy, Heterocyclyl-alkyloxy, COO-Alkyl, $NHSO_2$-Alkyl oder $NHSO_2$-Aryl sind,

wobei nicht mehr als drei von $R^a$ bis $R^d$ die gleiche Bedeutung haben sollen und $X^1$ nicht COO-Alkyl sein soll.

**3.** Verbindungen nach Anspruch 1 oder 2, worin E, $G^1$, $G^2$ und Q Wasserstoff sind.

**4.** Verbindungen nach Anspruch 1 oder 3, worin R durch $R^1$, $R^2$ und $R^3$ substituiertes Alkyl, insbesondere Methyl oder Ethyl, insbesondere worin $R^1$ Aryl, speziell Phenyl, oder COOH, $R^2$ Wasserstoff oder Aryl, speziell Phenyl, und $R^3$ Wasserstoff ist.

**5.** Verbindungen nach Anspruch 1, 3 oder 4, worin $X^1$ bis $X^4$ eine Gruppe $C(R^a)$ bis $C(R^d)$, insbesondere worin $R^a$ H, Aralkyloxy, speziell Carboxy-benzyloxy; $NHSO_2$-Aryl, speziell Phenylsulfonyl-amino; Aralkyl-CONH, speziell Phenylacetylamino, oder O-Alkyl-COOH, speziell Carboxymethoxy, $R^b$ H oder Alkoxy, speziell Methoxy oder Ethoxy, $R^c$ H, Alkoxy, speziell Methoxy; oder Aralkyloxy, speziell Benzyloxy, und $R^d$ H oder Alkoxy, speziell Methoxy ist.

**6.** Verbindungen nach einem der Ansprüche 1 bis 5 nämlich:

(R,S)-N-Benzyl-2-(4-benzyloxy-3-ethoxy-phenyl)-2-(4-carbamimidoyl-phenylamino)-acetamid,
2-(2-Phenylsulfonylamino-4-benzyloxy-5-methoxy-phenyl)-N-benzyl-2-(4-carbamimidoyl-phenylamino)-acetamid.

**7.** Verbindungen nach einem der Ansprüche 1 und 3-5 nämlich:

(RS)-[2-(4-Benzyloxy-3-methoxy-phenyl)-2-(4-carbamimidoyl-phenylamino)-acetylamino]-diphenyl-essigsäure,
(RS)- und (SR)-3-[(RS)-2-(4-Benzyloxy-3-methoxy-phenyl)-2-(4-carbamimidoylphenylamino)-acetylamino]-3-phenyl-propionsäure,
(S)-[(R)-2-(4-Benzyloxy-3-methoxy-phenyl)-2-(4-carbamimidoyl-phenylamino)-acetylamino]-phenyl-essigsäure,
(S)-[(S)-2-(4-Carbamimidoyl-phenylamino)-2-(3,5-dimethoxy-phenyl)-acetylamino]-phenyl-essigsäure,
(RS)-3-[2-[Benzylcarbamoyl-(4-carbamimidoyl-phenylamino)-methyl]-4,5-dimethoxy-phenoxymethyl]-benzoesäure,
(RS)-[2-[Benzylcarbamoyl-(4-carbamimidoyl-phenylamino)-methyl]-4,5-dimethoxy-phenoxy]-essigsäure,
(S)-[(R)-2-(2-Phenylsulfonylamino-4-benzyloxy-5-methoxy-phenyl)-2-(4-carbamimidoyl-phenylamino)-acetylamino]-phenylessigsäure,
(S)-[(S)-2-(2-Phenylsulfonylamino-4-benzyloxy-5-methoxy-phenyl)-2-(4-carbamimidoyl-phenylamino)-acetylamino]-phenyl-essigsäure,
(S)-[(R)-2-(4-Benzyloxy-5-methoxy-2-phenylacetylamino-phenyl)-2-(4-carbamimidoyl-phenylamino)-acetylamino]-phenyl-essigsäure
(S)-[(S)-2-(4-Benzyloxy-5-methoxy-2-phenylacetylamino-phenyl)-2-(4-carbamimidoyl-phenylamino)-acetylamino]-phenyl-essigsäure

**8.** Verbindungen der Formel

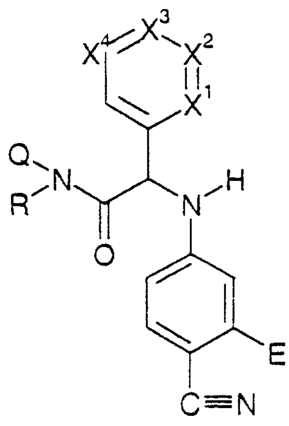 IV, 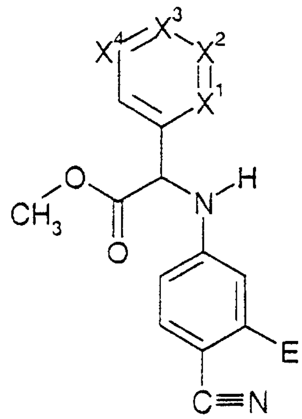 V oder 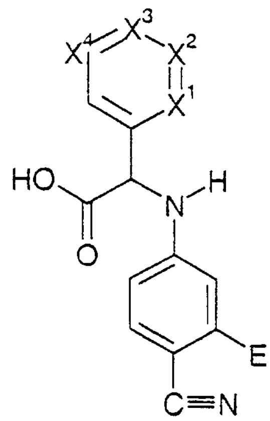 VI

worin E, Q, R und $X^1$ bis $X^4$ die gleiche Bedeutung wie in Anspruch 1 haben.

**9.** Pharmazeutische Präparate enthaltend eine Verbindung nach einem der Anprüche 1-7 als Wirkstoff.

**Claims**

**1.** N-(Carbamimido-phenyl)-glycinamide derivatives of the formula

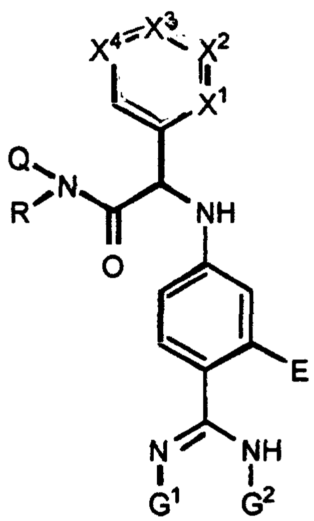

I

wherein

| | |
|---|---|
| E | is hydrogen or OH, |
| Q | is hydrogen or alkyl, |
| R | is aryl, cycloalkyl or alkyl substituted by $R^1$, $R^2$ and $R^3$, |
| $R^1$ | is hydrogen, COOH, COO-alkyl or aryl, |
| $R^2$ | is hydrogen, aryl, cycloalkyl or heteroaryl, |
| $R^3$ | is hydrogen, aryl or (in a position other than the $\alpha$-position to the N atom to which the alkyl group R is attached) OH, alkoxy or optionally protected amino, |
| N(Q,R) | xis pyrrolidino, piperidino or 1,2,3,4-tetrahydroisoquinolin-3-yl substituted by COOH or COO-alkyl, |
| three of $X^1$ to $X^4$ | independently of one another are a group $C(R^a)$, $C(R^b)$ or $C(R^c)$ and the fourth is a group C ($R^d$) or N, |
| $R^a$ to $R^d$ | independently of one another are H, OH, $NO_2$, dialkyl-amino, halogen, alkyl, alkoxy, aryloxy, |

aralkyloxy, heteroaryl-alkyloxy, heterocyclylalkyloxy, COOH, COO-alkyl, NH-$SO_2$-alkyl, $NHSO_2$-aryl, NHCO-alkyl, NHCO-aryl, NHCOO-alkyl, NHCO-alkyl-$NH_2$, NHCO-alkyl-NH-G, aralkyl-CONH, alkyl-O-alkyl-CONH, aryl-O-alkyl-CONH, alkyl-COOH, alkyl-COO-alkyl, O-allcyl-COOH or O-alkyl-COO-alkyl, or two adjacent groups $R^a$ to $R^d$ together are alkylenedioxy, whereby not more than three of $R^a$ to $R^d$ shall have the same significance and $X^1$ shall not be CCOOH or CCOO-alkyl,

G is an amino protecting group and

one of $G^1$ and $G^2$ is hydrogen and the other is hydrogen, alkyl, OH, alkoxy, aroyl, alkenoyl-$OCH_2$, aroyl-$OCH_2$ or a group COO-$R^g$ or OCO-$R^g$, $R^g$ is alkyl optionally substituted by halogen, OH, alkoxy, COOH or COO-alkyl,

as well as hydrates or solvates and physiologically acceptable salts thereof.

**2.** Compounds according to claim 1, wherein E, $G^2$ and Q are hydrogen,

R is alkyl, aryl, aralkyl, benzhydryl, cycloalkyl-alkyl or heteroaryl-alkyl,
$G^1$ is hydrogen, OH or COO-alkyl,
$X^1$ to $X^4$ are a group C($R^a$) to (C$R^d$) and
$R^a$ to $R^d$ independently of one another are H, OH, $NO_2$, dialkylamino-alkyl, halogen, alkyl, alkoxy, aryloxy, aralkyloxy, heteroaryl-alkyloxy, heterocydyl-alkyloxy, COO-alkyl, $NHSO_2$-alkyl or $NHSO_2$-aryl,

whereby not more than three of $R^a$ to $R^d$ shall have the same significance and $X^1$ shall not be COO-alkyl.

**3.** Compounds according to claim 1 or 2, wherein E, $G^1$ $G^2$ and Q are hydrogen.

**4.** Compounds according to claim 1 or 3, wherein R is alkyl, particularly methyl or ethyl, substituted by $R^1$, $R^2$ and $R^3$, particularly wherein $R^1$ is aryl, especially phenyl, or COON, $R^2$ is hydrogen or aryl, especially phenyl, and $R^3$ is hydrogen.

**5.** Compounds according to claim 1,3 or 4, wherein $X^1$ to $X^4$ are a group C($R^a$) to C($R^d$), particularly wherein $R^a$ is H, aralkyloxy, especially carboxy-benzyloxy; $NHSO_2$-aryl, especially phenylsulphonyl-amino; aralkyl-CONH, especially phenylacetylamino, or O-alkyl-COOH, especially carboxymethoxy, $R^b$ is H or alkoxy, especially methoxy or ethoxy, $R^c$ is H, alkoxy, especially methoxy; or aralkyloxy, especially benzyloxy, and $R^d$ is H or alkoxy, especially methoxy.

**6.** Compounds according to any one of claims 1 to 5, namely:

(R,S)-N-Benzyl-2-(4-benzyloxy-3-ethoxy-phenyl)-2-(4-carbamimidoyl-phenylamino)-acetamide,
2-(2-phenylsulphonylamino-4-benzyloxy-5-methoxy-phenyl)-N-benzyl-2-(4-carbamimidoyl-phenylamino)-acetamide.

**7.** Compounds according to any one of claims 1 to 5, namely:

(RS)-[2-(4-Benzyloxy-3-methoxy-phenyl)-2-(4-carbamimidoyl-phenylamino)-acetylamino]-diphenyl-acetic acid,
(RS)- and (SR)-3-[(RS)-2-(4-benzyloxy-3-memoxy-phenyl)-2-(4-carbamimidoylphenylamino)-acetylamino]-3-phenyl-propionic acid,
(S)-[(R)-2-(4-benzyloxy-3-methoxy-phenyl)-2-(4-carbamimidoyl-phenylamino)-acetylamino]-phenyl-acetic acid,
(S)-[(S)-2-(4-carbamimidoyl-phenylamino)-2-(3,5-dimethoxy-phenyl)-acetylamino]-phenyl-acetic acid,
(RS)-3-[2-[benzylcarbamoyl-(4-carbamimidoyl-phenylamino)-methyl]-4,5-dimethoxyphenoxymethyl]-benzoic acid,
(RS)-[2-[benzylcarbamoyl-(4-carbamimidoyl-phenylamino)-methyl]-4,5-dimethoxyphenoxy]-acetic acid,
(S)-[(R)-2-(2-phenylsulphonylamino)-4-benzyloxy-5-methoxy-phenyl)-2-(4-carbamimidoylphenylamino)-acetylamino]-phenylacetic acid,
(S)-[(S)-2-(2-phenylsulphonylamino)-4-benzyloxy-5-methoxy-phenyl)-2-(4-carbamimidoylphenylamino)-acetylamino]-phenyl-acetic acid,
(S)-[(R)-2-(4-benzyloxy-5-methoxy-2-phenylacetylamina-phenyl)-2-(4-carbamimidoylphenylamino)-acetylamino]-phenyl-acetic acid,
(S)- [(S)-2-(4-benzyloxy-5-methoxy-2-phenylacetylamino-phenyl)-2-(4-carbamimidoylphenylamino)-acetylamino]-phenyl-acetic acid.

**8.** Compounds of the formula

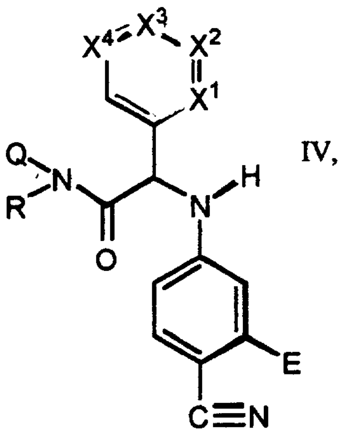
IV,
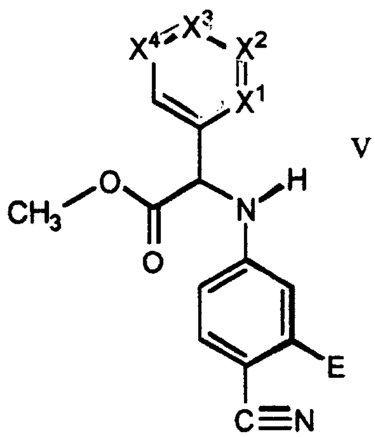
V or
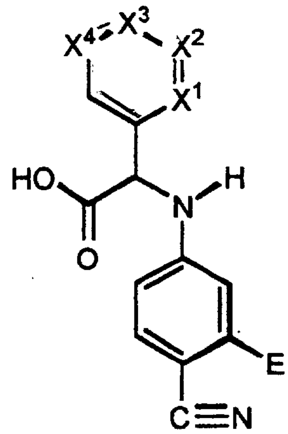
VI

wherein E, Q, R and $X^1$ to $X^4$ have the same significance as in claim 1.

**9.** Pharmaceutical preparations containing a compound according to any one of claims 1-7 as the active ingredient.

**Revendications**

**1.** Dérivés de N-(carbamimldophényl)glycinamide de formule

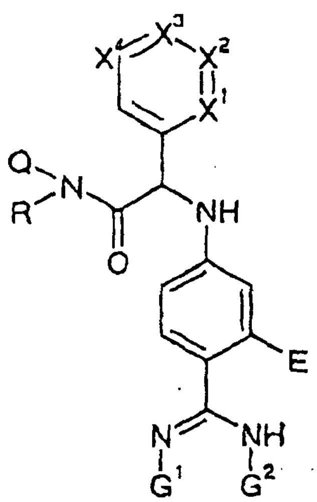
I

dans laquelle

| | |
|---|---|
| E | représente un atome d'hydrogène ou OH, |
| Q | représente un atome d'hydrogène ou un groupe alkyle, |
| R | représente un groupe aryle, cycloalkyle ou un groupe alkyle substitué par $R^1$, $R^2$ et $R^3$, |
| $R^1$ | représente un atome d'hydrogène ou un groupe COOH, COO-alkyle ou aryle, |
| $R^2$ | représente un atome d'hydrogène ou un groupe aryle, cycloalkyle ou hétéroaryle, |

$R^3$ représente un atome d'hydrogène, un groupe aryle ou (en une autre position que la position $\alpha$ par rapport à l'atome d'azote auquel est lié le groupe alkyle R) un groupe OH, alcoxy ou amino éventuellement protégé,

N(Q,R) représente un groupe pyrrolidino, pipéridino ou 1,2,3,4-tétrahydroisoquinolin-3-yle substitué par un groupe COOH ou COO-alkyle,

trois des radicaux $X^1$ à $X^4$ représentent, indépendamment les uns des autres, un groupe $C(R^a)$, $C(R^b)$ ou $C(R^c)$ et le quatrième représente un groupe $C(R^d)$ ou N,

$R^a$ à $R^d$ représentent, indépendamment les uns des autres, un atome d'hydrogène ou d'halogène ou un groupe OH, $NO_2$, dlalkylamino, alkyle, alcoxy, aryloxy, aralkyloxy, hétéroaryl-alkyloxy, hétérocyclyl-alkyloxy, COOH, COO-alkyle, $NHSO_2$-alkyle, $NHSO_2$-aryle, NHCO-alkyle, NHCO-aryle, NHCOO-alkyle, NHCO-alkyl-$NH_2$, NHCO-alkyl-NH-G, aralkyl-CONH, alkyl-O-alkyl-CONH, aryl-O-alkyl-CONH, alkyl-COOH, alkyl-COO-alkyle, O-alkyl-COOH ou O-alkyl-COO-alkyle, ou deux groupes $R^a$ à $R^d$ voisins peuvent former ensemble un groupe alkylènedioxy,

au maximum trois des radicaux $R^a$ à $R^d$ pouvant avoir la même signification et $X^1$ ne devant pas être un groupe CCOOH ou CCOO-alkyle,

G représente un groupe protecteur de fonction amino et

l'un des radicaux $G^1$ et $G^2$ représente un atome d'hydrogène et l'autre représente un atome d'hydrogène ou un groupe alkyle, OH, alcoxy, aroyle, alcanoyl-$OCH_2$, aroyl-$OCH_2$ ou un groupe COO-$R^g$ ou OCO-$R^g$, $R^g$ étant un groupe alkyle éventuellement substitué par un halogène ou par un groupe OH, alcoxy, COOH ou COO-alkyle,

et hydrates ou produits de solvatation et sels physiologiquement utilisables de tels composés.

**2.** Composés selon la revendication 1, dans lesquels E, $G^2$ et Q représentent des atomes d'hydrogène,

R représente un groupe alkyle, aryle, aralkyle, benzhydryle, cycloalkyl-alkyle ou hétéroaryl-alkyle,
$G^1$ représente un atome d'hydrogène ou un groupe OH ou COO-alkyle,
$X^1$ à $X^4$ représentent un groupe $C(R^a)$ à $C(R^d)$ et
$R^a$ à $R^d$ représentent, indépendamment les uns des autres, un atome d'hydrogène ou d'halogène ou un groupe OH, $NO_2$, dialkylamino, alkyle, alcoxy, aryloxy, aralkyloxy, hétéroaryl-alkyloxy, hétérocyclyl-alkyloxy, COO-alkyle, $NHSO_2$-alkyle ou $NHSO_2$-aryle,

au maximum trois des radicaux $R^a$ à $R^d$ pouvant avoir la même signification et $X^1$ ne devant pas être un groupe COO-alkyle.

**3.** Composés selon la revendication 1 ou 2, dans lesquels E, $G^1$, $G^2$ et Q sont des atomes d'hydrogène.

**4.** Composés selon la revendication 1 ou 3, dans lesquels R représente un groupe alkyle substitué par $R^1$, $R^2$ et $R^3$, notamment le groupe méthyle ou éthyle, en particulier dans lesquels $R^1$ est un groupe aryle, en particulier le groupe phényle, ou COOH, $R^2$ est un atome d'hydrogène ou un groupe aryle, en particulier phényle, et $R^3$ est un atome d'hydrogène.

**5.** Composés selon la revendication 1, 3 ou 4, dans lesquels $X^1$ à $X^4$ représentent un groupe $C(R^a)$ à $C(R^d)$, en particulier dans lesquels $R^a$ représente un atome d'hydrogène pu un groupe aralkyloxy, en particulier carboxybenzyloxy, $NHSO_2$-aryle, en particulier phénylsulfonylamino ; aralkyl-CONH, en particulier phénylacétylamino, ou O-alkyl-COOH, en particulier carboxyméthoxy, $R^b$ est un atome d'hydrogène ou un groupe alcoxy, en particulier méthoxy ou éthoxy, $R^c$ est un atome d'hydrogène ou un groupe aicoxy, en particulier méthoxy ; ou un groupe aralkyloxy, en particulier benzyloxy, et $R^d$ est un atome d'hydrogène ou un groupe alcoxy, en particulier méthoxy.

**6.** Composés selon l'une quelconque des revendications 1 à 5, à savoir :

(R,S)-N-benzyl-2-(4-benzyloxy-3-éthoxyphényl)-2-(4-carbamlmidoylphénylamino)acétamide,
2-(2-phénylsulfonylamino-4-benzyloxy-5-méthoxyphényl)-N-benzyl-2-(4-carbamimidoylphénylamino)acétamide.

**7.** Composés selon l'une quelconque des revendications 1 et 3-5, à savoir :

acide (RS)-[2-(4-benzyioxy-3-méthoxyphényl)-2-(4-carbamimidoylphénylamino)-acétylamino]diphénylacétique,
acide (RS)- et (SR)-3-[(RS)-2-(4-benzyloxy-3-méthoxyphényl)-2-(4-carbamimidoylphénylamino)acétylamino]-3-phénylpropionique,
acide (S)-[(R)-2-(4-benzyloxy-3-méthoxyphényl)-2-(4-carbamimidoylphénylamino)acétylamino]phénylacétique,
acide (S)-[(S)-2-(4-carbamimidoylphénylamino)-2-(3,5-diméthoxyphényl)acétylamino]phénylacétique,
acide (RS)-3-[2-[benzylcarbamoyl-(4-carbamimidoylphénylamino)méthyl]-4,5-diméthoxyphénoxyméthyl]benzoïque,
acide (RS)-[2-[benzylcarbamoyl-(4-carbamimidoylphénylamino)méthyl]-4,5-diméthoxyphénoxy]acétique,
acide (S)-[(R)-2-(2-phénylsulfonylamino-4-benzyloxy-5-méthoxyphényl)-2-(4-carbamimidoylphénylamino) acétylamino]phénylacétique,
acide (S)-[(S)-2-(2-phénylsulfonylamino-4-benzyloxy-5-méthoxyphényl)-2-(4-carbamimidoylphénylamino) acétylamino]phénylacétique,
acide (S)-[(R)-2-(4-benzyloxy-5-méthoxy-2-phénylacétylaminophényl)-2-(4-carbamimidoylphénylamino)acétylamino]phénylacétique,
acide (S)-[(S)-2-(4-benzyloxy-5-méthoxy-2-phénylacétylaminophényl)-2-(4-carbamimidoylphénylamino)acétylamino]phénylacétique.

**8.** Composés de formule

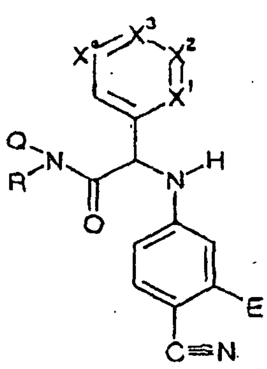 IV, 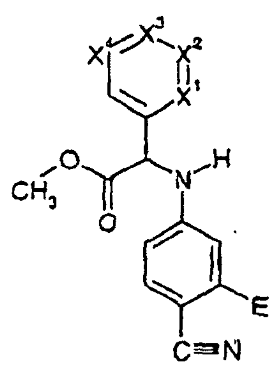 V ou 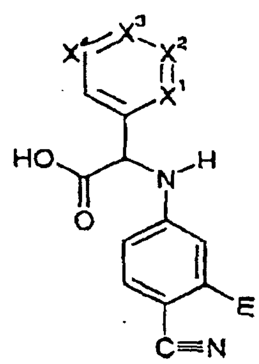 VI

dans lesquels E, Q, R et $X^1$ à $X^4$ ont les mêmes significations que dans la revendication 1.

**9.** Préparations pharmaceutiques contenant comme substance active un composé selon l'une quelconque des revendications 1 à 7.